# EUROPEAN PATENT APPLICATION

(11) **EP 4 317 435 A1**
(43) Date of publication of application: **07.02.2024**
(21) Application number: 22780696.5
(22) Date of filing: 28.03.2022
(51) Int. Cl.: C12N 15/13, A61K 39/395, A61K 47/55, A61K 47/68, A61P 35/00, A61P 35/02, C07K 16/28, C07K 16/30, C07K 16/46, C12N 1/15, C12N 1/19, C12N 1/21, C12N 5/10, C12N 15/62, C12N 15/63, C12P 21/08

(54) **STABLE MULTISPECIFIC MOLECULE AND USE THEREOF**

(30) Priority: 29.03.2021 JP 2021055375; 28.09.2021 JP 2021157580
(71) Applicant: Daiichi Sankyo Company, Limited, Tokyo 103-8426 (JP)
(72) Inventor: NAKAYAMA Makiko, Tokyo 103-8426 (JP); NAKAMURA Kensuke, Tokyo 103-8426 (JP); FURUZONO Shinji, Tokyo 103-8426 (JP); MATSUMOTO Ryota, Tokyo 103-8426 (JP)
(74) Representative: Marks & Clerk LLP
(86) International application number: PCT/JP2022/014868
(87) International publication number: WO 2022/210485

(57) **Abstract**

To provide a stable bispecific molecule.

A Fab that specifically binds to CD3, the Fab comprising the following CDRH1 to 3 and CDRL1 to 3:
CDRH1 consisting of the amino acid sequence represented by SEQ ID NO: 32;
CDRH2 consisting of the amino acid sequence represented by SEQ ID NO: 33;
CDRH3 consisting of the amino acid sequence represented by SEQ ID NO: 34;
CDRL1 consisting of the amino acid sequence represented by SEQ ID NO: 35;
CDRL2 consisting of the amino acid sequence represented by SEQ ID NO: 36; and
CDRL3 consisting of the amino acid sequence represented by SEQ ID NO: 37.

## Description

### Technical Field

The present invention relates to a multispecific antibody recognizing two or more antigens, a multispecific molecule and components of the molecule or the like.

### Background Art

As a molecule that binds multiple antigens an antibody that is a single molecule but binds to two or more types of antigens (bispecific antibody, multispecific antibody) has been studied.

A bispecific antibody is used as, for example, a T-cell redirecting bispecific antibody, which is a single molecule recognizing both CD3 on a T cell and a target antigen expressed on a cancer cell surface to connect by crosslinking the T cell and the cancer cell, with the result that cytotoxicity by the T cell to the cancer cell is induced. As a typical example of the bispecific antibody, blinatumomab is known, which has BiTE (trademark) format (scFv-scFv format), binds to CD3 and CD19 and approved as a therapeutic agent for adult and child's recurrent or refractory B cellular non-Hodgkin's lymphoma and acute lymphocytic leukemia in the USA. However, blinatumomab is short in half-life due to BiTE (trademark) format and produces a large amount of high molecular weights species (HMWS) in predetermined experimental conditions, compared to other antibodies approved as pharmaceuticals (Non Patent Literature 1). In other words, it is suggested that blinatumomab has a problem in stability

It is desired that preclinical studies for pharmaceuticals are carried out by using animals, particularly a higher primate, cynomolgus monkey. In the case of a candidate drug using an anti-CD3 antibody, an anti-CD3 antibody that can bind to CD3 of both a human and cynomolgus monkey is desirably used. When an anti-CD3 antibody, which binds to CD3 of both a human and cynomolgus monkey and recognizes a new epitope identified by X-ray structure analysis, was created, and combined to an antibody to a target antigen to create a bispecific antibody, the bispecific antibody showed cytotoxicity to cancer cells expressing the target antigen (Patent Literatures 1 and 2). Also, anti-CD3 antibody variants have been produced by partly modifying the anti-CD3 antibody (Patent Literature 3). To prepare multispecific antibodies and multispecific molecules composed of various formats as pharmaceuticals, it has been desired to develop various types of anti-CD3 antibody fragments.

### Citation List

### Patent Literatures

[Patent Literature 1] International Publication No. WO2018/117237
[Patent Literature 2] International Publication No. WO2018/147245
[Patent Literature 3] International Publication No. WO2019/244107

### Non Patent Literature

[Non Patent Literature 1] Journal of Chromatography B 1065-1066 (2017) 35-43

### Summary of Invention

### Technical Problem

An object of the present invention is to provide a bispecific molecule excellent in solution stability and reduced in production of high molecular weights species (HMWS) including a dimer when stored in the state of a solution.

### Solution to Problem

The present inventors conducted intensive studies with a view to solve the above problem. As a result, they produced a novel bispecific molecule comprising an anti-CD3 scFv or an anti-CD3 Fab and achieved the present invention.

More specifically, the present invention includes the following inventions.
[1] A Fab that specifically binds to CD3, comprising the following CDRH1 to 3 and CDRL1 to 3:
   CDRH1 consisting of the amino acid sequence represented by SEQ ID NO: 32;
   CDRH2 consisting of the amino acid sequence represented by SEQ ID NO: 33;
   CDRH3 consisting of the amino acid sequence represented by SEQ ID NO: 34;
   CDRL1 consisting of the amino acid sequence represented by SEQ ID NO: 35;
   CDRL2 consisting of the amino acid sequence represented by SEQ ID NO: 36; and
   CDRL3 consisting of the amino acid sequence represented by SEQ ID NO: 37.
[2] The Fab according to [1], comprising a heavy chain variable region consisting of amino acids at positions 1 to 118 in the amino acid sequence represented by SEQ ID NO: 1 and a light chain variable region consisting of amino acids at positions 1 to 107 in the amino acid sequence represented by SEQ ID NO: 2.
[3] The Fab according to [1] or [2], comprising a polypeptide consisting of the amino acid sequence represented by SEQ ID NO: 1 and a light chain consisting of the amino acid sequence represented by SEQ ID NO: 2.
[4] A multispecific molecule comprising the Fab according to any one of [1] to [3], wherein the multispecific molecule specifically binds to a target molecule that is not a complex of a cancer-testis antigen peptide and a major histocompatibility complex (MHC).
[5] The multispecific molecule according to [4], wherein the MHC is a human leukocyte antigen (HLA).
[6] The multispecific molecule according to [4] or [5], comprising an antibody that specifically binds to the target molecule or an antigen binding fragment thereof.
[7] The multispecific molecule according to any one of [4] to [6], wherein the multispecific molecule is a multispecific antibody.
[8] The multispecific molecule according to any one of [4] to [7], wherein the multispecific molecule comprises scFv-Fab-heterodimer Fc.
[9] The multispecific molecule according to any one of [4] to [8], wherein the multispecific molecule is a trispecific antibody.
[10] The multispecific molecule according to any one of [4] to [8], wherein the multispecific molecule is a bispecific antibody.
[11] The multispecific molecule according to any one of [4] to [10], wherein the target molecule is GPRC5D, CD98 or Trop 2.
[12] The multispecific molecule according to any one of [4] to [11], which comprises one polypeptide group selected from the following (i) to (iii);
   (i) a polypeptide comprising an amino acid sequence consisting of the 20-718th amino acid residues of SEQ ID NO: 29 or an amino acid sequence in which one or two amino acids are deleted at the carboxyl terminus of said amino acid sequence, a polypeptide comprising an amino acid sequence consisting of the 20-246th amino acid residues of SEQ ID NO: 9 or an amino acid sequence in which one or two amino acids are deleted at the carboxyl terminus of said amino acid sequence, and a polypeptide comprising an amino acid sequence consisting of the 21-233rd amino acid residues of SEQ ID NO: 12;
   (ii) a polypeptide comprising an amino acid sequence consisting of the 20-717th amino acid residues of SEQ ID NO: 30 or an amino acid sequence in which one or two amino acids are deleted at the carboxyl terminus of said amino acid sequence, a polypeptide comprising an amino acid sequence consisting of the 20-246th amino acid residues of SEQ ID NO: 9 or an amino acid sequence in which one or two amino acids are deleted at the carboxyl terminus of said amino acid sequence, and a polypeptide comprising an amino acid sequence consisting of the 21-233rd amino acid residues of SEQ ID NO: 12; and
   (iii) a polypeptide comprising an amino acid sequence consisting of the 20-713rdh amino acid residues of SEQ ID NO: 31 or an amino acid sequence in which one or two amino acids are deleted at the carboxyl terminus of said amino acid sequence, a polypeptide comprising an amino acid sequence consisting of the 20-246th amino acid residues of SEQ ID NO: 9 or an amino acid sequence in which one or two amino acids are deleted at the carboxyl terminus of said amino acid sequence; and a polypeptide comprising an amino acid sequence consisting of the 21-233rd amino acid residues of SEQ ID NO: 12.
[13] A polynucleotide comprising a nucleotide sequence encoding an amino acid sequence comprised in the multispecific molecule according to any one of [4] to [12].
[14] A vector comprising the polynucleotide according to [13].
[15] A cell comprising the polynucleotide according to [13] or the vector according to [14] or producing the multispecific molecule according to any one of [4] to [12].
[16] A method for producing the multispecific molecule according to any one of [4] to [12], comprising a step of culturing the cell according to [15].
[17] A multispecific molecule obtained by the method according to [16].
[18] A composition comprising the Fab according to any one of [1] to [3], the multispecific molecule according to any one of [4] to [12], the polynucleotide according to [13], the vector according to [14] or the cell according to [15].
[19] A scFv that specifically binds to CD3 and has a heavy chain variable region and a light chain variable region connected via a disulfide bond, the scFv comprising the following CDRH1 to 3 and CDRL1 to 3:
   CDRH1 consisting of the amino acid sequence represented by SEQ ID NO: 32;
   CDRH2 consisting of the amino acid sequence represented by SEQ ID NO: 33;
   CDRH3 consisting of the amino acid sequence represented by SEQ ID NO: 34;
   CDRL1 consisting of the amino acid sequence represented by SEQ ID NO: 35;
   CDRL2 consisting of the amino acid sequence represented by SEQ ID NO: 36; and
   CDRL3 consisting of the amino acid sequence represented by SEQ ID NO: 37.
[20] The scFv according to [19], comprising a heavy chain variable region consisting of amino acids at positions 1 to 118 in the amino acid sequence represented by SEQ ID NO: 3 and a light chain variable region consisting of amino acids at positions 134 to 240 in the amino acid sequence represented by SEQ ID NO: 3.
[21] The scFv according to [19] or [20], consisting of the amino acid sequence represented by SEQ ID NO: 3.
[22] The scFv according to [19], comprising a heavy chain variable region consisting of amino acids at positions 1 to 118 in the amino acid sequence represented by SEQ ID NO: 39 and a light chain variable region consisting of amino acids at positions 134 to 240 in the amino acid sequence represented by SEQ ID NO: 39.
[23] The scFv according to [19] or [22], consisting of the amino acid sequence represented by SEQ ID NO: 39.
[24] A multispecific molecule comprising the scFv according to any one of [19] to [23], wherein the multispecific molecule specifically binds to a target molecule that is not a complex of a cancer-testis antigen peptide and a major histocompatibility complex (MHC).
[25] The multispecific molecule according to [24], wherein the MHC is a human leukocyte antigen (HLA).
[26] The multispecific molecule according to [24] or [25], comprising an antibody that specifically binds to the target molecule or an antigen binding fragment thereof.
[27] The multispecific molecule according to any one of [24] to [26], wherein the multispecific molecule is a multispecific antibody.
[28] The multispecific molecule according to any one of [24] to [27], wherein the multispecific molecule comprises taFv-Fab-heterodimer Fc.
[29] The multispecific molecule according to any one of [24] to [28], wherein the multispecific molecule is a trispecific antibody.
[30] The multispecific molecule according to any one of [24] to [28], wherein the multispecific molecule is a bispecific antibody.
[31] The multispecific molecule according to any one of [24] to [30], wherein the target molecule is GPRC5D, CD98 or Trop 2.
[32] The multispecific molecule according to any one of [24] to [31], which comprises one polypeptide pair selected from the following (i) to (iii);
   (i) a polypeptide comprising an amino acid sequence consisting of the 20-744th amino acid residues of SEQ ID NO: 21 or an amino acid sequence in which one or two amino acids are deleted at the carboxyl terminus of said amino acid sequence, and a polypeptide comprising an amino acid sequence consisting of the 20-246th amino acid residues of SEQ ID NO: 9 or an amino acid sequence in which one or two amino acids are deleted at the carboxyl terminus of said amino acid sequence;
   (ii) a polypeptide comprising an amino acid sequence consisting of the 20-743rd amino acid residues of SEQ ID NO: 23 or an amino acid sequence in which one or two amino acids are deleted at the carboxyl terminus of said amino acid sequence, and a polypeptide comprising an amino acid sequence consisting of the 20-246th amino acid residues of SEQ ID NO: 9, or an amino acid sequence in which one or two amino acids are deleted at the carboxyl terminus of said amino acid sequence; and
   (iii) a polypeptide comprising an amino acid sequence consisting of the 20-739th amino acid residues of SEQ ID NO: 25 or an amino acid sequence in which one or two amino acids are deleted at the carboxyl terminus of said amino acid sequence, and a polypeptide comprising an amino acid sequence consisting of the 20-246th amino acid residues of SEQ ID NO: 9 or an amino acid sequence in which one or two amino acids are deleted at the carboxyl terminus of said amino acid sequence.
[33] A polynucleotide comprising a nucleotide sequence encoding an amino acid sequence comprised in the multispecific molecule according to any one of [24] to [32].
[34] A vector comprising the polynucleotide according to [33].
[35] A cell comprising the polynucleotide according to [33] or the vector according to [34], or producing the multispecific molecule according to any one of [24] to [32].
[36] A method for producing the multispecific molecule according to any one of [24 to [32], comprising a step of culturing the cell according to [25].
[37] A multispecific molecule obtained by the method according to [36].
[38] A composition comprising the scFv according to any one of [19] to [23], the multispecific molecule according to any one of [24] to [32] or [37], the polynucleotide according to [33], the vector according to [34] or the cell according to [35].
[39] A Fab that specifically binds to CD3 and comprises the following CDRH1 to 3 and CDRL1 to 3:
   CDRH1 consisting of the amino acid sequence represented by SEQ ID NO: 40;
   CDRH2 consisting of the amino acid sequence represented by SEQ ID NO: 41;
   CDRH3 consisting of the amino acid sequence represented by SEQ ID NO: 42;
   CDRL1 consisting of the amino acid sequence represented by SEQ ID NO: 43;
   CDRL2 consisting of the amino acid sequence represented by SEQ ID NO: 44; and
   CDRL3 consisting of the amino acid sequence represented by SEQ ID NO: 45.
[40] The Fab according to [39], comprising a heavy chain variable region consisting of amino acids at positions 1 to 118 in the amino acid sequence represented by SEQ ID NO: 46 and a light chain variable region consisting of amino acids at positions 1 to 109 in the amino acid sequence represented by SEQ ID NO: 47.
[41] The Fab according to [39] or [40], comprising a polypeptide consisting of the amino acid sequence represented by SEQ ID NO: 46 and a light chain consisting of the amino acid sequence represented by SEQ ID NO: 47.
[42] A multispecific molecule comprising the Fab according to any one of [39] to [41], wherein the multispecific molecule specifically binds to a target molecule that is not a complex of a cancer-testis antigen peptide and a major histocompatibility complex (MHC).
[43] The multispecific molecule according to [42], wherein the MHC is a human leukocyte antigen (HLA).
[44] The multispecific molecule according to [42] or [43], comprising an antibody that specifically binds to the target molecule or an antigen binding fragment thereof.
[45] The multispecific molecule according to any one of [42] to [44], wherein the multispecific molecule is a multispecific antibody.
[46] The multispecific molecule according to any one of [42] to [45], wherein the multispecific molecule is a bispecific antibody.
[47] A polynucleotide comprising a nucleotide sequence encoding an amino acid sequence comprised in the multispecific molecule according to any one of [42] to [46].
[48] A vector comprising the polynucleotide according to [47].
[49] A cell comprising the polynucleotide according to [47] or the vector according to [48], or producing the multispecific molecule according to any one of [42] to [46].
[50] A method for producing the multispecific molecule according to any one of [344] to [38], comprising a step of culturing the cell according to [49].
[51] A multispecific molecule obtained by the method according to [50].
[52] A composition comprising the Fab according to any one of [39] to [41], the multispecific molecule according to any one of [42] to [46], the polynucleotide according to [47], the vector according to [48] or the cell according to [49.
[53] A scFv that specifically binds to CD3 and has a heavy chain variable region and a light chain variable region connected via a disulfide bond, the scFv comprising the following CDRH1 to 3 and CDRL1 to 3:
   CDRH1 consisting of the amino acid sequence represented by SEQ ID NO: 40;
   CDRH2 consisting of the amino acid sequence represented by SEQ ID NO: 41;
   CDRH3 consisting of the amino acid sequence represented by SEQ ID NO: 42;
   CDRL1 consisting of the amino acid sequence represented by SEQ ID NO: 43;
   CDRL2 consisting of the amino acid sequence represented by SEQ ID NO: 44; and
   CDRL3 consisting of the amino acid sequence represented by SEQ ID NO: 45.
[54] The scFv according to [53], comprising a heavy chain variable region consisting of amino acids at positions 1 to 118 in the amino acid sequence represented by SEQ ID NO: 48 and a light chain variable region consisting of amino acids at positions 134 to 242 in the amino acid sequence represented by SEQ ID NO: 48.
[55] The scFv according to [53] or [54], consisting of the amino acid sequence represented by SEQ ID NO: 48.
[56] The scFv according to [53], comprising a heavy chain variable region consisting of amino acids at positions 1 to 118 in the amino acid sequence represented by SEQ ID NO: 49 and a light chain variable region consisting of amino acids at positions 134 to 242 in the amino acid sequence represented by SEQ ID NO: 49.
[57] The scFv according to [53] or [56], consisting of the amino acid sequence represented by SEQ ID NO: 49.
[58] A multispecific molecule comprising the scFv according to any one of [53] to [57], wherein the multispecific molecule specifically binds to a target molecule that is not a complex of a cancer-testis antigen peptide and a major histocompatibility complex (MHC).
[59] The multispecific molecule according to [58], wherein the MHC is a human leukocyte antigen (HLA).
[60] The multispecific molecule according to [58] or [59], comprising an antibody that specifically binds to the target molecule or an antigen binding fragment thereof.
[61] The multispecific molecule according to any one of [58] to [60], wherein the multispecific molecule is a multispecific antibody.
[62] The multispecific molecule according to any one of [58] to [61], wherein the multispecific molecule is a bispecific antibody.
[63] A polynucleotide comprising a nucleotide sequence encoding an amino acid sequence comprised in the multispecific molecule according to any one of [58] to [62].
[64] A vector comprising the polynucleotide according to [63].
[65] A cell comprising the polynucleotide according to [63] or the vector according to [64], or producing the multispecific molecule according to any one of [58] to [62].
[66] A method for producing the multispecific molecule according to any one of [58] to [62], comprising a step of culturing the cell according to [65].
[67] A multispecific molecule obtained by the method according to [66].
[68] A composition comprising the scFv according to any one of [53] to [57], the multispecific molecule according to any one of [58] to [62], the polynucleotide according to [63], the vector according to [64] or the cell according to [65]
[69] A pharmaceutical composition comprising the multispecific molecule according to any one of [4] to [12], [24] to [32], [42] to [46] and [58] to [62].

This description includes the contents as disclosed in the Japanese Patent Application No. 2021-055375 and 2021-157580, which are priority documents of the present application. Advantageous Effects of Invention

According to the present invention, it is possible to obtain a novel bispecific antibody (bispecific molecule), which binds to a scFv that binds CD3 and has a heavy chain variable region and a light chain variable region connected via a disulfide bond or a Fab that binds CD3, and which binds to a target molecule that is not a complex of a cancer-testis antigen peptide and a human leukocyte antigen (HLA). The antibody has a high stability in a solution and can be stored in a solution state without deterioration.

### Brief Description of Drawings

[Figure 1] Figure 1 shows time-course change of HMWS(%) with respect to C3E-0002 to 0004.
[Figure 2] Figure 2A shows time-course change (degradation) of HMWS(%) by shaking stress with respect to C3E-0002 to 0004, and Figure 2B shows time-course changes (deterioration) (%) of peak area by shaking stress with respect to C3E-0002 to 0004.
[Figure 3] Figure 3A shows time-course change of HMWS(%) with respect to RX3-0001 to 0003; Figure 3B shows time-course change of HMWS(%) with respect to CX3-0001 to 0003; and Figure 3C shows time-course change of HMWS(%) with respect to TX3-0001 to 0003.
[Figure 4-1] Figure 4-1A shows time-course change (degradation) of HMWS(%) by shaking stress with respect to RX3-0001 to 0003, and Figure 4-1B shows time-course change (degradation) of HMWS(%) in peak area by shaking stress with respect to RX3-0001 to 0003.
[Figure 4-2] Figure 4-2A shows time-course change (degradation) of HMWS(%) by shaking stress with respect to CX3-0001 to 0003; and Figure 4-2B shows time-course change (degradation) of HMWS(%) in peak area by shaking stress with respect to CX3-0001 to 0003.
[Figure 4-3] Figure 4-3A shows time-course change (degradation) of HMWS(%) by shaking stress with respect to TX3-0001 to 0003; and Figure 4-3B shows time-course change (degradation) of HMWS(%) in peak area by shaking stress with respect to TX3-0001 to 0003.
[Figure 5] Figure 5 shows the amino acid sequence (SEQ ID NO: 1) of C3E-7085-Fab.
[Figure 6] Figure 6 shows the amino acid sequence (SEQ ID NO: 2) of C3E-7085-LC.
[Figure 7] Figure 7 shows the amino acid sequence (SEQ ID NO: 3) of C3E-7096.
[Figure 8] Figure 8 shows the full-length nucleotide sequence (SEQ ID NO: 4) of HC_h.
[Figure 9] Figure 9 shows the full-length nucleotide sequence (SEQ ID NO: 5) of C3E-7085-HC-k.
[Figure 10] Figure 10 shows the full-length nucleotide sequence (SEQ ID NO: 6) of C3E-7085-Fab-HC-k.
[Figure 11] Figure 11 shows the full-length nucleotide sequence (SEQ ID NO: 7) of C3E-7085-LC.
[Figure 12] Figure 12 shows the full-length nucleotide sequence (SEQ ID NO: 8) of C3E-7096-HC-k.
[Figure 13] Figure 13 shows the full-length amino acid sequence (SEQ ID NO: 9) of HC_h.
[Figure 14] Figure 14 shows the full-length amino acid sequence (SEQ ID NO: 10) of C3E-7085-HC-k.
[Figure 15] Figure 15 shows the full-length amino acid sequence (SEQ ID NO: 11) of C3E-7085-Fab-HC-k.
[Figure 16] Figure 16 shows the full-length amino acid sequence (SEQ ID NO: 12) of C3E-7085-LC.
[Figure 17] Figure 17 shows the full-length amino acid sequence (SEQ ID NO: 13) of C3E-7096-HC-k.
[Figure 18] Figure 18 shows the full-length nucleotide sequence (SEQ ID NO: 14) of TX3-0001-HC_k.
[Figure 19] Figure 19 shows the full-length nucleotide sequence (SEQ ID NO: 15) of TX3-0003-HC_k.
[Figure 20] Figure 20 shows the full-length nucleotide sequence (SEQ ID NO: 16) of CX3-0001-HC_k.
[Figure 21] Figure 21 shows the full-length nucleotide sequence (SEQ ID NO: 17) of CX3-0003-HC_k.
[Figure 22] Figure 22 shows the full-length nucleotide sequence (SEQ ID NO: 18) of RX3-0001-HC_k.
[Figure 23] Figure 23 shows the full-length nucleotide sequence (SEQ ID NO: 19) of CX3-0001-HC_k.
[Figure 24] Figure 24 shows the full-length amino acid sequence (SEQ ID NO: 20) of TX3-0001-HC_k.
[Figure 25] Figure 25 shows the full-length amino acid sequence (SEQ ID NO: 21) of TX3-0003-HC_k.
[Figure 26] Figure 26 shows the full-length amino acid sequence (SEQ ID NO: 22) of CX3-0001-HC_k.
[Figure 27] Figure 27 shows the full-length amino acid sequence (SEQ ID NO: 23) of CX3-0003-HC_k.
[Figure 28] Figure 28 shows the full-length amino acid sequence (SEQ ID NO: 24) of RX3-0001-HC_k.
[Figure 29] Figure 29 shows the full-length amino acid sequence (SEQ ID NO: 25) of RX3-0003-HC_k.
[Figure 30] Figure 30 shows the full-length nucleotide sequence (SEQ ID NO: 26) of TX3-0002-HC_k.
[Figure 31] Figure 31 shows the full-length nucleotide sequence (SEQ ID NO: 27) of CX3-0002-HC_k.
[Figure 32] Figure 32 shows the full-length nucleotide sequence (SEQ ID NO: 28) of RX3-0002-HC_k.
[Figure 33] Figure 33 shows the full-length amino acid sequence (SEQ ID NO: 29) of TX3-0002-HC_k.
[Figure 34] Figure 34 shows the full-length amino acid sequence (SEQ ID NO: 30) of CX3-0002-HC_k.
[Figure 35] Figure 35 shows the full-length amino acid sequence (SEQ ID NO: 31) of RX3-0002-HC_k.
[Figure 36] Figure 36 shows the amino acid sequences (SEQ ID NO: 32 to 37) of heavy chain CDRH1 to CDRH3 and light chain CDRL1 to L3 of C3E-7085.
[Figure 37] Figure 37 shows the amino acid sequence (SEQ ID NO: 38) of a peptide linker.
[Figure 38A] Figure 38A shows a scFv that binds to CD3 and has a heavy chain variable region and a light chain variable region connected via a disulfide bond. (The black bar between VH and VL indicates that both are disulfide bonded. The same applies hereafter.)
[Figure 38B] Figure 38B shows a Fab that binds to CD3.
[Figure 38C] Figure 38C shows bispecific molecules comprising: a scFv (hatched lines from top right to bottom left) that binds to CD3 and has a heavy chain variable region and a light chain variable region connected via a disulfide bond; and X (another binding portion) that binds to a molecule other than CD3. The orientation of the scFv indicated by hatched lines from top right to bottom left may be opposite, with the order of linkage of the VH and VL domains swapped. A scFv formed via a disulfide bond may be constituted of VH and VL not via a linker.
[Figure 38D] Figure 38D shows bispecific molecules comprising a Fab that binds to CD3 and X ("another binding portion") that binds to a molecule other than CD3.
[Figure 38E] Figure 38E shows a tandem scFv (taFv) having a scFv that binds to CD3 and a scFv that binds to a molecule other than CD3 connected via a linker. The orientation of each scFv may be opposite, with the order of linkage of the VH and VL domains swapped.
[Figure 38F] Figure 38F shows a scFv-Fabs each having a Fab that binds to CD3 and a scFv that binds to a molecule other than CD3 connected via a linker. The orientation of each scFv may be opposite, with the order of linkage of the VH and VL domains swapped.

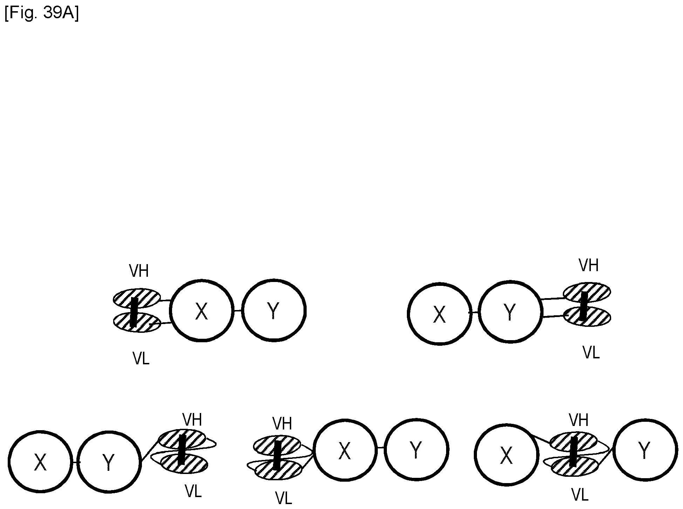

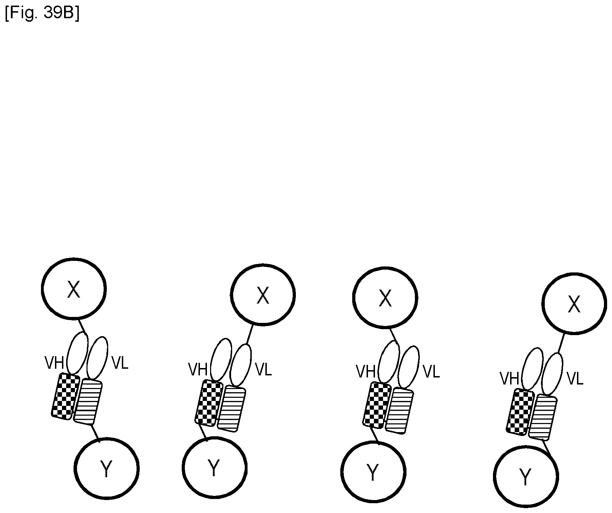

[Figure 39A] Figure 39A shows multispecific molecules each comprising a scFv that binds to CD3 and X ("another binding portion") and Y ("another binding portion" or "another group") that binds to a molecule other than CD3. The orientation of scFv shown by hatched lines from top right to bottom left may be opposite, with the order of linkage of the VH and VL domains swapped. X and Y may be various antibody fragments such as scFv, VHH and Fab or complete antibodies. In scFv bound via a disulfide bond, VH and VL may be constituted not via a linker. If Y is "another binding portion", the case shows a trispecific molecule.
[Figure 39B] Figure 39B shows multispecific molecules each comprising a Fab that binds to CD3 and X ("another binding portion") and Y ("another binding portion" or "another group") that each bind to a molecule other than CD3. X and Y ("another binding portion") may be various antibody fragments such as scFv, VHH and Fab, complete antibodies or molecules except immunoglobulins. If Y is "another binding portion", the case shows a trispecific molecule.

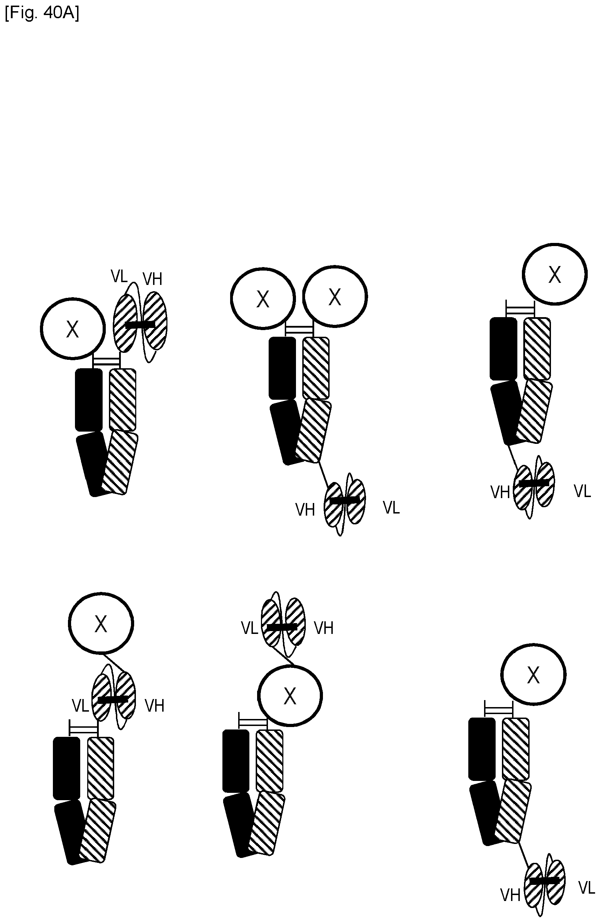

[Figure 40A] Figure 40A shows a Fc-added bispecific molecule comprising: a scFv (hatched lines from top right to bottom left) that binds to CD3 and has a heavy chain variable region and a light chain variable region connected via a disulfide bond; and X ("another binding portion") that binds to a molecule other than CD3. Fc has a format having a first polypeptide (indicated by hatched lines from top left to bottom right) and a second polypeptide painted in black associated (Fc may be a wild type or a mutant). The orientation of each scFv indicated by hatched lines from top right to bottom left may be opposite, with the order of linkage of the VH and VL domains swapped. Note that, in each figure, the positions of X and scFv may be interchanged.

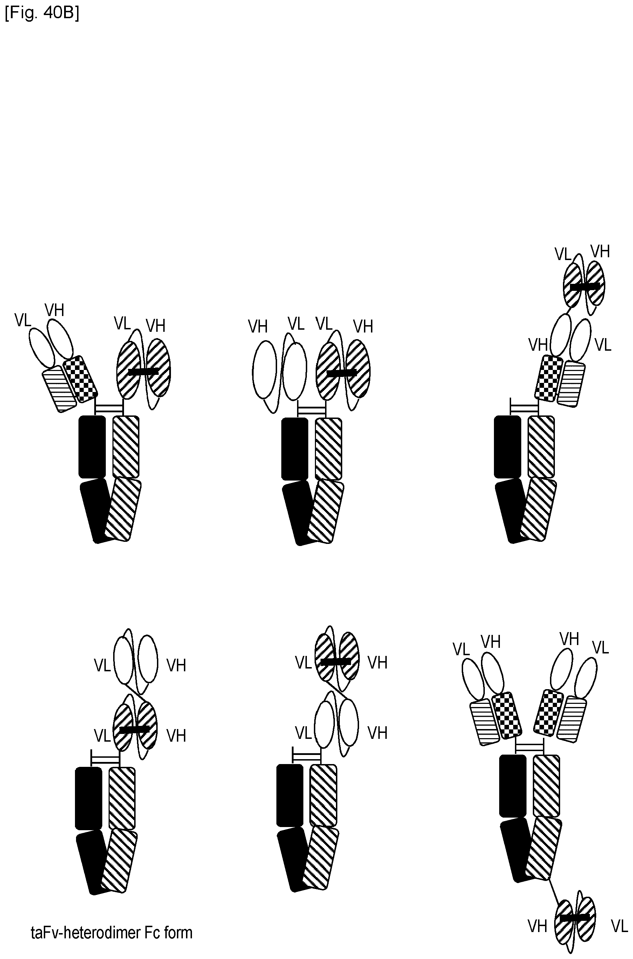

[Figure 40B] Figure 40B shows a Fc-added bispecific molecule comprising a scFv (hatched lines from top right to bottom left) that binds to CD3 and has a heavy chain variable region and a light chain variable region connected via a disulfide bond; and Fab or scFv that binds to a molecule other than CD3. Fc has a format having a first polypeptide indicated by hatched lines from top left to bottom right and a second polypeptide painted in black associated (Fc may be a wild type or a mutant). The orientation of each scFv may be opposite, with the order of linkage of the VH and VL domains swapped. The lower-left figure shows a taFv-heterodimer Fc-format (in the case Fc is a heterodimer).

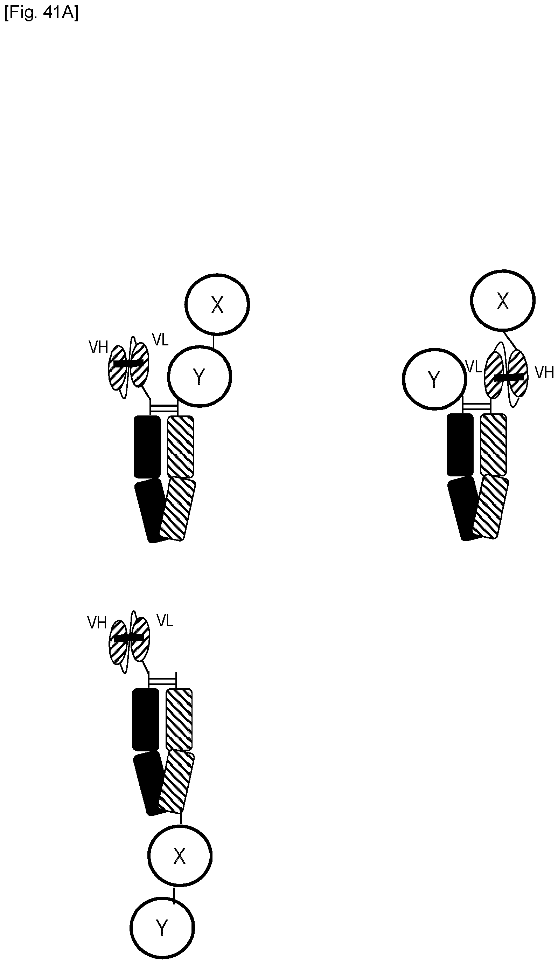

[Figure 41A] Figure 41A shows a Fc-added multispecific molecule comprising: a scFv (hatched lines from top right to bottom left) that binds to CD3 and has a heavy chain variable region and a light chain variable region connected via a disulfide bond; and X ("another binding portion") and Y ("another binding portion" or "another group") that each bind to a molecule other than CD3. Fc has a format having a first polypeptide indicated by hatched lines from top left to bottom right and a second polypeptide painted in black associated (Fc may be a wild type or a mutant). The orientation of scFv indicated by hatched lines from top right to bottom left may be opposite, with the order of linkage of the VH and VL domains swapped. If Y is "another binding portion", the case shows a trispecific molecule. Note that, in each figure, the positions of X, Y and scFv may be interchanged.

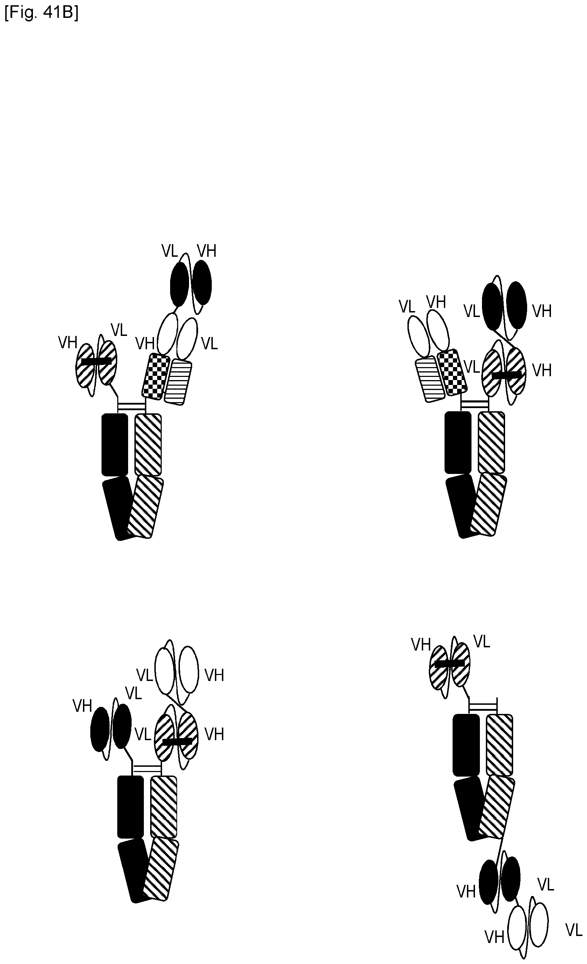

[Figure 41B] Figure 41B shows a Fc added trispecific molecule comprising a scFv (hatched lines from top right to bottom left) that binds to CD3 and has a heavy chain variable region and a light chain variable region connected via a disulfide bond, and two antibody fragments (Fab or scFv) that binds to a molecule other than CD3. Fc has a format having a first polypeptide indicated by hatched lines from top left to bottom right and a second polypeptide painted in black associated (Fc may be a wild type or a mutant). The orientation of each scFv may be opposite, with the order of linkage of the VH and VL domains swapped.

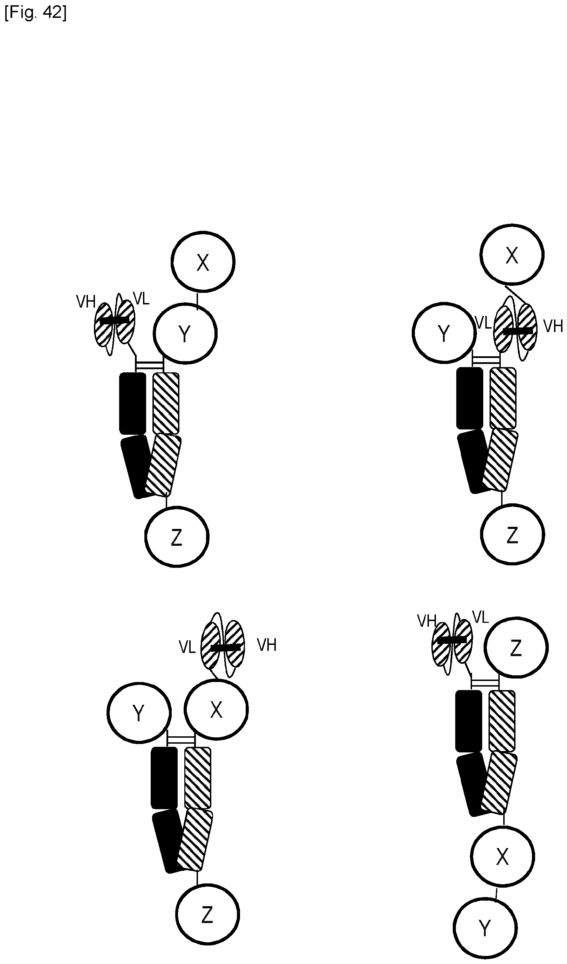

[Figure 42] Figure 42 shows a Fc-added multispecific molecule comprising: a scFv that binds to CD3; a scFv (hatched lines from top right to bottom left) having a heavy chain variable region and a light chain variable region connected via a disulfide bond; and "another binding portion" or "another group" that binds to a molecule other than CD3 (represented by X, Y and Z). Fc has a format having a first polypeptide indicated by hatched lines from top left to bottom right and a second polypeptide painted in black associated (Fc may be a wild type or a mutant). The orientation of the scFv indicated by hatched lines from top right to bottom left may be opposite, with the order of linkage of the VH and VL domains swapped. If two or three elements of X, Y and Z are each "another binding portion", the case shows a trispecific molecule or a quadruple-specific molecule. Note that, in each figure, the positions of X, Y, Z and scFv may be interchanged.
[Figure 43A] Figure 43A shows a Fc-added bispecific molecule comprising a Fab that binds to CD3 and X (another binding portion) that binds to a molecule other than CD3. Fc has a format having a first polypeptide indicated by hatched lines from top left to bottom right and a second polypeptide painted in black associated (Fc may be a wild type or a mutant).
[Figure 43B] Figure 43B shows a Fc-added bispecific molecule comprising a Fab that binds to CD3 and an antibody fragment (scFv or Fab) that binds to a molecule other than CD3. Fc has a format having a first polypeptide indicated by hatched lines from top left to bottom right and a second polypeptide painted in black associated (Fc may be a wild type or a mutant). The upper left figure shows a scFv-Fab-heterodimer Fc-format (in the case Fc is a heterodimer). The orientation of each scFv may be opposite, with the order of linkage of the VH and VL domains swapped.
[Figure 44A] Figure 44A shows a Fc-added multispecific molecule comprising a Fab that binds to CD3, and X ("another binding portion") and Y ("another binding portion" or "another group") that each bind to a molecule other than CD3. Fc has a format having a first polypeptide indicated by hatched lines from top left to bottom right and a second polypeptide painted in black associated (Fc may be a wild type or a mutant). If Y is "another bonding molecule", the case shows a trispecific molecule.
[Figure 44B] Figure 44B shows a Fc-added trispecific molecule (bispecific molecule if two antibody fragments are identical) comprising a Fab that binds to CD3 and two antibody fragments (scFv or Fab) that each bind to a molecule other than CD3. Fc has a format having a first polypeptide indicated by hatched lines from top left to bottom right and a second polypeptide painted in black associated (Fc may be a wild type or a mutant). The orientation of each scFv may be opposite, with the order of linkage of the VH and VL domains swapped.

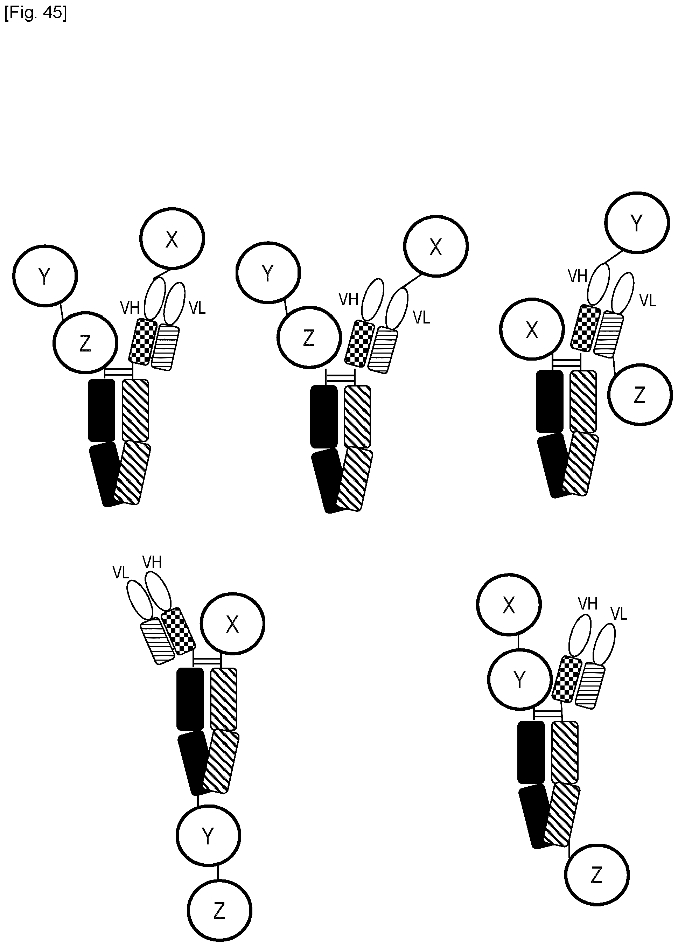

[Figure 45] Figure 45 shows a Fc-added multispecific molecule comprising a Fab that binds to CD3 and "another binding portion" or "another group" (each represented by X, Y and Z, at least one of which is "another binding molecule") that binds to a molecule other than CD3. Fc has a format having a first polypeptide indicated by hatched lines from top left to bottom right and a second polypeptide painted in black associated (Fc may be a wild type or a mutant). If two and three elements of X, Y and Z are "another binding site", the cases show a trispecific molecule and a quadruple-specific molecule, respectively.
[Figure 46] Figure 46 shows the amino acid sequence (SEQ ID NO: 39) of C3E-7097.
[Figure 47] Figure 47 shows the amino acid sequences (SEQ ID NO: 40 to 45) of heavy chain CDRH1 to CDRH3 and light chain CDRL1 to L3 of C3E-7093.
[Figure 48] Figure 48 shows the amino acid sequence (SEQ ID NO: 46) of C3E-7093-Fab.
[Figure 49] Figure 49 shows the amino acid sequence (SEQ ID NO: 47) of C3E-7093-LC.
[Figure 50] Figure 50 shows the amino acid sequence (SEQ ID NO: 48) of C3E-7098.
[Figure 51] Figure 51 shows the amino acid sequence (SEQ ID NO: 49) of C3E-7099.

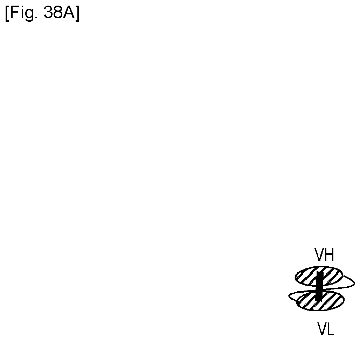

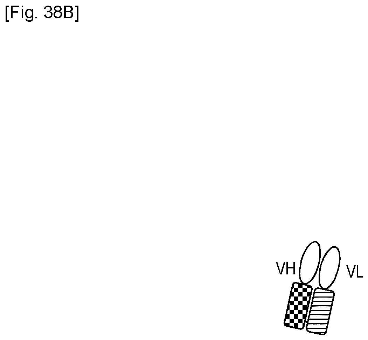

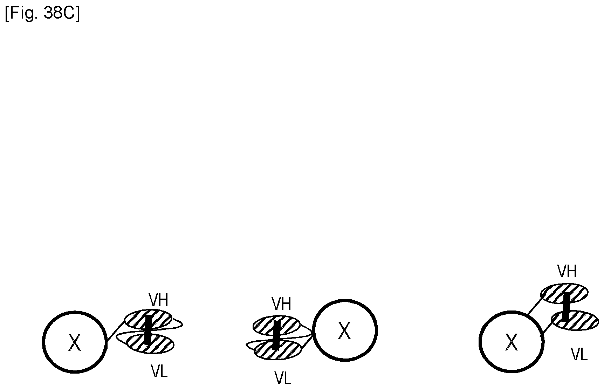

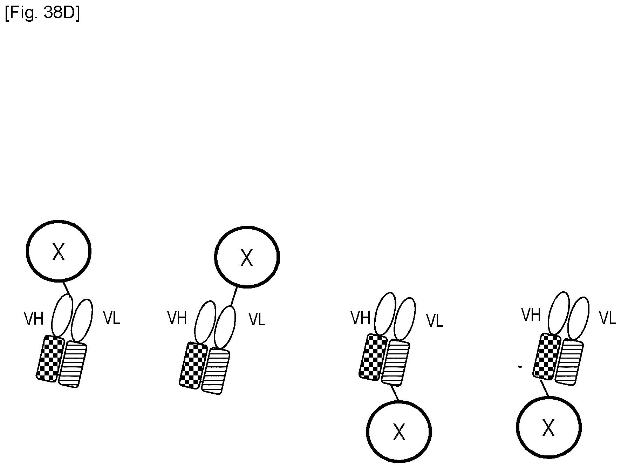

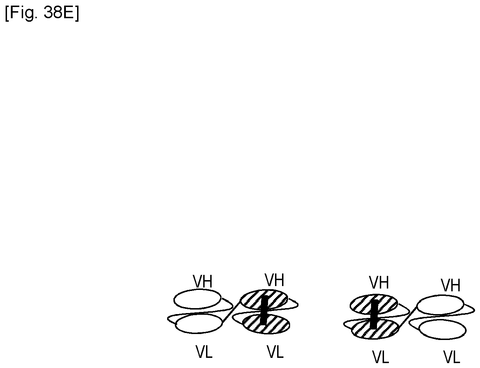

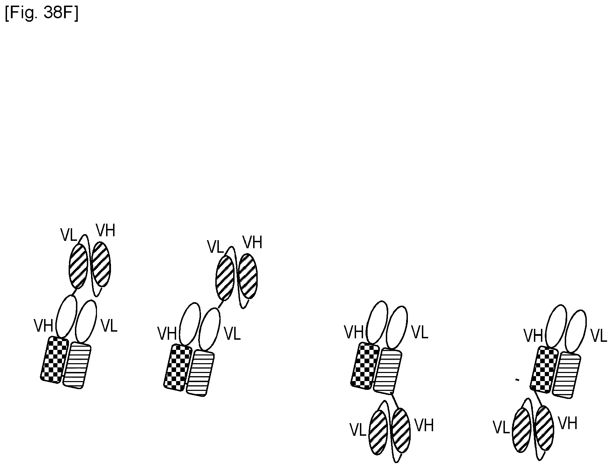

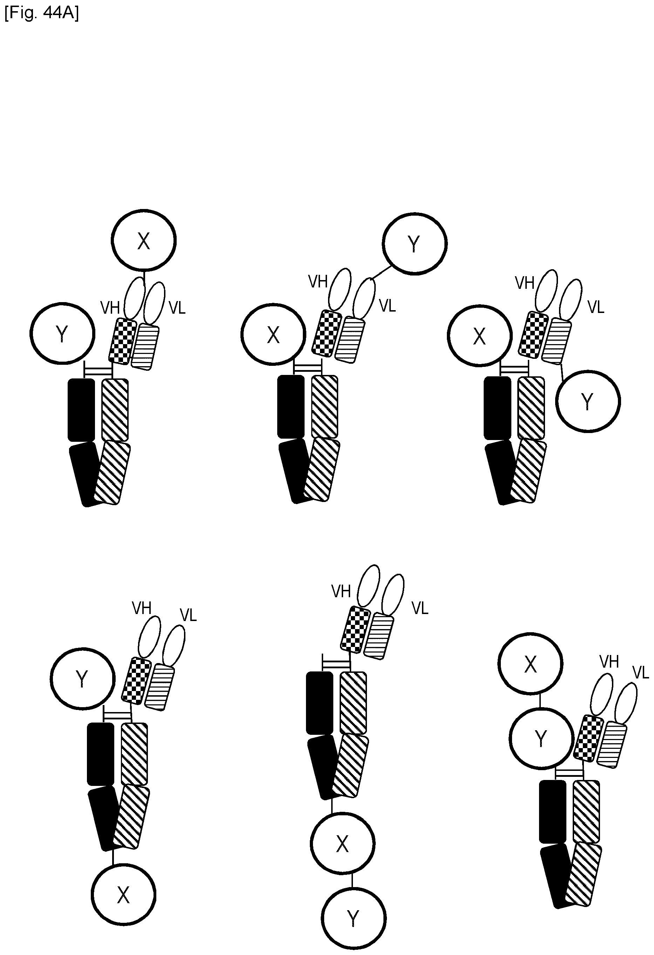

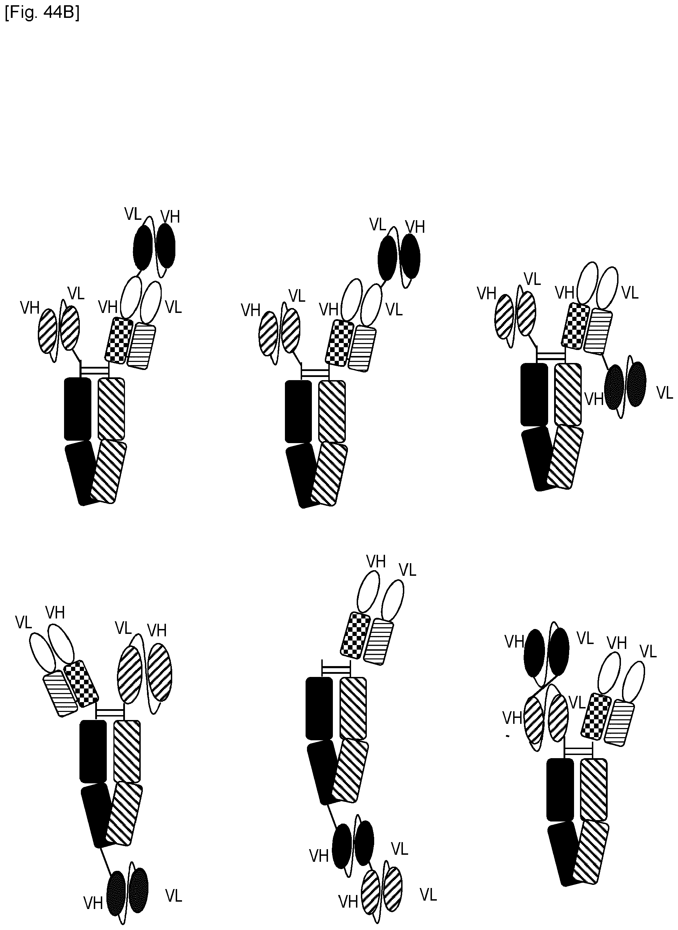

In Figures 38 to 45, X, Y and Z appear to be in contact with the ends of the portions in adjacent to them but binding or fusion to the end is not essential. For example, when X, Y and Z are (appear to) in contact with a Fc, they may be bound or fused to the N or C terminal of the Fc; bound to portions except the N or C terminal of the Fc; or bound or fused to a sugar chains added to amino acids comprised in the Fc. The cases of not binding or fusing to the ends of portions in adjacent to them are not limited to these.

### Description of Embodiments

Now, the present invention will be more specifically described.

### 1. Definition

In the present invention, the term "gene" refers to a nucleotide chain comprising a nucleotide sequence encoding the amino acid of a protein or a complementary strand thereof. Examples of the "gene" include, a nucleotide chain comprising a nucleotide sequence encoding the amino acid of a protein or a complementary strand thereof, such as a polynucleotide, an oligonucleotide, DNA, mRNA, cDNA and cRNA. The gene is a single stranded, double stranded or three or more stranded nucleotide chain. A DNA chain/RNA chain associate, a single nucleotide chain on which a ribonucleotide (RNA) and a deoxyribonucleotide (DNA) are present in combination, and a double stranded or triple (or more) stranded nucleotide comprising such a nucleotide chain are included in the sense of the "gene". In the present invention, the term "base sequence" is synonymous with the term "nucleotide sequence"..

In the present invention, "polynucleotide", "nucleotide chain", "nucleic acid" and "nucleic acid molecule" are interchangeably used. For example, DNA, RNA, a probe, an oligonucleotide and a primer are included in the sense of "polynucleotide". The polynucleotide is a polynucleotide composed of a single strand, double strands or three or more strands. A DNA chain/RNA chain associate, a single polynucleotide chain on which a ribonucleotide (RNA) and a deoxyribonucleotide (DNA) are present in combination, and a double stranded or triple (or more) stranded associate comprising such a polynucleotide chain are included in the sense of the "polynucleotide".

In the present invention, the terms of "polypeptide", "peptide" and "protein" are interchangeably used.

In the present invention, the term "antigen" is sometimes used in the sense of "immunogen".

In the present invention, examples of the "cell" include various cells derived from animal individuals, subcultured cells, primary cultured cells, cell strains, recombinant cells and microorganisms.

In the present invention, the term "antibody" refers to an immunoglobulin having a constant region and a variable region. The antibody is not particularly limited as to whether it is a natural immunoglobulin or an immunoglobulin partially or completely synthesized.

A basic antibody is constituted of four chains, that is, two identical light chains (L chains) and two identical heavy chains (H chains). A light chain binds to a heavy chain via a single disulfide bond. Two heavy chains mutually bind via a single or a plurality of disulfide bonds depending on the isotype of heavy chains. Each of the light chains and heavy chains has disulfide bonds arranged in regular intervals within the chain. The heavy chain and light chain have constant regions high in similarity of amino acid sequence and variable regions low in similarity of amino acid sequence. The light chain has a variable region (VL) and a constant region (CL) sequentially in the order from the amino terminal. The heavy chain has a variable region (VH) and three constant regions (CH1/CH2/CH3) sequentially in the order from the amino terminal. VL and VH are paired and CL is aligned with the first constant region (CH1) of the heavy chain. VL and VH are paired to form a single antigen binding site.

The constant region of the antibody of the present invention is not particularly limited. However, as the antibody of the present invention for treating or preventing diseases of humans, a constant region of a human antibody is suitably used. Examples of the heavy-chain constant region of a human antibody include Cγ1, Cγ2, Cγ3, Cγ4, Cµ, Cδ, Cα1, Cα2 and Cε. Examples of the light chain constant region of a human antibody include Cκ and Cλ.

A Fab is composed of VH of a heavy chain followed by CH1 and VL of a light chain followed by CL. VH and VL comprise complementarity determining regions (CDR). A linker or a linking portion may be present between VH and CH1 and between VL and CL.

Fc (also referred to as a Fc region) is a carboxyl-terminal region of a heavy-chain constant region and a dimer comprising CH2 and CH3. The Fc of the present invention may be Fc having a natural sequence or a mutant-type Fc (referred to as mutant Fc) having a mutation in the natural sequence. A preferable Fc region of each of a multispecific molecule and bispecific molecule of the present invention is a mutant Fc and more preferable Fc region is a pair of Fc regions that can form a hetero dimer. Examples of the pair of Fc regions include a combination of Fc (i) comprised in a first polypeptide and Fc (ii) comprised in a second polypeptide. The pair of Fc regions is not limited to the combination of Fc (i) and Fc (ii) as long as a pair of Fc regions can be associated (to form a hetero dimer).

Examples of the mutant Fc include, but are not limited to, a modified Fc region (comprising a heterodimer Fc region) comprised in a heteromultimer having an improved stability and disclosed in WO2013/063702; a Fc comprising an immunoglobulin CH3 region induced from an IgG antibody having "protuberances" and "cavities" comprised in a heteromultimer and disclosed in WO96/27011; a Fc comprising CH3 domain comprised in an electrostatically advantageous heterodimer prepared by replacing one or more amino acid residues by charged amino acids and disclosed in WO2009/089004; a heterodimer Fc region comprised in a heterodimer using a three-dimensional structural mutation and/or pI (isoelectric point) mutation and disclosed in WO2014/110601; and a heterodimer Fc comprising a CH3 domain having a modification for eliminating or reducing binding to protein A, and disclosed in WO2010/151792.

The variable region is composed of extremely variable regions called hypervariable regions (HVR) and relatively less variable regions called framework regions (FR) separated by the HVRs. A naturally occurring variable regions of a heavy chain and a light chain each comprise four FRs connected via three hypervariable regions. The hypervariable regions of each chain are held by FRs in the close vicinity of the hypervariable regions of the other chain to contribute to the formation of an antigen binding site of the antibody.

It is known that a heavy chain and light chain of an antibody molecule each have three complementarity determining regions (CDR). The complementarity determining regions are also called hypervariable regions, which are sites positioned within the variable region of a heavy chain and light chain of an antibody and having a highly mutational primary structure. Three complementarity determining regions are usually non-consecutively arranged on the primary structures of polypeptide chains of a heavy chain and a light chain. In the present invention, complementarity determining regions of an antibody are expressed as follows: the complementarity determining regions of a heavy chain are expressed as CDRH1, CDRH2, CDRH3 sequentially in the order from the amino terminal of the heavy chain amino acid sequence, whereas, the complementarity determining regions of a light chain are expressed as CDRL1, CDRL2, CDRL3 sequentially in the order from the amino terminal of the light chain amino acid sequence. These regions are three-dimensionally closely positioned to each other to determine a specificity to the antigen to be bound.

In the present invention, the positions and lengths of CDRs were determined based on the definition of IMGT (Developmental and Comparative Immunology 27 (2003) 55-77).

FRs are present in a variable region except CDR residues. The variable region usually has four FRs: FR1, FR2, FR3 and FR4.

CDRs and FRs comprised in a heavy chain and a light chain are arranged in the order of FRH1-CDRH1-FRH2-CDRH2-FRH3-CDRH3-FRH4, and FRL1-CDRL1-FRL2-CDRL2-FRL3-CDRL3-FRL4, from the amino terminal to the carboxyl terminal of the respective chains.

The positions of CDRs and FRs can be determined based on various definitions known in the technical field, not only IMGT but also Kabat, Chothia, AbM and Contact.

In the present invention, the "site" that an antibody is bound to, more specifically, the "site" that an antibody recognizes, refers to a partial peptide or a part of a higher-order structure on the antigen that an antibody is bound to or recognizes.

In the present invention, the site is also referred to as an epitope or an antibody-binding site. In the present invention, a "variant antibody" refers to a polypeptide which has an amino acid sequence with substitution, deletion, addition (including insertion) (hereinafter collectively referred to as "variation") of amino acids in the original amino acid sequence of the antibody and binds to a target antigen of the present invention. The number of variant amino acids in the variant antibody may be 1, 2, 3, 4, 5, 6, 7, 8, 9, 10, 12, 15, 20, 25, 30, 40 or 50. The variant antibody is included in the "antibody" of the present invention.

In the present invention, the term "several" in the phrase of "one or several" refers to 2 to 10.

In the specification, the term "molecule" refers to a molecule including an antibody as mentioned above and an antigen binding fragment of the antibody, and a multispecific molecule formed of an antibody or a plurality of antigen binding fragments derived from the antibody.

In the specification, the phrases "molecule that is multi specific", "multispecific molecule" and "molecule having multi specificity" are interchangeably used. The multi specific molecule is not particularly limited as long as it is a molecule that can bind to a plurality of epitopes mutually different and present in a single molecule and/or mutually different epitopes on two or more molecules. Examples of the multispecific molecule include an antibody comprising heavy chain variable regions (VH) and light chain variable regions (VL). In the present invention, such a molecule having multi specificity will be sometimes referred to as, e.g., a "multispecific antibody" or an "antibody having multi specificity" . "Recognizing two or more antigens" means "binding to multiple mutually distinct epitopes on one molecule" or "binding to mutually distinct epitopes on two or more molecules."

In the specification, aggregates are formed by the aggregation of proteins, and the analysis method for detecting aggregates varies depending on the particle size. An aggregate having a particle size of 100 nm or less can be evaluated by size exclusion chromatography such as HPLC. Subvisible particles (particle size of 0.1 to 10 µm) having a particle size of 100 nm or more and insoluble fine particles (particle size of 10 µm or more) can be evaluated by use of, e.g., Microflow Imaging.

In the specification, the term "HMWS" is an abbreviation of a high molecular weight species and collectively refers to protein aggregate fractions, in a chromatogram, which are obtained by size fraction, such as size exclusion chromatography, and contain larger size proteins than in a fraction constituting a main peak of a desired protein. The term "HMWS (%)" refers to a value obtained by subjecting a solution sample containing a desired protein to size exclusion chromatography such as HPLC, monitoring an elution pattern at a detection wavelength of 280 nm and calculating the area ratio of a peak fraction that appears in a higher molecule side of a main peak fraction of a desired protein relative to the total peak area in the chromatogram by a percentage method.

### 2. Antigen

### 2-1. CD3 antigen

In the present invention, the term "CD3" is interchangeably used with the term "CD3 protein".

CD3 is expressed on T cells as a part of a multi-molecule T cell receptor complex. CD3 is a complex of five types of polypeptides, i.e., γ chain, δ chain, ε chain, ζ chain and η chain (having a molecular weight of 25000-28000, 21000, 20000, 16000 and 22000, sequentially in this order).

The CD3 complex includes γ, δ, ε, ζ and η chains. These chains are referred to as subunits. An anti-CD3 antibody binds to a T cell and activates the T cell, thereby inducing cytotoxicity. Anti-CD3 antibodies mostly bind to CD3ε.

The nucleotide sequence of cDNA encoding human CD3ε is registered in the NCBI/GenBank under an accession number: NM_000733 (NM_000733.3); and the amino acid sequence of human CD3ε is registered in the NCBI/GenPept under an accession number: NP_000724 (NM_000724. 1). The nucleotide sequence of cDNA encoding cynomolgus monkey CD3 is registered in the GenBank under an accession number: NM_001283615.1.

### 2-2. Target molecule

In the present invention, a target molecule is not particularly limited as long as it is a molecule other than CD3 and present in a living body, and preferably a molecule expressed on the surface of a target cell for therapy (e.g., cancer cell, stromal cell and immunosuppressive cell, but not limited thereto) and producing cytotoxic activity to the cancer cell by redirection of CD3 expression T cells, and more preferably, a molecule that is not a complex of a cancer-testis antigen peptide and a major histocompatibility complex (MHC).

The cancer-testis antigen peptide is a cancer cell surface antigen peptide such as a MAGE peptide, a XAGE peptide and a LAGE family peptide. Examples of the MHC include a human leukocyte antigen (HLA) and mouse H-2. The HLAs are classified into class-1 HLA and class-2 HLA. Examples of class-1 HLA include HLA-A, HLA-B and HLA-C. Examples of class-2 HLA include HLA-DR, HLA-DP and HLA-DQ. Examples of the complex of a cancer-testis antigen peptide and a human leukocyte antigen (HLA) include a triple complex of HLA-A2/MAGE-A3, A4, A10 or HLA-A2/LAGE, which is composed of (1) HLA-A2, (2) β2-microglobulin, and (3) MAGE-A3, A4, A10 or LAGE peptide (for example, amino acid No. 157 to 165 of UniProtKB-P78358 (CTG1B HUMAN)).

In the present invention, as a suitable target molecule that is not a complex of a cancer-testis antigen peptide and a human leukocyte antigen (HLA), a cancer antigen peptide or a cancer antigen is preferred. Specific examples thereof include WT1, gp100, p53, KRAS, CD19, CD20, CD33, CD37, CD38, CD98, CD123, EGFR, HER2, HER3, HER4, EpCAM, CEA, PSMA, B7-H3, Trop-2, BCMA and GPRC5D. Examples of the target molecule to stromal cells include FAP, CDH17 and LRRC15. Examples of the target molecule to bone marrow-derived immunosuppressive cells (MDSC) include CD33.

### 2-3. Preparation of antigen

The aforementioned antigen proteins to be used in the present invention, CD3 and another antigen protein, i.e., a target molecule that is not a complex of a cancer-testis antigen peptide and a human leukocyte antigen (HLA), can be prepared through, e.g., isolation/purification from an animal tissue (including body fluid), cells derived from the animal tissue or a culture of the cells; gene recombination; *in-vitro* translation; and chemosynthesis.

The cDNA of the antigen protein can be obtained, for example, by a polymerase chain reaction (hereinafter referred to as "PCR") (Saiki, R. K., et al., Science (1988) 239, 487-49) called a PCR method using a cDNA library of an organ expressing mRNA of the antigen protein as a template, and primers specifically amplifying cDNA of the antigen protein.

A polynucleotide, which is hybridized with a polynucleotide consisting of a nucleotide sequence complementary to a nucleotide sequence encoding an antigen protein expressed in a human or a rat in stringent conditions and which encodes a protein having the same biological activity as the antigen protein, is also included in cDNA of the antigen protein. Also, a splicing variant transcribed from a locus of the antigen protein expressed in a human or a rat, or a polynucleotide that is to be hybridized with the splicing variant and that encodes a protein having the same biological activity as the antigen protein, is included in cDNA of the antigen protein.

A nucleotide sequence encoding a protein, which consists of an amino acid sequence having a substitution, deletion or addition of one to several amino acids in the amino acid sequence of the antigen protein of a human or a rat, or the amino acid sequence prepared by removing a signal sequence from the amino acid sequence of the antigen protein, and which has the same biological activity as the antigen protein, is also included in the nucleotide sequence of the antigen protein gene.

A protein, which consists of the amino acid sequence encoded by a splicing variant transcribed from a locus of the antigen protein of a human or a rat or an amino acid sequence having a substitution, deletion or addition of one to several amino acids in the amino acid sequence, and which has the same biological activity as the antigen protein, is also included in the antigen protein.

A target molecule that is not a complex of a cancer-testis antigen peptide and a human leukocyte antigen (HLA) can be prepared in the same method.

### 2-4 Binding specificity to antigen protein

The anti-CD3 antibody included in the multispecific molecule of the present invention and a binding fragment thereof recognize, i.e., bind to a CD3 antigen. The anti-CD3 antibody or a fragment thereof preferably binds to a human CD3 and monkey CD3, and more preferably, to a human CD3 and cynomolgus monkey CD3. In contrast, the preferable anti-CD3 antibody does not bind to CD3 of a rat and/or a mouse.

An antibody to a target molecule, preferably a target molecule that is not a complex of a cancer-testis antigen peptide and a human leukocyte antigen (HLA), comprised in the multispecific molecule of the present invention, and a binding fragment thereof recognize, i.e., bind to the target molecule.

In the present invention, the term "recognition", i.e., "binding" refers to binding which is not non-specific adsorption. Whether recognition is made or not, in other words, whether binding is made or not, can be determined based on, for example, the dissociation constant (hereinafter referred to as "KD"). A preferable KD value of the antibody or the like of the present invention to an antigen protein is 1 × 10⁻⁵ M or less, 5 × 10⁻⁶ M or less, 2 × 10⁻⁶ M or less or 1 × 10⁻⁶ M or less.

Binding between an antigen and an antibody in the present invention can be measured or determined by a biomolecular interaction analysis system such as SPR method or BLI method, ELISA method, or RIA method. Binding between an antigen expressed on a cell surface and an antibody can be measured by, e.g., flow cytometry.

The SPR method (surface plasmon resonance method) is used as an analysis method for obtaining, e.g., dissociation constant (KD value) serving as an affinity index, based on an association rate constant (Ka value) and a dissociation rate constant (Kd value) measured by reaction kinetics analysis. Examples of the device to be used in SPR method (analysis) include, Biacore (trademark) (manufactured by GE healthcare), ProteOn (trademark) (manufactured by BioRad), SPR-Navi (trademark) (manufactured by BioNavis), Spreeta (trademark) (manufactured by Texas Instruments), SPRi-PlexII (trademark) (manufactured by Horiba) and Autolab SPR (trademark) (manufactured by Metrohm).

The BLI method (BioLayer Interferometry) is a method of measuring interaction between biomolecules by use of biolayer interference. Examples of the devices to be used in interaction analysis by the BLI method include Octet system (manufactured by Pall ForteBio).

The ELISA (enzyme-linked immunosorbent assay) method is a method for detecting and quantifying a target antigen or antibody contained in a sample solution by capturing it by a specific antibody or antigen and using an enzyme reaction. The antigen or antibody is labeled with an enzyme and added to a reaction system, and detection can be made based on enzyme activity. The enzyme activity is detected by use of a substrate whose absorption spectrum changes depending on the reaction and expressed as a numerical value based on absorbance measurement.

Cell-ELISA is a method of capturing a measurement target present on a cell surface together with the cell and detecting/quantifying the measurement target by use of an enzyme reaction.

In the RIA method (Radio Immunoassay), the amount of an antibody can be determined by labelling an antibody with a radioactive substance and measuring radioactivity emitted from the antibody.

The flow cytometry is a method optically analyzing individual cells by dispersing the cells in a fluid and make the fluid to thinly flow. The antigen binding ability of an antibody is determined by labelling the antibody with a fluorescent dye, allowing the antibody to bind to a cell surface antigen through an antigen-antibody reaction, and measuring the intensity of fluorescence emitted from the labelled antibody bound to the cell.

### 3. Antibody or binding fragment thereof that specifically binds to an antigen protein

### 3-1. Antibody or binding fragment thereof that specifically binds to CD3

The anti-CD3 antibody of the present invention that specifically binds to CD3 and an antigen binding fragment thereof (hereinafter, referred to as the antibody or the like of the present invention) may be a monoclonal antibody and a polyclonal antibody. Examples of the isotype of the monoclonal antibody of the present invention include, but are not particularly limited to, IgG such as IgG1, IgG2, IgG3 and IgG4; IgM, IgA such as IgA1 and IgA2, IgD and Ig. The isotypes and subclasses of the monoclonal antibody can be determined, for example, by Ouchterlony method, ELISA method or RIA method. Examples of the monoclonal antibody of the present invention include an antibody derived from a non-human animal (non-human animal antibody), a human antibody, a chimeric antibody (also referred to as a "chimera antibody") and a humanized antibody. A human antibody can be preferably used. In the range of the antibody of the present invention, an antibody mutant ("variant antibody" described later) is included. More specifically, a human variant antibody is included in the range of a human antibody.

Examples of the non-human animal antibody include antibodies derived from vertebrates such as a mammal and a bird. Examples of the antibodies derived from mammals include antibodies derived from rodents such as a mouse antibody and a rat antibody. Examples of the antibodies derived from birds include a chicken antibody.

Examples of the chimeric antibody include, but are not limited to, antibodies prepared by connecting a variable region of a non-human animal antibody and a constant region of a human antibody (human immunoglobulin).

Examples of the humanized antibody include, but are not limited to, an antibody obtained by transplanting a CDR in a variable region of a non-human animal antibody to a human antibody (in a variable region of a human immunoglobulin); an antibody obtained by transplanting not only CDR but also a part of the sequence of a framework region of a non-human animal antibody to a human antibody; and an antibody obtained by substituting one or more amino acids derived from a non-human animal antibody in either one of them with a human amino acids.

An antibody can be prepared by a wide variety of methods known in the technical field. An antibody can be prepared by a method known in the technical field such as a method using a hybridoma or a cellular immunity method and a genetic recombination method. Also, as a known method, a human antibody is obtained by selecting it from a human antibody library and allowing a phage to display it. As an example for a phage display method that can be used herein, a variable region of a human antibody is expressed on a phage surface as a scFv and a phage bound to the antigen is selected. The gene of a phage bound to an antigen thus selected is analyzed to successfully determine a DNA sequence encoding a variable region of a human antibody that binds to the antigen. If the DNA sequence of the scFv that binds to an antigen is determined, a human antibody can be obtained by preparing an expression vector having the DNA sequence, introducing the vector into an appropriate host and expressing it therein (WO92/01047, WO92/20791, WO93/06213, WO93/11236, WO93/19172, WO95/01438, WO95/15388, Annu. Rev. Immunol (1994)12, 433-455).

An antibody having a high activity and obtained as described above is used as a lead antibody and a gene encoding the lead antibody is mutated to successfully prepare a variant ("variant antibody" described later) having a higher activity.

As an embodiment of the present invention, there is provided an antigen binding fragment (hereinafter referred to simply as a "binding fragment") of the antibody of the present invention. Examples of the binding fragment of the anti-CD3 antibody of the present invention or an antibody to a target molecule that is not a complex of a cancer-testis antigen peptide and a human leukocyte antigen (HLA) include binding fragments of a chimeric antibody, a humanized antibody and a human antibody. The binding fragment of an antibody refers to a fragment having at least an antigen-binding activity among the functions that the antibody has or a modified fragment thereof. Examples of the actions of an antibody generally include an antigen-binding activity, activity to control the antigen activity (for example, agonist activity), an activity to internalize an antigen into a cell, and an activity to inhibit or promote the interaction between an antigen and a substance interacting with the antigen.

The antigen binding fragment of an antibody is not particularly limited as long as the fragment of the antibody has at least an antigen-binding action among the actions that the antibody has. Examples of the antigen binding fragment include, but are not limited to, Fab, Fab', F(ab')₂, Fv, a single-chainFv (scFv) prepared by connecting Fvs of a heavy chain and a light chain via an appropriate linker, and a single domain antibody (sdAb). Like a scFv having a linker portion, a molecule comprising a portion other than an antigen binding fragment of the antibody of the present invention is included in the sense of the antigen binding fragment of the antibody of the present invention.

Of them, a Fab and a scFv are preferred.

A Fab is an antigen binding fragment comprising, a heavy-chain variable region and constant region CH1, and a light chain variable region and constant region CL, as shown in Figure 38B. A Fab can be obtained by digesting a complete antibody with an enzyme such as papain.

A scFv is an antigen binding fragment of an antibody obtained by connecting a heavy chain variable region and a light chain variable region of an antibody by a polypeptide linker (Pluckthun A., The Pharmacology of Monoclonal Antibodies 113, edited by Rosenburg and Moore, Springer Verlag, New York, 269-315 (1994), Nature Biotechnology (2005), 23, 1126-1136). A tandem scFv prepared by connecting two scFvs by a polypeptide linker can be used as a bispecific molecule. Also, e.g., a triabody composed of three or more scFvs can be used as a multispecific molecule.

A scFv can be obtained by a phage display method (Nature Biotechnology (2005), 23, (9), p. 1105-1116), in which an antibody variable region is expressed on the surface of a phage, as a single-chain Fv (scFv), and the phage that binds to an antigen is selected. By analyzing the gene of the phage bound to an antigen and selected, the DNA sequence encoding a human-antibody variable region that binds to an antigen can be determined. If the DNA sequence of a scFv that binds to an antigen is determined, a human antibody can be obtained by preparing an expression vector having the sequence, introducing the vector into an appropriate host and expressing the sequence therein (WO92/01047, WO92/20791, WO93/06213, WO93/11236, WO93/19172, WO95/01438, WO95/15388, Annu. Rev. Immunol (1994)12, p. 433-455, Nature Biotechnology (2005)23 (9), p. 1105-1116).

In the anti-CD3 scFv of the present invention, a heavy chain variable region and a light chain variable region are connected via a disulfide bond. The anti-CD3 scFv can be expressed by use of mammalian cultured cells similarly to a scFv where a heavy chain variable region and a light chain variable region are not connected via a disulfide bond.

Compared to the case determined based on the definition of IMGT, in the case where the position and length of a light chain variable region are determined based on the definition (for example, Kabat, Chothia, AbM, contact) different from IMGT, one or two or more amino acids, for example, arginine and glycine, may sometimes be comprised in the carboxyl terminal of the amino acid sequence of the light chain variable region determined based on the definition of IMGT. An antibody having such a light chain variable region or a binding fragment thereof is also included in the antibody of the present invention or a binding fragment thereof.

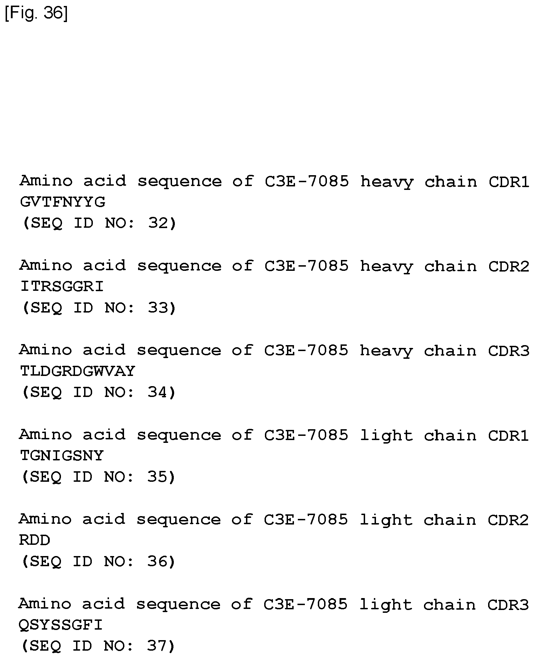

An anti-CD3 antibody or an antigen binding fragment thereof, which is not particularly limited as long as it is an antibody or a binding fragment thereof that binds to human CD3, preferably binds to CD3 of a non-human primate such as a cynomolgus monkey. Examples of the preferable anti-CD3 antibody or an antigen binding fragment thereof include an antibody comprising heavy chain variable region CDRH1 consisting of the amino acid sequence represented by SEQ ID NO: 32; heavy chain variable region CDRH2 consisting of the amino acid sequence represented by SEQ ID NO: 33; heavy chain variable region CDRH3 consisting of the amino acid sequence represented by SEQ ID NO: 34; light chain variable region CDRL1 consisting of the amino acid sequence represented by SEQ ID NO: 35; light chain variable region CDRL2 consisting of the amino acid sequence represented by SEQ ID NO: 36; and light chain variable region CDRL3 consisting of the amino acid sequence represented by SEQ ID NO: 37 (all shown in Figure 36) or an antigen binding fragment thereof.

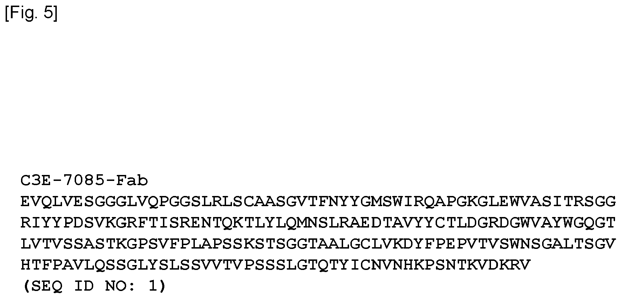

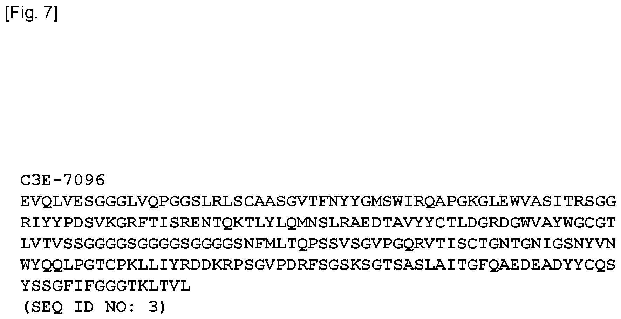

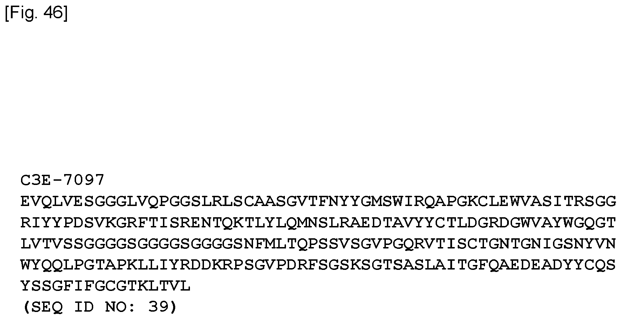

Examples of a more preferable antibody comprising the CDRH1 to CDRH3 and CDRL1 to CDRL3 shown in Figure 36 or an antigen binding fragment thereof include an antibody comprising C3E-7085 heavy chain variable region consisting of the amino acid sequence of amino acids at positions 1 to 118 in the amino acid sequence represented by SEQ ID NO: 1 (Figure 5), C3E-7096 heavy chain variable region consisting of the amino acid sequence of amino acids at positions 1 to 118 in the amino acid sequence represented by SEQ ID NO: 3 (Figure 7), or C3E-7097 heavy chain variable region consisting of the amino acid sequence of amino acids at positions 1 to 118 in the amino acid sequence represented by SEQ ID NO: 39 (Figure 46) or an antigen binding fragment thereof. Note that, an amino acid at position 110 in the 7096 heavy chain variable region additionally comprises cysteine so as to form a S-S bond with an amino aicd a position 177 in the C3E-7096 light chain variable region, and an amino acid at position 44 in the 7097 heavy chain variable region additionally comprises cysteine so as to form a S-S bond with an amino aicd a position 233 in the C3E-7097 light chain variable region.

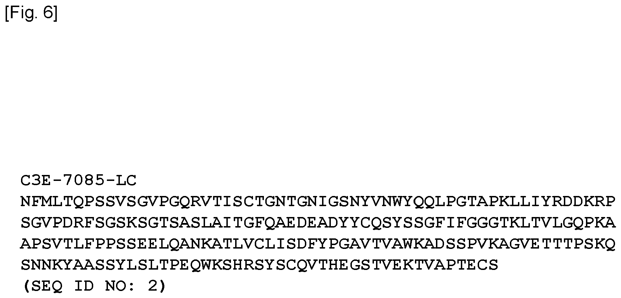

Examples of a more preferable antibody comprising the CDRH1 to CDRH3 and CDRL1 to CDRL3 shown in Figure 36 or an antigen binding fragment thereof include an antibody comprising C3E-7085 light chain variable region consisting of the amino acid sequence of amino acids at positions 1 to 107 in the amino acid sequence represented by SEQ ID NO: 2 (Figure 6) or an antigen binding fragment thereof; an antibody comprising C3E-7096 light chain variable region consisting of the amino acid sequence of amino acids at positions 134 to 240 in the amino acid sequence represented by SEQ ID NO: 3 (Figure 7) or an antigen binding fragment thereof, or C3E-7097 light chain variable region consisting of the amino acid sequence of amino acids at positions 134 to 240 in the amino acid sequence represented by SEQ ID NO: 39 (Figure 46) or an antigen binding fragment thereof. Note that, an amino acid at position 177 in the 7096 light chain variable region additionally comprises cysteine so as to form a S-S bond with an amino acid at position 110 in the 7096 heavy chain variable region, and an amino acid at position 233 in the 7097 light chain variable region additionally comprises cysteine so as to form a S-S bond with an amino acid at position 44 in the 7097 heavy chain variable region.

Examples of a more preferable antibody comprising the CDRH1 to CDRH3 and CDRL1 to CDRL3 shown in Figure 36 or an antigen binding fragment thereof include an antibody comprising a combination of C3E-7085 heavy chain variable region and light chain variable region consisting of No. 1 to 118 amino acids represented by SEQ ID NO: 1 (Figure 5) and No. 1 to 107 amino acids represented by SEQ ID NO: 2 (Figure 6) or an antigen binding fragment thereof, an antibody comprising a combination of C3E-7096 heavy chain variable region and light chain variable region consisting of No. 1 to 118 amino acids and 134 to 240 amino acids represented by SEQ ID NO: 3 (Figure 7) or an antigen binding fragment thereof, or an antibody comprising a combination of C3E-7097 heavy chain variable region and light chain variable region consisting of No. 1 to 118 amino acids and 134 to 240 amino acids represented by SEQ ID NO: 39 (Figure 46) or an antigen binding fragment thereof.

Examples of a more preferable antibody comprising the CDRH1 to CDRH3 and CDRL1 to CDRL3 shown in Figure 36 or an antigen binding fragment thereof include C3E-7085 Fab consisting of the amino acid sequence of amino acids at positions 1 to 216 in the amino acid sequence represented by SEQ ID NO: 1 (Figure 5) and the amino acid sequence of amino acids at positions 1 to 213 in the amino acid sequence represented by SEQ ID NO: 2 (Figure 6).

Examples of a more preferable antibody comprising the CDRH1 to CDRH3 and CDRL1 to CDRL3 shown in Figure 36 or an antigen binding fragment thereof include C3E-7096 scFv consisting of the amino acid sequence of amino acids at positions 1 to 240 in the amino acid sequence represented by SEQ ID NO: 3 (Figure 7), and C3E-7097 scFv consisting of the amino acid sequence of amino acids at positions 1 to 240 in the amino acid sequence represented by SEQ ID NO: 39 (Figure 46).

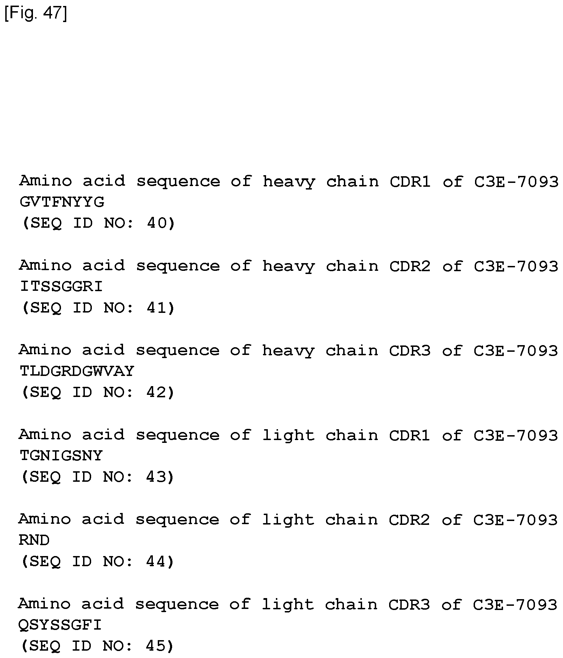

Examples of another preferable anti-CD3 antibody or an antigen binding fragment thereof include an antibody comprising heavy chain variable region CDRH1 consisting of the amino acid sequence represented by SEQ ID NO: 40; heavy chain variable region CDRH2 consisting of the amino acid sequence represented by SEQ ID NO: 41; heavy chain variable region CDRH3 consisting of the amino acid sequence represented by SEQ ID NO:42; light chain variable region CDRL1 consisting of the amino acid sequence represented by SEQ ID NO: 43; light chain variable region CDRL2 consisting of the amino acid sequence represented by SEQ ID NO: 44; and light chain variable region CDRL3 consisting of the amino acid sequence represented by SEQ ID NO: 45 (all shown in Figure 47) or an antigen binding fragment thereof.

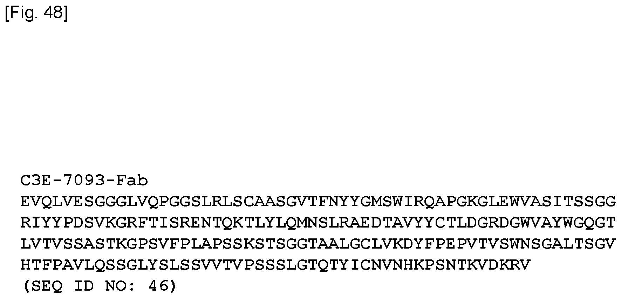

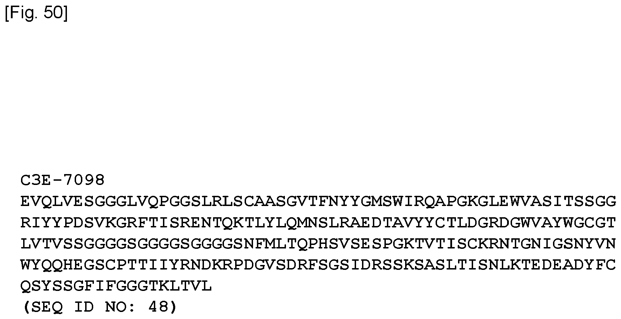

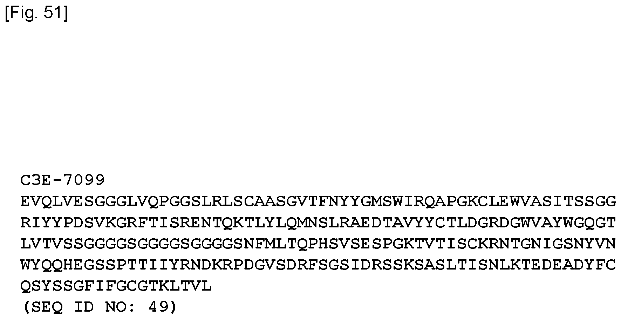

Examples of a preferable antibody comprising the CDRH1 to CDRH3 and CDRL1 to CDRL3 shown in Figure 47 or an antigen binding fragment thereof include an antibody comprising C3E-7093 heavy chain variable region consisting of the amino acid sequence of amino acids at positions 1 to 118 in the amino acid sequence represented by SEQ ID NO: 46 (Figure 48); C3E-7098 heavy chain variable region consisting of the amino acid sequence of amino acids at positions 1 to 118 in the amino acid sequence represented by SEQ ID NO: 48 (Figure 50); and C3E-7099 heavy chain variable region consisting of the amino acid sequence of amino acids at positions 1 to 118 in the amino acid sequence represented by SEQ ID NO: 49 (Figure 51) or an antigen binding fragment thereof. Note that, an amino acid at position 110 in the C3E-7098 heavy chain variable region additionally comprises cysteine so as to form a S-S bond with an amino acid at position 177 in the C3E-7098 light chain variable region; and an amino acid at position 44 in the C3E-7099 heavy chain variable region additionally comprises cysteine so as to form a S-S bond with an amino acid at position 235 in the C3E-7099 light chain variable region.

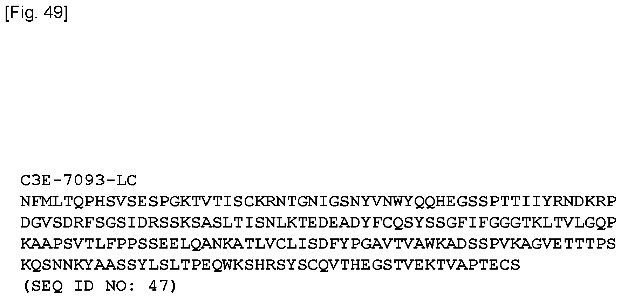

Examples of a preferable antibody comprising the CDRH1 to CDRH3 and CDRL1 to CDRL3 shown in Figure 47 or an antigen binding fragment thereof include an antibody comprising C3E-7093 light chain variable region consisting of the amino acid sequence of amino acids at positions 1 to 118 in the amino acid sequence represented by SEQ ID NO: 47 (Figure 49); C3E-7098 light chain variable region consisting of the amino acid sequence of amino acids at positions 134 to 242 in the amino acid sequence represented by SEQ ID NO: 48 (Figure 50); and C3E-7099 light chain variable region consisting of the amino acid sequence of amino acids at positions 134 to 242 in the amino acid sequence represented by SEQ ID NO: 49 (Figure 51) or an antigen binding fragment thereof. Note that, an amino acid at position 177 in the C3E-7098 light chain variable region additionally comprises cysteine so as to form a S-S bond with an amino acid at position 110 in the C3E-7098 heavy chain variable region; and an amino acid at position 235 in the C3E-7099 heavy chain variable region additionally comprises cysteine so as to form a S-S bond with an amino acid at position 44 in the C3E-7099 light chain variable region.

Examples of a preferable antibody comprising the CDRH1 to CDRH3 and CDRL1 to CDRL3 shown in Figure 47 or an antigen binding fragment thereof include an antibody comprising a combination of C3E-7093 heavy chain variable region and light chain variable region composed of amino acids at positions 1 to 118 represented by SEQ ID NO: 46 (Figure 48) and No. 1 to 109 amino acids represented by SEQ ID NO: 47 (Figure 49), respectively or an antigen binding fragment thereof; a combination of C3E-7098 heavy chain variable region and light chain variable region composed of No. 1 to 118 amino acids and No. 134 to 242 amino acids represented by SEQ ID NO: 48 (Figure 50), respectively or an antigen binding fragment thereof; and a combination of C3E-7099 heavy chain variable region and light chain variable region composed of No. 1 to 118 amino acids and No. 134 to 242 amino acids represented by SEQ ID NO: 49 (Figure 51), respectively, or an antigen binding fragment thereof.

Examples of a more preferable antibody comprising the CDRH1 to CDRH3 and CDRL1 to CDRL3 shown in Figure 46 or an antigen binding fragment thereof include C3E-7093Fab consisting of the amino acid sequence of amino acids at positions 1 to 216 in the amino acid sequence represented by SEQ ID NO: 46 (Figure 48) and the amino acid sequence of amino acids at positions 1 to 215 in the amino acid sequence represented by SEQ ID NO: 47 (Figure 49).

The scFv to be bound to CD3 and having VH and VL connected via a disulfide bond, may have a linker, a linking portion, or other optional portions between VH and VL. The same is applied to the case where the scFv is comprised in the multispecific molecule of the present invention. For example, "another binding portion" or "another group" or a part thereof may be present between VH and VL of CD3 scFv.

### 3-2. Antibody or binding fragment thereof that binds to target molecule

An antibody or a binding fragment thereof that binds to a "target molecule" mentioned in the section 2-2., may be a monoclonal antibody and a polyclonal antibody. Examples of the isotype of the monoclonal antibody of the present invention include, but are not particularly limited to, IgG such as IgG1, IgG2, IgG3 and IgG4; IgM, IgA such as IgA1 and IgA2, IgD and Ig. The isotype and subclass of the monoclonal antibody can be determined, for example, by Ouchterlony method, ELISA method or RIA method. Examples of the monoclonal antibody of the present invention include an antibody (non-human animal antibody) derived from a non-human animal, a human antibody, a chimeric antibody (chimera antibody) and a humanized antibody. A human antibody can be preferably used. In the range of the antibody of the present invention, an antibody mutant ("variant antibody" described later) is included. More specifically, a human variant antibody is included in the range of a human antibody.

Examples of the non-human animal antibody include antibodies derived from vertebrates such as a mammal and a bird. Examples of the antibody derived from a mammal include antibodies derived from rodents, such as a mouse antibody and a rat antibody. Examples of the antibody derived from a bird include a chicken antibody.

Examples of the chimeric antibody include, but are not limited to, antibodies each prepared by connecting a variable region derived from non-human animal antibody and a constant region derived from a human antibody (human immunoglobulin).

Examples of the humanized antibody include, but are not limited to, an antibody obtained by transplanting a CDR in a variable region of a non-human animal antibody to a human antibody (in a variable region of a human immunoglobulin); an antibody obtained by transplanting not only CDR but also a part of the sequence of a framework region of a non-human animal antibody to a human antibody; and an antibody obtained by substituting one or two or more amino acids derived from a non-human animal antibody in either one of them by a human amino acid.

An antibody can be prepared by a wide variety of methods known in the technical field. An antibody can be prepared by a method known in the technical field such as a method using a hybridoma or a cellular immunity method and a genetic recombination method. Also, a method of obtaining a human antibody derived from phage display selected from a human antibody library is known. For example, in a phage display method that can be used herein, a variable region of a human antibody is expressed on a phage surface as a scFv and a phage bound to the antigen is selected. The gene of a phage bound to an antigen thus selected is analyzed to successfully determine a DNA sequence encoding a variable region of a human antibody that binds to the antigen. If the DNA sequence of the scFv that binds to an antigen is determined, a human antibody can be obtained by preparing an expression vector having the DNA sequence, introducing the vector into an appropriate host and expressing it therein (WO92/01047, WO92/20791, WO93/06213, WO93/11236, WO93/19172, WO95/01438, WO95/15388, Annu. Rev. Immunol (1994)12, 433-455).

An antibody having a high activity obtained as described above is used as a lead antibody and a gene encoding the lead antibody is mutated to successfully prepare a variant ("variant antibody" described later) having a higher activity.

The antigen binding fragment of an antibody is not particularly limited as long as the fragment of the antibody has at least an antigen-binding action among the actions that the antibody has. Examples of the antigen binding fragment include, but are not limited to, Fab, Fab', F(ab')₂, Fv, a single-chain Fv (scFv) prepared by connecting Fvs of a heavy chain and a light chain via an appropriate linker, and a single domain antibody (sdAb). Like a scFv having a linker portion, a molecule comprising a portion other than an antigen binding fragment of the antibody of the present invention is included in the sense of the antigen binding fragment of the antibody of the present invention.

In the scFv to a target molecule that is not a complex of a cancer-testis antigen peptide and a human leukocyte antigen (HLA), a heavy chain variable region and a light chain variable region may be connected via a disulfide bond.

### 3-3. Variant of antibody or antigen binding fragment thereof

A variant of the antibody or an antigen binding fragment thereof of the present invention can be prepared preferably for reducing sensitivity to protein degradation or oxidation; maintaining, improving or reducing of biological activity and function and suppressing a change thereof; improving or modifying antigen binding ability; or adding physicochemicalproperties or functional characteristics. A protein is known to change in function and activity when a predetermined amino acid side chain present on a surface thereof changes. Examples of the change of a protein include deamidation of an asparagine side chain and isomerization of aspartic acid side chain. A variant prepared by replacing an amino acid by another amino acid in order to prevent a change in amino acid side chain, is included in the range of the variant of the present invention.

Examples of the variant of the present invention include an antibody or an antigen binding fragment thereof obtained by conservative amino acid substitution in the amino acid sequence of the antibody or an antigen binding fragment thereof. The conservative amino acid substitution refers to a substitution that occurs within a group of the amino acids having a connection to amino acid side chains.

A preferable amino acid group is as follow: acidic group = aspartic acid, glutamic acid; basic group = lysine, arginine, histidine; non-polar group = alanine, valine, leucine, isoleucine, proline, phenylalanine, methionine, tryptophan; and nonpolar family = glycine, asparagine, glutamine, cysteine, serine, threonine and tyrosine. Another preferable amino acid group is as follows: aliphatic hydroxy group = serine and threonine; amide-containing group = asparagine and glutamine; aliphatic group = alanine, valine, leucine and isoleucine; and aromatic group = phenylalanine, tryptophan and tyrosine. Amino acid substitution in a variant antibody is preferably carried out within the range where the antigen binding activity of an original antibody is not lowered.

### 3-4. Modified antibody or a binding fragment thereof and complex thereof

The present invention provides a modified antibody or a binding fragment thereof. The modified antibody of the present invention or a binding fragment thereof refers to an antibody of the present invention or a binding fragment thereof chemically or biologically modified. Examples of the chemically modified antibody or the like include an antibody or the like having an amino acid skeleton to which a chemical moiety, a N-linkage carbon chain or O-linkage carbon chain is added. Examples of biologically modified antibody or the like include an antibody or the like having post-translational modification (for example, glycosylation of N-linkage or O-linkage, processing of an amino terminal region or carboxyl-terminal region, deamidation, isomerization of aspartic acid, oxidation of methionine) and an antibody having a methionine residue added to the amino terminal, which is prepared by expressing the antibody in a prokaryotic host cell. Also, an antibody or a binding fragment thereof labeled with a substance that enables detection or isolation of the antibody of the present invention or antigen. Examples of the antibody or a binding fragment thereof include an enzyme labeled antibody, a fluorescent label antibody and an affinity-marker labeled antibody or a binding fragment thereof. The modified antibody or a fragment thereof according to the present invention is useful for improving stability and blood retention of an original antibody of the present invention or a binding fragment thereof, reducing antigenicity thereof, and detection or isolation of the antibody or an antigen.

Examples of the chemical moiety to be comprised in the modified antibody and a binding fragment thereof include water-soluble polymers such as polyethylene glycol (PEG), ethylene glycol/propylene glycol copolymer, carboxymethyl cellulose, dextran and polyvinyl alcohol.

Examples of the biologically modified antibody or a binding fragment thereof include an antibody or a binding fragment thereof modified through an enzymatic treatment and cell treatment, a fused antibody or a binding fragment thereof having a peptide such as a tag added thereto by gene recombination, and an antibody or a binding fragment thereof prepared in a cell expressing endogenous or exogeneous glycosylation enzyme as a host.

Modification may be made to any site(s) or a desired site of an antibody or a binding fragment thereof. The modification sites may be one or two or more. Modifications to two or more sites may be the same or different.

However, a deletion in a heavy chain sequence or modification to a heavy chain or a light chain sequence does not significantly affect the antigen binding ability and effector function of the antibody (e.g., activation of a complement and antibody-dependent cellular cytotoxicity).

Thus, the present invention includes antibodies having the deletion or the modification. Examples of the antibodies include a deletion variant having a deletion of one or two amino acids in a heavy chain carboxyl terminal (Journal of Chromatography A; 705; 129-134 (1995)); a deletion variant prepared by deleting two amino acid residues of glycine and lysine from a heavy chain carboxyl terminal and amidating a proline residue that newly emerges at the carboxyl terminal (Analytical Biochemistry, 360: 75-83 (2007)); and an antibody prepared by pyroglutamylation of a glutamine or glutamic acid residue at the amino terminal of a heavy chain or light chain thereof (International Publication No. WO2013/147153). (These will be collectively referred to as a "deletion variant".) Note that, as long as an antigen binding ability and effector function are maintained, deletion variants according to the present invention having deletions of heavy-chain and light-chain carboxyl terminals are not particularly limited to the aforementioned ones. When the antibody according to the present invention comprises two or more chains (for example, heavy chain), the two or more chains (for example, heavy chain) may be constituted of at least one type of heavy chain selected from the group consisting of full-length and deletion variant as mentioned above or constituted of two or more heavy chains selected from the group in combination. The quantity ratio or molecular-number ratio of deletion variants may vary depending on the type and culture conditions of mammalian cells producing the antibody according to the present invention. As the main component of the antibody according to the present invention, a case of having a single amino acid residue deletion from the carboxyl terminals of both of the two heavy chains, can be mentioned.

The antibody of the present invention or an antigen binding fragment thereof (comprised in, e.g., a molecule, multispecific molecule, bispecific molecule according to the present invention), even if one to several amino acids derived from, e.g., an expression vector and/or signal sequence are added to the amino terminal and/or carboxyl terminal (and a part or whole thereof is modified as mentioned above), as long as it maintains a desired antigen binding activity, is included in the range of the modified antibody of the present invention or an antigen binding fragment thereof. A molecule comprising the modified antibody or antigen binding fragment thereof is included in the range of the molecule of the present invention.

In the present invention, a "modified antibody or an antigen binding fragment thereof" is included in the sense of the "antibody or a binding fragment thereof". The "modified antibody or an antigen binding fragment thereof" is included in the sense of the "antibody or an antigen binding fragment thereof" comprised in, e.g., the molecule, multispecific molecule and a bispecific molecule of the present invention.

The antibody-dependent cellular cytotoxicity can be enhanced by controlling glycosylation (e.g., glycosylation, defucosylation) bound to the antibody of the present invention. For controlling glycosylation of an antibody, the techniques disclosed in International Publication No. WO99/54342, WO00/61739 and WO02/31140 are known but a technique for controlling glycosylation is not limited to these.

The present invention includes a conjugate (immunoconjugate) prepared by connecting an antibody as mentioned above and another molecule via a linker. Examples of the conjugate include ADC (antibody-drug conjugate) such as a conjugate of an antibody with a radioactive substance and a conjugate of an antibody with a compound (drug) having a pharmacological action (Methods Mol Biol. (2013)1045: 1-27; Nature Biotechnology (2005) 23, p. 1137-1146).

The present invention includes a conjugate prepared by connecting an antibody and an additional functional polypeptide. Examples of the antibody-peptide conjugate include a conjugate of an antibody and an albumin binding polypeptide (Protein Eng Des Sel. (2012) (2): 81-8).

A modified antibody as mentioned above, an antibody modified in glycosylation and a conjugate are included in the antibody of the present invention. The binding fragments of the modified antibody, antibody modified in glycosylation and conjugate are included in the binding fragment of the antibody of the present invention.

### 4. Method for producing antibody or antigen binding fragment thereof

The antibody of the present invention or an antigen binding fragment thereof can be produced by inserting, for example, DNA encoding a heavy chain variable region or DNA encoding a light chain variable region, into an expression vector, transforming a host cell for expression with the expression vector and culturing the host cell to produce a recombinant antibody.

DNA encoding an antibody or an antigen binding fragment thereof is produced by obtaining DNA encoding a heavy chain by connecting DNA encoding a heavy chain variable region and DNA encoding a heavy chain constant region, further by obtaining DNA encoding a light chain by connecting DNA encoding a light chain variable region and DNA encoding a light chain constant region.

The antibody of the present invention or an antigen binding fragment thereof can be produced by inserting DNA encoding a heavy chain and DNA encoding a light chain in an expression vector, transforming a host cell with the vector and culturing the host cell. At this time, the DNA encoding a heavy chain and the DNA encoding a light chain may be introduced into the same expression vector and a host cell may be transformed by the vector. Alternatively, the DNA encoding a heavy chain and the DNA encoding a light chain may be introduced into different vectors and a host cell is transformed with the two vectors. In this case, DNA encoding a heavy chain constant region and DNA encoding a light chain constant region are previously introduced into a vector, to which DNA encoding a heavy chain variable region and a light chain variable region may be introduced. The vector may comprise DNA encoding a signal peptide for promoting secretion of an antibody from a host cell. In this case, DNA encoding a signal peptide and DNA encoding an antibody or an antigen binding fragment thereof are connected in frame. After the antibody or an antigen binding fragment thereof is produced, the signal peptide is removed. In this manner, the antibody or an antigen binding fragment thereof can be obtained as a mature protein.

In this case, DNA encoding a heavy chain variable region, DNA encoding a light chain variable region, DNA obtained by connecting DNA encoding a heavy chain variable region and DNA encoding a heavy chain constant region, DNA obtained by connecting DNA encoding a light chain variable region and DNA encoding a light chain constant region may be functionally connected to an element such as a promoter, an enhancer and a polyadenylation signal. The phrase "functionally connecting" means that connection is made to an element such that the element performs its function.

The expression vector, which is not particularly limited as long as it is replicable in a host such as an animal cell, a bacterium and a yeast. Examples of the expression vector include a plasmid or a phage known in the technical field. Examples of a vector for use in construction of the expression vector include pcDNA (trademark) (Thermo Fisher Scientific), Flexi (registered trademark) vector (Promega), pUC19, pUEX2 (manufactured by Amersham), pGEX-4T, pKK233-2 (manufactured by Pharmacia) and pMAM-neo (manufactured by Clontech). Examples of the host cell that can be used include prokaryotic cells such as *Escherichia coli* and *Bacillus subtilis,* eukaryotic cells such as yeasts and animal cells. Eukaryotic cells are preferably used. Examples of the animal cells that may be used include human fetal kidney cell line, HEK293 cells and Chinese Hamster Ovary (CHO) cells. The expression vector is introduced in a host cell by a method known in the technical field to transform the host cell. Examples of the introduction method include an electroporation method, a calcium phosphate precipitation method and a DEAE-dextran transfection method. The antibody produced can be purified by a separation/purification method usually applied to proteins. For example, affinity chromatography, another chromatography, filtration, ultrafiltration, salting out and dialysis may be appropriately selected or used in combination.

### 5. Multispecific molecule

The multispecific molecule of the present invention is not particularly limited as long as the molecule comprises a scFv that binds to CD3 of the present invention in which a heavy chain variable region and a light chain variable region are connected via a disulfide bond, or comprises a Fab that binds to CD3 (hereinafter referred to as a "CD3 antibody of the present invention") and a moiety at which the molecule binds to a target molecule except CD3, preferably, a target molecule that is not a complex of a cancer-testis antigen peptide and a major histocompatibility complex (MHC) (hereinafter referred to as "another binding portion"), and may comprise another group (hereinafter referred to as "another group") except the "CD3 antibody of the present invention" and "another binding portion". The examples of "another binding portion" include an antibody or a binding fragment thereof, a protein or peptide not derived from an immunoglobulin, a nucleic acid molecule such as a nucleic acid aptamer, a low molecular-weight compound and a T cell receptor. Examples of "another group" include a compound having a biological activity, a pharmacological action, a therapeutic effect, a preventive effect, an adjuvant action, *in-vivo* kinetics control function or labelling function (e.g., protein, peptide, nucleic acid molecule, low molecular-weight compound, a radioisotope containing compound, a labelling substance containing compound, an artificially synthesized compound, a pharmaceutical component, an anti-tumor agent); its precursor (e.g., prodrug); and a compound prepared by connecting or fusing a linker or a linking portion to a compound mentioned above. The "CD3 antibody of the present invention" and "another binding portion", and optionally "another group" may be mutually directly connected or fused, or indirectly connected or fused with a linker or a linking portion interposed between them. The number of "another binding portion" may be one or more, 2, 3 or more. The number of "another group" may be 1, 2 or more. They may be linearly bound or fused, or nonlinearly bound or fused. The order of them to be bound or fused and the position of a branch, etc. are not limited. Such a multispecific molecule of the present invention may have formats shown in Figures 38A to F, Figures 39A and B, Figures 40A and B, Figures 41A and B, Figure 42, Figures 43A and B, Figures 44A and B, and Figure 45 or contain the formats mentioned above but are not limited to these.

As a preferable multispecific molecule of the present invention, a multispecific protein (a molecule wholly composed of a polypeptide and/or peptide) such as a multispecific antibody, can be mentioned. Examples of the multispecific antibody include a bispecific antibody, a trispecific antibody and a quadruple-specific antibody. The term "bi-specificity" (or, e.g., "triple specificity", "quadruple specificity") means that binding can be made to two (or, e.g., three, four) different epitopes on the same molecule or different epitopes on two molecules (or, e.g., three molecules, four molecules). An antibody or antigen binding fragment having such bi-specificity is included. The bispecific molecule of the present invention binds to CD3 and further binds to a target molecule, preferably, a target molecule that is not a complex of a cancer-testis antigen peptide and a human leukocyte antigen (HLA).

As the multispecific molecule of the present invention, molecules having the following structures (formats) can be mentioned.

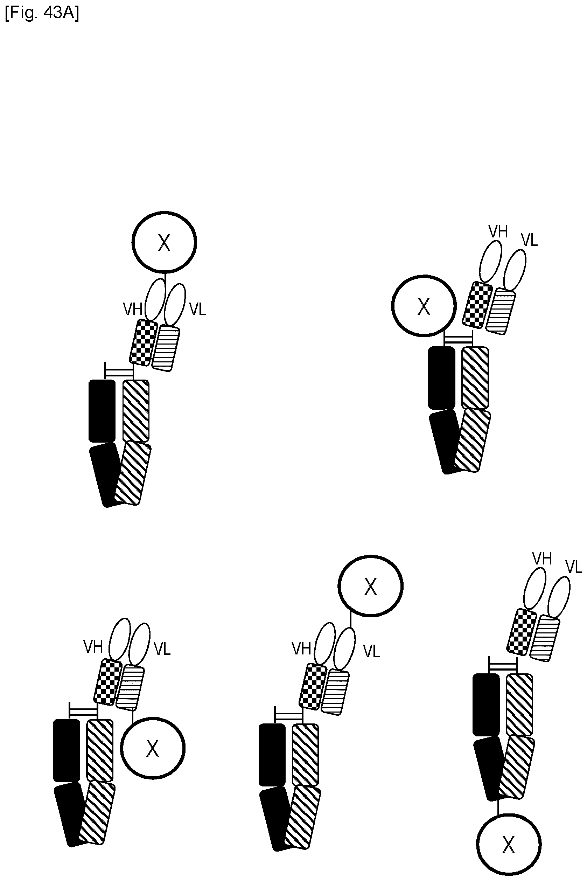

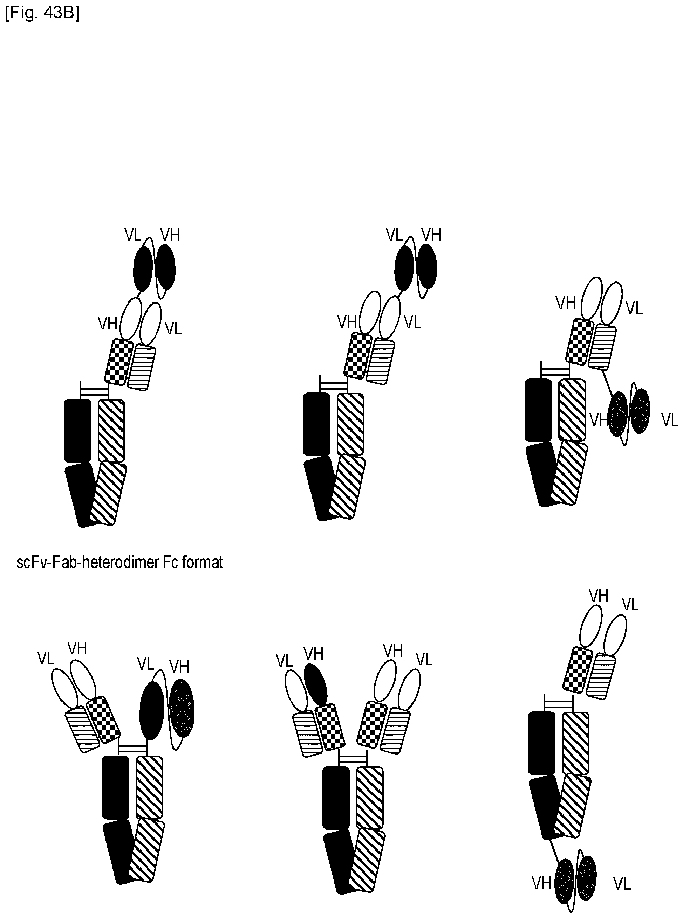

As the bispecific molecule of the present invention, a bispecific molecule prepared by connecting Fab of a first antibody and scFv of a second antibody to one of the molecules of a dimer Fc via a linker, is mentioned. In this case, it is possible that Fc is connected to Fab, and then, scFv is connected to the Fab; or that scFv is connected to Fc, and then, Fab is connected to the scFv. It is preferable that Fc is connected to Fab, and then, scFv is connected to the Fab. Fab and scFv may be connected by connecting scFv to a variable region of Fab via a linker. The bispecific molecule may have a format in which Fc having a mutation for forming a heterodimer introduced therein is associated with a site downstream of a site where scFv and Fab are connected via a linker. Such a bispecific molecule is referred to as a scFv-Fab-heterodimer Fc-type bispecific molecule or a scFv-Fab-heterodimer Fc-format (Figure 43B, upper left).

In the present invention, for example, a scFv-Fab-heterodimer Fc-format composed of an anti-CD3 Fab and a scFv to a target molecule that is not a complex of a cancer-testis antigen peptide and a human leukocyte antigen (HLA), may be used.

In an anti-CD3 scFv comprised in the bispecific molecule of the present invention, a heavy chain variable region and a light chain variable region are connected via a disulfide bond. In the scFv to a target molecule that is not a complex of a cancer-testis antigen peptide and a human leukocyte antigen (HLA), a heavy chain variable region and a light chain variable region may be connected via a disulfide bond.

A bispecific molecule obtained by connecting a taFv (Figure 38E) having a format where two types of scFvs, a first antibody and a second antibody, are connected via a linker, to one of the molecules of a dimer Fc, directly (not via a linker) or via a linker, is mentioned. Such a bispecific molecule is referred to as a taFv-heterodimer Fc-type bispecific molecule or a taFv-heterodimer Fc-format (Figure 40B, lower left). The bispecific molecule has a format in which Fc having a mutation for forming a heterodimer is heterologously associated with a site downstream of a taFv. The order of connecting the first antibody and the second antibody in a taFv is not limited.

Figure 43B, in the upper left, the structure of a scFv-Fab-heterodimer Fc-type bispecific molecule is shown. The structure of a scFv is shown in Figure 38A, a Fab in Figure 38B, a taFv in Figure 38E, a scFv-Fab in Figure 38F, a taFv-heterodimer Fc-type bispecific molecule in Figure 40B lower left, and a taFv-Fab-heterodimer Fc-type bispecific molecule in Figure 41B, upper right.

The bispecific molecule of the present invention has a plurality of polypeptides mutually associated.

In the present invention, for example, as a taFv, an anti-CD3 scFv and a target molecule, preferably taFv of scFv to a target molecule that is not a complex of a cancer-testis antigen peptide and a human leukocyte antigen (HLA), may be used. The taFv-heterodimer Fc-type bispecific molecule preferably comprises (a) a first polypeptide comprising a scFv that specifically binds to a target molecule, preferably a target molecule that is not a complex of a cancer-testis antigen peptide and a human leukocyte antigen (HLA), a scFv that specifically binds to CD3, and an immunoglobulin Fc region (i) sequentially in this order from the N terminal toward the C terminal, and a second polypeptide comprising a hinge regionand a Fc region (ii) of an immunoglobulin; is more preferably, (b) formed by associating the first polypeptide and the second polypeptide at the Fc region (i) and Fc region (ii). The Fc regions of the first polypeptide and the second polypeptide comprise a mutation for forming a heterodimer. Examples of the taFv-heterodimer Fc-type bispecific molecule are shown in Figure 40B, lower left. As shown in Figure 40B, lower left, Fc region (i) site of the first polypeptide and Fc region (ii) site painted in black of the second polypeptide are connected to associate the first polypeptide and the second polypeptide. For example, in Figure 40B lower left, the scFv indicated by white is a scFv to a target molecule, preferably a target molecule that is not a complex of a cancer-testis antigen peptide and a human leukocyte antigen (HLA); and a scFv indicated by hatched lines from top right to bottom left is an anti-CD3 scFv (the solid bar between VH and VL indicates that VH and VL are connected by a disulfide bond).

A first polypeptide comprised in a more preferable taFv-heterodimer Fc-type bispecific molecule of the present invention comprises the amino acid sequence represented by SEQ ID NO: 3. The second polypeptide in a preferable taFv-heterodimer Fc-type bispecific molecule of the present invention comprises a hinge region and wild-type or mutant Fc derived from human antibodies.

The scFv-Fab-heterodimer Fc-type bispecific molecule preferably comprises (a) a first polypeptide comprising a scFv that specifically binds to a target molecule that is not a complex of a cancer-testis antigen peptide and a human leukocyte antigen (HLA), an antibody heavy-chain variable region that specifically binds to CD3 and constant region CH1, and immunoglobulin Fc region (i) sequentially in this order from the N terminal toward the C terminal; a second polypeptide comprising a hinge region and a Fc region (ii) of an immunoglobulin; and a third polypeptide comprising an antibody light-chain consisting of a variable region and a constant region, and is more preferably formed of (b) the first polypeptide and the second polypeptide are associated at the Fc region (i) and Fc region (ii) and the first polypeptide is associated with the third polypeptide (antibody light-chain) at the variable region and constant region CH1 of the antibody heavy-chain.

The Fc region of the first polypeptide and the second polypeptide may be a wild type or may comprise a mutation for forming a heterodimer. An example of the scFv-Fab-heterodimer Fc-type bispecific molecule is shown in Figure 43B, upper left. The right half of the Figure 43B (upper left) indicates the first polypeptide and third polypeptide, whereas the left half indicates the second polypeptide. As shown in Figure 43B, upper left, Fc region (i) of a first polypeptide and Fc region (ii) of the second polypeptide painted in black are associated, and the first polypeptide and the third polypeptide are associated. For example, in Figure 43B, upper left, the scFv painted in black is a scFv to a target molecule, preferably a target molecule that is not a complex of a cancer-testis antigen peptide and a human leukocyte antigen (HLA); Fabs shown in white, a checkered pattern and horizontal lines are anti-CD3 Fabs.

As the amino acid sequence comprised in the first polypeptide comprised in a preferable scFv-Fab-heterodimer Fc-type bispecific molecule, the amino acid sequence represented by SEQ ID NO: 1 can be exemplified.

The second polypeptide comprised in a preferable scFv-Fab-heterodimer Fc-type bispecific molecule of the present invention comprises a hinge region and a mutant Fc derived from a human antibody. As the second polypeptide comprised in a more preferable scFv-Fab-heterodimer Fc-type bispecific molecule of the present invention comprises a hinge region derived from a human antibody and a wild type or mutant Fc.

The third polypeptide comprised in a preferable scFv-Fab-heterodimer Fc-type bispecific molecule of the present invention comprises a light chain derived from a human antibody. As the third polypeptide comprised in a more preferable scFv-Fab-heterodimer Fc-type bispecific molecule, the amino acid sequence represented by SEQ ID NO: 2 can be exmplified.

One, two or three or more peptides comprised in the bispecific molecule of the present invention may be a "deletion variant(s)" as mentioned above, in other words, may have one or two (or more) amino-acid mutations (including deletion) at the carboxyl terminal, particularly the carboxyl terminal derived from an antibody heavy-chain. For example, the carboxyl terminus of the amino acid sequence of the first polypeptide comprised in RX3-0002, which is one of the preferred scFv-Fab-heterodimer Fc-type bispecific molecule of the present invention, may be any of 713rd Lys of the amino acid sequence of SEQ ID NO: 31, 712nd Gly of the amino acid sequence of SEQ ID NO: 31 in which one amino acid is deleted, or a mixture comprising them. Similarly, the carboxyl terminus of the amino acid sequence of the second polypeptide in a preferable scFv-Fab-heterodimer Fc-type bispecific molecule of the present invention may be any of 246th Lys of the amino acid sequence of SEQ ID NO:9, 245th Gly of the amino acid sequence of SEQ ID NO:9 in which one amino acid is deleted, or a mixture comprising them.

A scFv and Fab comprised in the bispecific molecule of the present invention, are preferably, a scFv and Fab of a humanized antibody or a human antibody, and Fc is preferably Fc of a human antibody.

In the variable region comprised in the bispecific molecule of the present invention, a heavy chain variable region and a light chain variable region are connected sequentially in this order, or a light chain variable region and a heavy chain variable region are connected in this order, from the amino terminal side of the antibody. A linker may be (optionally) present between both variable regions. A glycine residue may be (optionally) present at the amino terminal of the variable region on the amino terminal side of the antibody. A tandem scFv-type bispecific molecule (optionally) has a linker, a FLAG tag, and/or, a His tag, which may be attached to the carboxyl terminal of the variable region on the carboxyl terminal side of the antibody. As a preferable embodiment, a molecule formed of a heavy chain variable region, a first linker, a light chain variable region, a second linker, a FLAG tag and a His tag, which are sequentially connected in this order from the amino terminal is mentioned.

Examples of the linker include a single-strand polypeptide, a single-strand oligopeptide and a synthesized linker such as PEG, a nucleotide, a sugar chain or a compound. The linker is not particularly limited as long as it connects two polypeptides and any one of the linkers known in the technical field can be used.

The linker, for example, a peptide linker, has a length of 5 to 30 amino acids. When a bispecific molecule comprises a plurality of linkers, same-length peptide linkers or peptide linkers mutually different in length may be used.

Examples of the peptide linker, for example, a sequence repeat (Gly·Gly·Gly·Gly·Ser) (SEQ ID NO: 38) may be mentioned. One to several amino acid residues different from Gly and Ser may be added to the sequence repeat.

Of the possible structures (formats) of the multispecific antibody of the present invention, particularly a bispecific antibody, as mentioned above, a taFv-heterodimer Fc format, and a scFv-Fab-heterodimer Fc-format are preferred, and a scFv-Fab-heterodimer Fc-format is more preferable. A format (taFv-heterodimer Fc-format: Figure 40B, lower left) having a scFv to a target molecule, preferably a format having a scFv to a target molecule that is not a complex of a cancer-testis antigen peptide and a human leukocyte antigen (HLA) and an anti-CD3 scFv positioned sequentially in this order from the N terminal toward the C terminal, is further more preferred as a taFv-heterodimer Fc-format. The scFv-Fab-heterodimer Fc-format (Figure 43B, upper left) comprising a scFv to a target mtolecule, preferably a target molecule that is not a complex of a cancer-testis antigen peptide and a human leukocyte antigen (HLA) at the N terminal, is a further more preferable format that the bispecific antibody of the present invention can take as a scFv-Fab-heterodimer Fc-format.

Among multispecific antibodies, especially bispecific antibodies, as described above, preferable one including the scFv-Fab-heterodimer Fc format, comprises one polypeptide group selected fromat
(i) a polypeptide comprising an amino acid sequence consisting of the 20-718th amino acid residues of SEQ ID NO: 29 or an amino acid sequence in which one or two amino acids are deleted at the carboxyl terminus of said amino acid sequence, a polypeptide comprising an amino acid sequence consisting of the 20-246th amino acid residues of SEQ ID NO: 9 or an amino acid sequence in which one or two amino acids are deleted at the carboxyl terminus of said amino acid sequence, and a polypeptide comprising an amino acid sequence consisting of the 21-233rd amino acid residues of SEQ ID NO: 12;
(ii) a polypeptide comprising an amino acid sequence consisting of the 20-717th amino acid residues of SEQ ID NO: 30 or an amino acid sequence in which one or two amino acids are deleted at the carboxyl terminus of said amino acid sequence, a polypeptide comprising an amino acid sequence consisting of the 20-246th amino acid residues of SEQ ID NO: 9 or an amino acid sequence in which one or two amino acids are deleted at the carboxyl terminus of said amino acid sequence, and a polypeptide comprising an amino acid sequence consisting of the 21-233rd amino acid residues of SEQ ID NO: 12; and
(iii) a polypeptide comprising an amino acid sequence consisting of the 20-713rd amino acid residues of SEQ ID NO: 31 or an amino acid sequence in which one or two amino acids are deleted at the carboxyl terminus of said amino acid sequence, a polypeptide comprising an amino acid sequence consisting of the 20-246th amino acid residues of SEQ ID NO: 9 or an amino acid sequence in which one or two amino acids are deleted at the carboxyl terminus of said amino acid sequence; and a polypeptide comprising an amino acid sequence consisting of the 21-233rd amino acid residues of SEQ ID NO: 12.

Among multispecific antibodies, especially bispecific antibodies, as described above, preferable one including the taFv-heterodimer Fc format, comprise one polypeptide pair selected from
(i) a polypeptide comprising an amino acid sequence consisting of the 20-744th amino acid residues of SEQ ID NO: 21 or an amino acid sequence in which one or two amino acids are deleted at the carboxyl terminus of said amino acid sequence, and a polypeptide comprising an amino acid sequence consisting of the 20-246th amino acid residues of SEQ ID NO: 9 or an amino acid sequence in which one or two amino acids are deleted at the carboxyl terminus of said amino acid sequence;
(ii) a polypeptide comprising an amino acid sequence consisting of the 20-743rd amino acid residues of SEQ ID NO: 23 or an amino acid sequence in which one or two amino acids are deleted at the carboxyl terminus of said amino acid sequence, and a polypeptide comprising an amino acid sequence consisting of the 20-246th amino acid residues of SEQ ID NO: 9, or an amino acid sequence in which one or two amino acids are deleted at the carboxyl terminus of said amino acid sequence; and
(iii) a polypeptide comprising an amino acid sequence consisting of the 20-739th amino acid residues of SEQ ID NO: 25 or an amino acid sequence in which one or two amino acids are deleted at the carboxyl terminus of said amino acid sequence, and a polypeptide comprising an amino acid sequence consisting of the 20-246th amino acid residues of SEQ ID NO: 9 or an amino acid sequence in which one or two amino acids are deleted at the carboxyl terminus of said amino acid sequence.

Examples of the format that the multispecific molecule of the present invention may take, include not only the formats shown in Figures 38 to 45 and other formats disclosed in the specification but also formats disclosed in Godar et.al., Expert Opinion on Therapeutic Patents, Vol. 28 (No. 3), Page 251-276 (2018), Brinkmann and Knotermann MABS, Vol. 9 (No. 2), Page 182-212 (2017), and Wu and Demarest Methods, Vol. 154, Page 3-9 (2019). All contents of these literatures are incorporated in the disclosure of the present application.

The present invention includes a molecule which comprises an amino acid sequence having a sequence identity of 90%, 91%, 92%, 93%, 94%, 95%, 96%, 97%, 98%, or 99% or more with the amino acid sequence comprised in the molecule of the present invention, binds to CD3 and further binds to a target molecule that is not a complex of a cancer-testis antigen peptide and a human leukocyte antigen (HLA).

The multispecific antibody of the present invention has excellent biological activities, physicochemical properties (hereinafter referred to as "physical properties"), safety and *in-vivo* kinetics, etc. Impurities comprised in biopharmaceuticals have a relation to safety of the pharmaceuticals. Because of this, increase (or not) of impurities must be appropriately controlled during manufacturing and storage based on the standard of impurities. Of them, an aggregate, which is a major item of impurities that must be strictly controlled, since it has a relation to immunogenicity risk and decrease of medicinal effect or the like (Guidance for Industry Immunogenecity Assessment for Therapeutic Protein Products,U.S. Department of Health and Human Services/Food and Drug Administration/Center for Drug Evaluation and Research(CDER)/Center for Biologics Evaluation and Research(CBER),August 2014,p. 15-16). Impurities must be controlled not only during manufacturing steps but also after manufacturing including evaluation of time-course change thereof (stability, increase or not). Since effective periods of pharmaceuticals are determined based on the results of long-term stability test, an antibody stable overtime is advantageous since a long effective period can be set. Thus, physicochemical properties serving as an index for selecting an antibody preferable as a biopharmaceutical are solution stability and shaking stability in the present invention. If a multispecific antibody with concerns about solution stability, shaking stability, etc. is selected as a drug product, significant resources and costs will be required to add purification processes to reduce the amount of aggregates (which will inevitably reduce yields) and to establish formulations that are resistant to aggregates, etc. Therefore, it is preferable to select an antibody with fewer of these concerns.

Solution stability can be evaluated, for example, by preparing samples at 20 mg/mL and measuring the HMWS content (%) after storage at 40°C for 1, 7, and 14 days by size exclusion chromatography. Samples with a relatively low rate of increase in HMWS (%) can be judged to have relatively high solution stability.

Shaking stability can be evaluated, for example, by preparing samples at 2 mg/mL, shaking them at 2500 rpm for 1 to 2 hours, performing size exclusion chromatography, and calculating the peak area and HMWS (%) for each sample. However, the aggregates detected as HMWS by this method are about 10 to 100 nm in diameter, and subvisible particles (0.1 to 10 µm in diameter) and insoluble particles (10 µm or larger in diameter) with a particle size of 100 nm or larger cannot be detected. To confirm whether or not particles of such size are formed, for example, the number of particles is evaluated by the MicroFlow Imaging method using FlowCAM8100 (DKSH Japan) or other devices, using samples before shaking and after 2 hours of shaking treatment. If the number of particles after shaking increases than before shaking, it can be assumed that particles are formed from the target protein monomer via HMWS due to shaking degradation. Therefore, in the evaluation of shaking stability, the results of (1) the rate of increase in HMWS (%), (2) the rate of decrease in peak area, and/or (3) the increase or decrease in the number of particles by MicroFlow Imaging method are interpreted and evaluated. For example, if the increase in HMWS (%) of one specimen is greater than that of other specimen, but the decrease in peak area and/or the increase in the number of particles by MicroFlow Imaging method of that specimen is smaller than that of the other specimen, the specimen can be evaluated as more shaking stable than the other specimen. The reason for this is that the lower increase (%) in HMWS for the other sample is that aggregates equivalent to HMWS may aggregate to form larger multimeric aggregates that are not detectable by size exclusion chromatography. The fact that the aggregates are composed of the target protein can be confirmed by the fact that the aggregates, which contained very little before shaking, increase after shaking.

The multispecific molecule of the present invention, such as the bispecific antibody that is taFv-heterodimer Fc-format, in which the heavy and light chain variable regions of the anti-CD3 scFv are linked by disulfide bonds, and scFv-Fab-heterodimer Fc-format, have high solution stability because HMWS containing dimers do not occur easily over long periods of time in solution. For example, in the method of evaluating solution stability illustrated in paragraph 0137, it is preferable to have a higher solution stability than a bispecific antibody that is of the taFv-heterodimer Fc format and in which the heavy and light chain variable regions of the anti-CD3 scFv are not linked by disulfide bonds.

In addition, the multispecific molecule of the present invention, such as the bispecific antibody that is taFv-heterodimer Fc format, in which the heavy and light chain variable regions of the anti-CD3 scFv are linked by disulfide bonds, and the bispecific antibody that is scFv-Fab -heterodimer-Fc format have high shaking stability as interpreted based on the results of (1) the rate of increase in HMWS, (2) the rate of decrease in peak area, and/or (3) the increase or decrease in particle number by MicroFlow Imaging method, including the dimer. For example, in the shaking stability evaluation method illustrated in paragraph 0138, it is preferable to have higher shaking stability than a bispecific antibody that is of the taFv-heterodimer Fc format in which the heavy and light chain variable regions of the anti-CD3 scFv are not linked by disulfide bonds.

The present invention provides pharmaceutical composition comprising anti-CD3 antibodies, antigen-binding fragments thereof or modifications thereof, and/or multispecific molecules comprising them (hereinafter referred to as "anti-CD3 antibodies, etc.").

In the present invention, treatment and/or prevention of disease includes, but is not limited to, prevention of the onset, suppression or inihibition of exacerbation or progression, alleviation of one or more symptoms exhibited by an individual afflicted with such disease, suppression or remission of exacerbation or progression, treatment or prevention of secondary diseases. For example, the diseases include, but are not limited to, cancer in the case of pharmaceutical composition comprising multispecific molecules.

In addition to an effective amount of anti-CD3 antibody or the like for treatment or prevention, the pharmaceutical composition of the present invention can contain pharmaceutically acceptable diluents, carriers, solubilizers, emulsifiers, preservatives and/or auxiliary agents.

The term "effective amount for treatment or prevention" means an amount that produces a therapeutic or preventive effect for a particular disease, dosage form, and route of administration.

The pharmaceutical composition of the present invention may contain substances to change, maintain, or retain pH, osmolality, viscosity, transparency, color, isotonicity, sterility, stability of the composition or the antibody comprised therein, solubility, sustained release, absorption, permeability, dosage form, strength, properties, shape, etc. (hereinafter referred to as "substances for formulation"). The substances for formulation can include substances for pharmaceuticals are not limited as long as the formulation substances are pharmacologically acceptable substances. For example, non-toxic or low toxicity is a property that is preferably possessed by the substances for formulation.

The substances for formulation include, but are not limited to: amino acids such as glycine, alanine, glutamine, asparagine, histidine, arginine, or lysine; antibacterial agents; antioxidants such as ascorbic acid, sodium sulfate, or sodium hydrogen sulfite; buffers such as phosphoric acid, citric acid, boric acid buffer, sodium bicarbonate, or Tris-HCl solution; fillers such as mannitol or glycine; chelating agents such as ethylenediaminetetraacetic acid (EDTA); complexing agents such as caffeine, polyvinylpyrrolidine, β-cyclodextrin and hydroxypropyl-β-cyclodextrin; bulking agents such as glucose, mannose or dextrin; other carbohydrates such as monosaccharide, disaccharide, glucose, mannose and dextrin; coloring agents; flavoring agents; diluents; emulsifiers; hydrophilic polymers such as polyvinylpyrrolidine; low molecular weight polypeptides; preservatives such as salt forming counterions, benzalkonium chloride, benzoic acid, salicylic acid, thimerosal, phenethyl alcohol, methylparaben, propylparaben, chlorhexidine, sorbic acid or hydrogen peroxide; solvents such as glycerin, propylene glycol or polyethylene glycol; sugar alcohols such as mannitol or sorbitol; suspending agents; PEG; surfactants such as polysorbate such as sorbitan esters, polysorbate 20, polysorbate 80, triton, tromethamine, lecithin or cholesterol; stabilizing enhancers such as sucrose or sorbitol; elasticity enhancers such as sodium chloride, potassium chloride, mannitol or sorbitol; transport agents; diluents; excipients formulators; and/or pharmaceutically adjuvant.

The amount of these substances for formulation is 0.001 to 1000 times the weight of the anti-CD3 antibody or the like, preferably 0.01 to 100 times, more preferably 0.1 to 10 times.

Immunoliposome containing anti-CD3 antibodies or the like in liposomes, and antibody-modified products in which antibodies are bound to liposomes (U.S. Patent No. 6214388, etc.) are also included in the pharmaceutical compositions of the present invention.

Excipients and carriers are usually liquids or solids and are not limited to water for injection, saline solution, artificial cerebrospinal fluid, or other substances used in formulations for oral or parenteral administration. Phsiological saline solutions include neutral saline, those containing serum albumin, and others.

Examples of buffers include a Tris buffer prepared so that the final pH of the pharmaceutical composition is between 7.0 and 8.5, an acetic acid buffer prepared so that the final pH is between 4.0 and 5.5, a citric acid buffer prepared so that the pH is between 5.0 and 8.0, a histidine buffer prepared so that the pH is between 5.0 and 8.0, etc.

The pharmaceutical compositions are solids, liquids, suspensions, etc. Lyophilized formulations can be included. Excipients such as sucrose can be used to form lyophilized formulations.

The route of administration of the pharmaceutical composition of the present invention can be enteral, topical, or parenteral, and a preferable route of administration should be selected depending on the target disease. Specifically, intravenous, intra-arterial, intramuscular, intradermal, subcutaneous, intraperitoneal, transdermal, intraosseous, intraarticular, etc. can be included.

The composition of such pharmaceutical compositions can be determined according to the method of administration, CD3 protein binding affinity of the antibody, etc.

The dosage of the anti-CD3 antibody, etc. can be determined according to the species of the individual, the type of disease, symptoms, sex, age, pre-existing disease, CD3 protein binding affinity of the antibody or its biological activity, and other factors. It is usually 0.01 to 1000 mg/kg, preferably 0.1 to 100 mg/kg can be administered once a day to 180 days or twice or three or more times a day. Examples of form of pharmaceutical composition include injectable formulation (including lyophilized and intravenous formulation), suppository, nasal absorption formulation, transdermal absorption formulation, sublingual formulation, capsule, tablet, ointment, granule, aerosol, round, dispersion, suspension, emulsion, eye drop, and bioembedded formulation.

The anti-CD3 antibody, etc. can be used in combination with other agents. The anti-CD3 antibody, etc. or pharmaceutical composition containing the anti-CD3 antibody, etc. can be administered simultaneously or separately with other agents, i.e., pharmaceutical compositions containing agents other than the anti-CD3 antibody, etc. as active ingredients. For example, the pharmaceutical composition containing the anti-CD3 antibody, etc. as an active ingredient may be administered after the administration of the other agent, the other agent may be administered after the administration of the pharmaceutical composition containing the anti-CD3 antibody, etc. as an active ingredient, or the pharmaceutical composition containing the anti-CD3 antibody, etc. as an active ingredient, may be administered simultaneously with the other agent. In the present invention, "pharmaceutical composition containing the anti-CD3 antibody, etc. and other agents" refers to both cases where the active ingredients of both the anti-CD3 antibody, etc. and other agents are contained in a single pharmaceutical composition and where both active ingredients are contained in multiple pharmaceutical compositions. In the present invention, such "pharmaceutical composition" is synonymous with "a pharmaceutical composition administered in combination with an anti-CD3 antibody, etc. and other agent".

In the present invention, when the anti-CD3 antibody, etc is "administered in combination" with other agents, it means that the anti-CD3 antibody, etc. and the other agents are taken into the body of the subject during a certain period of time. Formulations containing the anti-CD3 antibody, etc. and other agents in a single formulation may be administered, or each may be formulated separately and administered separately. When formulated separately, the timing of their administration is not limited, and they may be administered at the same time, at different times, or on different days. If the anti-CD3 antibody, etc, and the other agents are administered at different times or on different days, the order of their administration is not limited. Since each formulation is usually administered according to its own method of administration, they may be administered at the same or different times. When each is formulated separately, the administration method (route of administration) of each formulation may be the same or may be administered by different methods (routes of administration). In addition, it is not necessary for the anti-CD3 antibody, etc. and the other agent to be present in the body at the same time; they need only be taken into the body over a certain period of time (e.g., one month, preferably one week, even more preferably several days, even more preferably one day), and the other active ingredient may be lost from the body at the time of either administration.

The dosage forms of "pharmaceutical compositions administered in combination with the anti-CD3 antibody, etc. and other agents" include, for example, 1) administration of a single preparation containing an anti-CD3 antibody, etc. and other agent, 2) simultaneous administration of two preparations obtained by separately formulating an anti-CD3 antibody, etc, and other agent through the same route of administration, 3) administration of two preparations obtained by separately formulating an anti-CD3 antibody, etc. and other agent through the same route of administration with a time lag, 4) simultaneous administration of two preparations obtained by separately formulating an anti-CD3 antibody, etc. and other agent through different routes of administration, and 5) administration of two preparations obtained by separately formulating an anti-CD3 antibody, etc, and other agent through different routes of administration, with a time lag. The dosase, interval between administrations, dosage form, formulation, etc. of "pharmaceutical compositions administered in combination with the anti-CD3 antibody, etc. and other agents" can be according to those for pharmaceutical compositions comprising anti-CD3 antibodies, but not limited thereto.

Such pharmaceutical composition may be a kit containing them if they are made into two different formulations.

In the present invention, "combination" of an anti-CD3 antibody, etc. and other agent means that the anti-CD3 antibody, etc. and the other agent are "administered in combination".

Further other medicines may be used in the combination or pharmaceutical composition of the present invention.

The present invention also provides methods of treatment or prevention of diseases relating to CD3, the use of antibodies of the invention for preparing pharmaceutical compositions for treatment or prevention of such diseases, and the use of antibodies of the invention for the treatment or prevention of such diseases. A kit for the treatment or prevention containing the antibodies of the invention is also included in the present invention.

### Examples

The present invention will be more specifically described by way of Examples. However, the present invention is not limited by Examples.

### (Reference Example 1) Evaluation for solution stability of C3E-7085

C3E-7085 purified protein described in International Publication No. WO2018/117237 (scFv of which amino acid sequence is represented by SEQ ID NO: 64 in the sequence listing of International Publication No. WO2018/117237 and shown in Figure 72) was evaluated for solution stability. C3E-7085 was centrifugally concentrated by Amicon Ultra-4 (Millipore). After the buffer was exchanged to a buffer containing a 25 mM sodium acetate and 5% sorbitol (pH5.5), the concentration of the solution was adjusted to 10 mg/ml and stored at 4°C or 40°C for two weeks. The samples stored were subjected to size exclusion chromatography (SEC) using TSKgel UP-SW3000 (2 µm 4.6X 300 mm). The mobile phase was analyzed by use of 3XPBS at a flow rate of 0.2 ml/min (detection wavelength 280 nm). Peak analysis of HMWS contained in each sample was carried out and HMWS (%) was calculated in accordance with a peak area percentage method. The results are shown in Table 1.

**[Table 1]**

| C3E-7085 Storage condition | HMWS (%) |
|---|---|
| 40°C, Two weeks | 24 |
| 4°C, Two weeks | 22 |

In C3E-7085 samples stored at 4°C and 40°C for two weeks, the peak of a dimer particularly in HMWS increased up to 20% (data not shown). The dimer formed had reversibility, which is a nature to return to monomers by dilution (data not shown). Due to this, it was considered that generation of HMWS including a dimer is a problem to be solved for obtaining solution stability.

### (Example 1) Evaluation for solution stability of anti-CD3 antibody fragment-heterodimer Fc-type molecule

### 2)-1 Design of amino acid sequence of anti-CD3 antibody fragment

In order to improve physical properties for obtaining solution stability of C3E-7085, the format of C3E-7085-Fab was modified. The amino acid sequences of C3E-7085-Fab (SEQ ID NO: 1, Figure 5) and C3E-7085-LC (SEQ ID NO: 2, Figure 6) were designed. Also, a scFv having a disulfide introduction bond added thereto was designed with reference to literature (Proteins, 1994, May; 19 (1): 35-47) and designated as C3E-7096 (SEQ ID NO: 3, Figure 7).

### 2)-2 Preparation of anti-CD3 antibody fragment-heterodimer Fc expression vector

Various anti-CD3 antibody fragment-heterodimer Fc expression vectors were designed by using pcDNA3.1, pcDNA3.3 or pcDNA3.4 (Thermo Fisher Scientific) as a skeleton. As the anti-CD3 antibody fragment, C3E-7085 (WO2018/117237) and an anti-CD3 antibody fragment designed in the step 2)-1 were used. As the heterodimer Fc sequence (hereinafter referred to as "HC_h" and "HC_k"), the heterodimer Fc sequence reported in the literature (Nat Biotechnol. 1998, Jul; 16 (7)677-81.) was used.

An expression vector for mammalian cells was prepared by integrating a DNA fragment encoding the amino acid sequence comprised in a polypeptide consisting of Fc (HC_h) having a mutation for forming a heteromultimer introduced therein and designated as "p_HC_h".

An expression vector for mammalian cells was prepared by integrating a DNA fragment encoding the amino acid sequence comprised in a polypeptide, which is prepared by adding a Fc region having a mutation for forming a heteromultimer introduced therein, to the carboxyl terminal of C3E-7085, and designated as "p_C3E-7085-HC-k".

An expression vector for mammalian cells was prepared by integrating a DNA fragment encoding the amino acid sequence comprised in the polypeptide, which was prepared by adding a heavy chain variable region of C3E-7085, a human IgG derived CH1 region, a Fc region having a mutation for forming a heteromultimer introduced therein were added sequentially in this order and designated as "p_C3E-7085-Fab-HC-k". An expression vector for mammalian cells was prepared by integrating a DNA fragment encoding an amino acid sequence comprised in a polypeptide prepared by adding a human Ig Labmda-derived CL region to the carboxyl terminal of a C3E-7085 light chain variable region and designated as "p_C3E-7085-LC".

An expression vector for mammalian cells was prepared by integrating a DNA fragment encoding the amino acid sequence comprised in a polypeptide, which is prepared by adding a Fc region having a mutation for forming a heteromultimer introduced therein, to the carboxyl terminal of C3E-7096, and designated as "p_C3E-7096-HC-k"

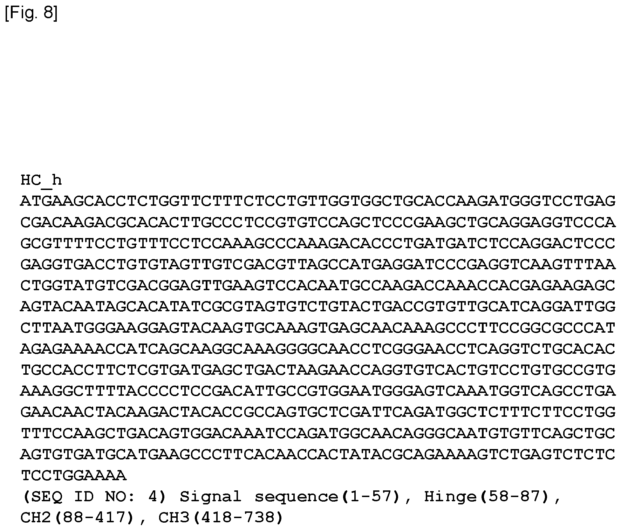

The nucleotide sequence of p_HC_h was reanalyzed. As a result, it was confirmed that the full-length nucleotide sequence of HC_h is the nucleotide sequence represented by SEQ ID NO: 4 (Figure 8) in the sequence listing.

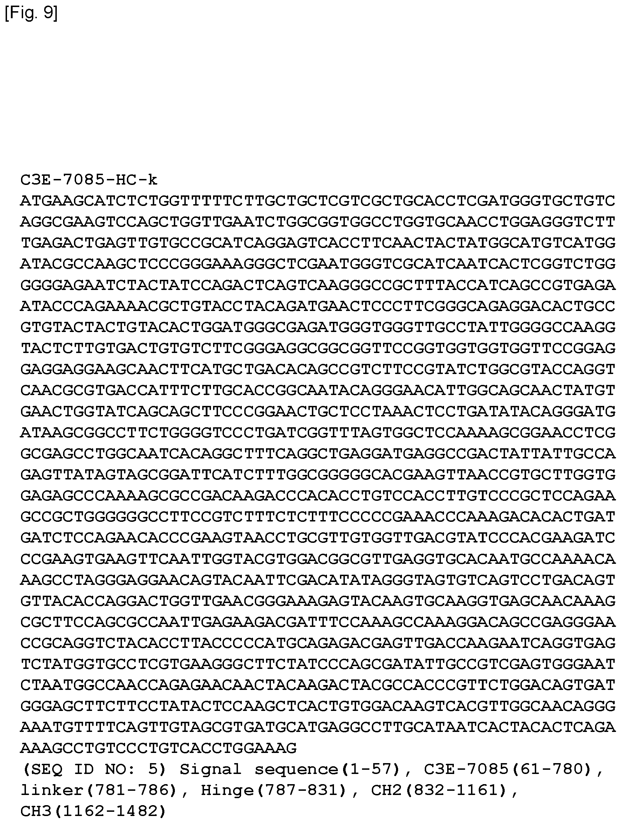

The nucleotide sequence of p_C3E-7085-HC-k was reanalyzed. As a result, it was confirmed that the full-length nucleotide sequence of C3E-7085-HC-k is the nucleotide sequence represented by SEQ ID NO: 5 (Figure 9) in the sequence listing.

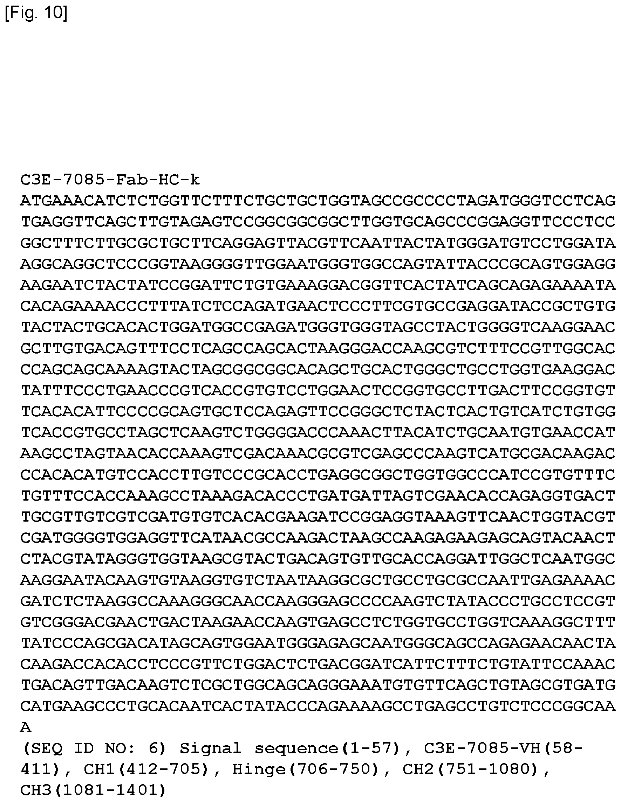

The nucleotide sequence of p_C3E-7085-Fab-HC-k was reanalyzed. As a result, it was confirmed that the full-length nucleotide sequence of C3E-7085-Fab-HC-k is the nucleotide sequence represented by SEQ ID NO: 6 (Figure 10) in the sequence listing.

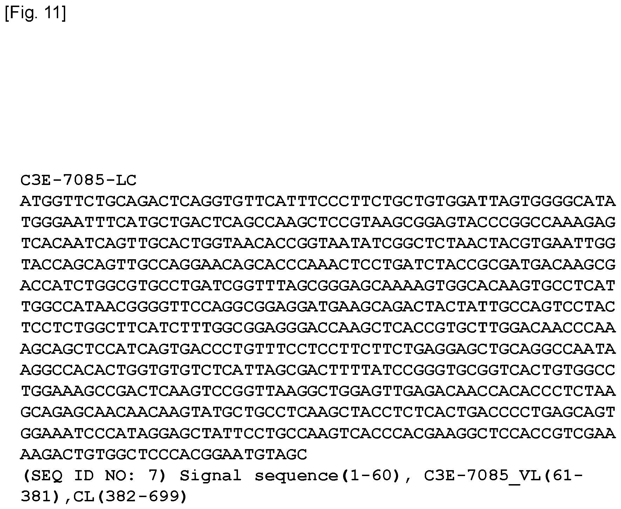

The nucleotide sequence of p_C3E-7085-LC was reanalyzed. As a result, it was confirmed that the full-length nucleotide sequence of C3E-7085-LC is the nucleotide sequence represented by SEQ ID NO: 7 (Figure 11) in the sequence listing.

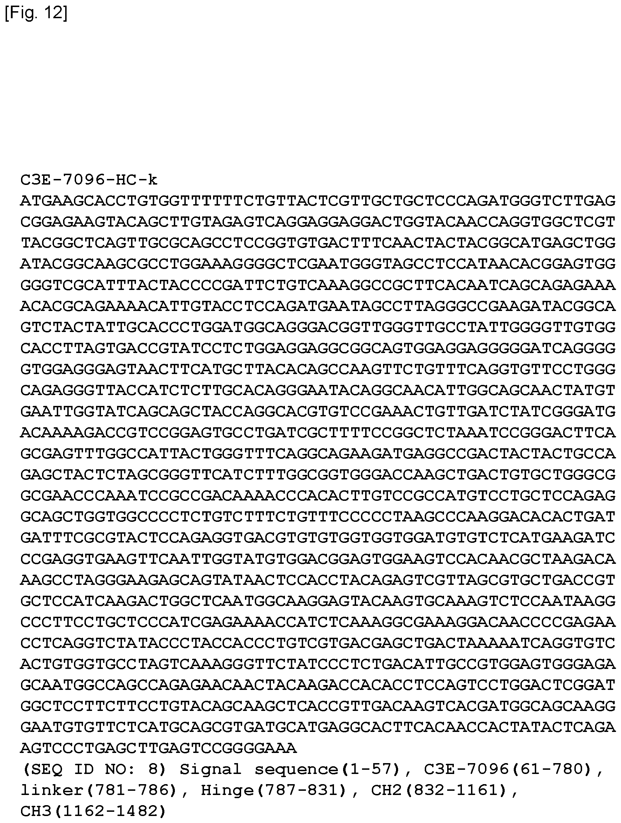

The nucleotide sequence of p_C3E-7096-HC-k was reanalyzed. As a result, it was confirmed that the full-length nucleotide sequence of C3E-7096-HC-k is the nucleotide sequence represented by SEQ ID NO: 8 (Figure 12) in the sequence listing.

Based on the above nucleotide sequences, the full-length amino acid sequences of HC_h, C3E-7085-HC-k, C3E-7085-Fab-HC-k, C3E-7085-LC and C3E-7096-HC-k encoded by the nucleotide sequences were confirmed.

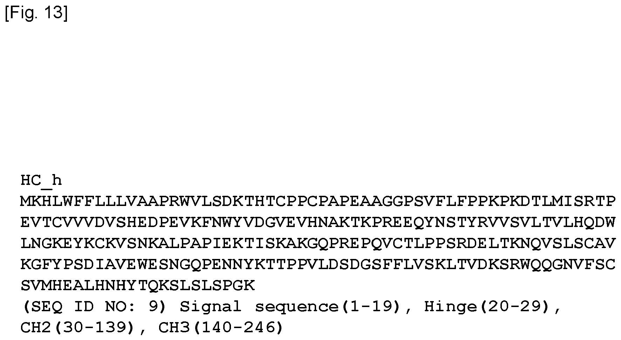

The full-length amino acid sequence of HC_h is the amino acid sequence represented by SEQ ID NO: 9 (Figure 13) of the sequence listing. In the sequence, the sequence of the 1st to 19th amino acids represents a signal sequence and the sequence of the 20th to 246th amino acids represents HC_h.

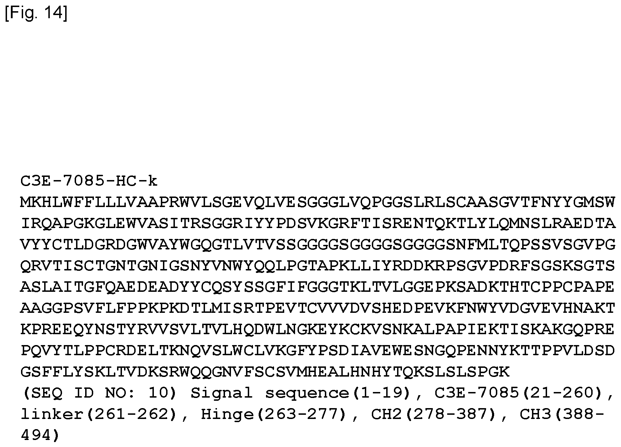

The full-length amino acid sequence of C3E-7085-HC-k is the amino acid sequence represented by SEQ ID NO: 10 (Figure 14) of the sequence listing. In the sequence, the sequence of the 1st to 19th amino acids represents a signal sequence, the sequence of the 21st to 260th amino acids represents C3E-7085; the sequence of the 261st to 262nd amino acids represents a linker, and the sequence of the 263rd to 494th amino acids represents HC-k.

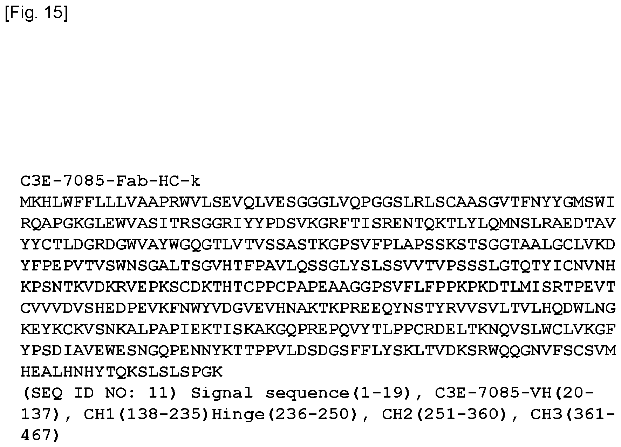

The full-length amino acid sequence of C3E-7085-Fab-HC-k is the amino acid sequence represented by SEQ ID NO: 11 (Figure 15) of the sequence listing. In the sequence, the sequence of the 1st to 19th amino acids represents a signal sequence, the sequence of the 20th to 137th amino acids represents C3E-7085-heavy chain variable region, and the sequence of the 138th to 467th amino acids represents a constant region.

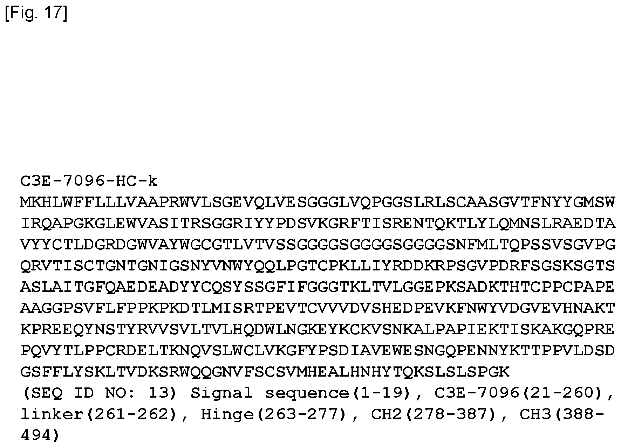

The full-length amino acid sequence of C3E-7085-LC is the amino acid sequence represented by SEQ ID NO: 12 (Figure 17) of the sequence listing. The sequence of the 1st to 20th amino acids represents a signal sequence, the sequence of the 21st to 127th amino acids represents a variable region, and the sequence of the 128th to 233rd amino acids represents a constant region.

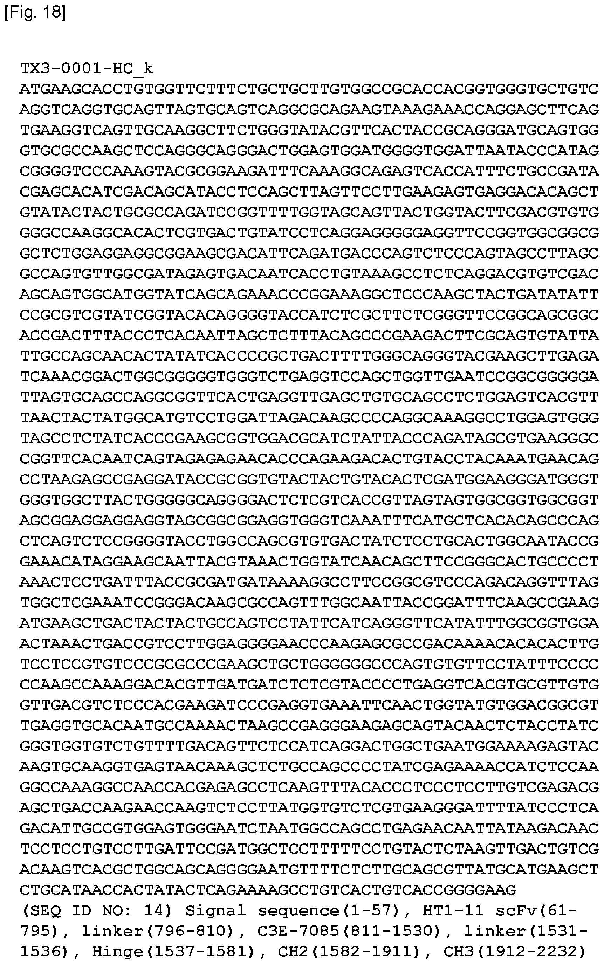

The full-length amino acid sequence of C3E-7096-HC-k is the amino acid sequence represented by SEQ ID NO: 13 (Figure 18) of the sequence listing. In the sequence, the sequence of the 1st to 19th amino acids represents a signal sequence; the sequence of the 21st to 260th amino acids represents C3E-7096; the sequence of the 261st to 262nd amino acids represents a linker, and the sequence of 263rd to 494th amino acids represents HC-k.

### 2)-3 Expression of anti-CD3 antibody fragment-heterodimer Fc

Expi293F cells (Thermo Fisher Scientific) were subcultured in accordance with the manual. The culture solution of the Expi293F cells in the logarithmic growth phase was taken, diluted with Expi293 Expression medium (Thermo Fisher Scientific) so as to contain 2.5 × 10⁶ cells/mL and used for producing various anti-CD3 antibody fragment-heterodimers Fc. In culturing 300 mL, 1.44 mg of polyethyleneimine (Polyscience #24765) was dissolved in 5 mL of Opti-Pro SFM (Thermo Fisher Scientific). Then, 0.3 mg of a vector mixture comprising vector p_C3E-7085-HC-k and p_HC_h prepared by a NucleoBond Xtra Midi kit (Takara Bio Inc.) was added to 5 mL of Opti-Pro SFM (Invitrogen). To 5 mL of the polyethyleneimine/Opti-Pro SFM mixed solution, 5 mL of the expression vector/Opti-Pro SFM mixed solution was added. The mixture was gently stirred, allowed to stand still for further 5 minutes, and then, added to Expi293F cells. The cells were subjected to shaking culture performed at 37°C in an 8% CO₂ incubator for 6 days at 135 rpm. The resultant culture supernatant was filtered by 0.2 µm filter (Thermo Fisher Scientific) to obtain an anti-CD3 antibody fragment-heterodimer Fc (C3E-0002) culture supernatant. The amino acid sequences constituting C3E-0002 and obtained by expressing individual vectors are represented by SEQ ID NO: 9 (Figure 13) and 10 (Figure 14) in the sequence listing.

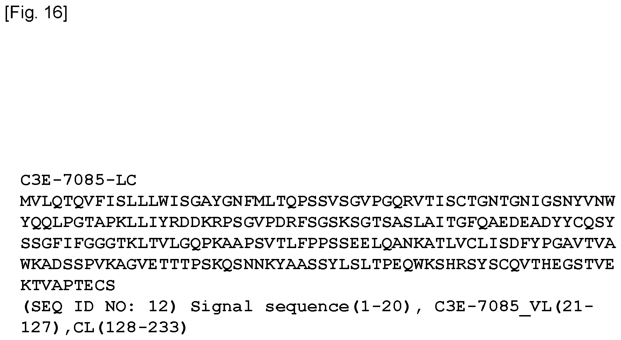

An anti-CD3 antibody fragment-heterodimer Fc (C3E-0003) was expressed by culturing using p_C3E-7085-Fab-HC-k, p_HC_h and p_C3E-7085-LC to prepare the culture supernatant of C3E-0003, in the same manner as above. The sequences of amino acids obtained by expressing individual vectors constituting C3E-0003 are represented by SEQ ID NO: 9 (Figure 13), SEQ ID NO: 11 (Figure 15) and SEQ ID NO: 12 (Figure 16) in the sequence listing.

An anti-CD3 antibody fragment-heterodimer Fc (C3E-0004) was expressed by culturing using p_C3E-7096-HC-k and p_HC_h to prepare the culture supernatant of C3E-0004, in the same manner as above. The sequences of amino acids obtained by expressing individual vectors constituting C3E-0004 are represented by SEQ ID NO: 9 (Figure 13), and SEQ ID NO: 13 (Figure 17) in the sequence listing.

### 2)-4 Purification of anti-CD3 antibody fragment-heterodimer Fc

An anti-CD3 antibody fragment-heterodimer Fc was purified from the culture supernatant obtained in the step 2)-3 by a two-step process of Protein A affinity chromatography and gel filtration chromatography.

The culture supernatant was applied to MabSelectSuRe column (Cytiva) equilibrated with PBS (pH7.4) to allow a desired anti-CD3 antibody fragment-heterodimer Fc to adsorb to the column. After non-adsorbed components were removed with PBS, the adsorbed component was eluted with an acetate buffer (pH3.5). After the pH of elution fraction was controlled to be neutral with a Tris buffer (pH9.5), the elution fraction was concentrated and subjected to gel filtration column, Superdex 200 10/300 (Cytiva) equilibrated in advance with 25 mM histidine, 300 mM NaCl, 5% sorbitol, (pH5.5). From the peak fractions obtained, a fraction corresponding to a desired heterodimer was recovered and analyzed by capillary electrophoresis (LabChip GX Touch, PerkinElmer). As a result, it was confirmed that the desired anti-CD3 antibody fragment-heterodimer Fc is formed. A protein sample purified was subjected to analytical SEC to determine the purity and concentration thereof and evaluated.

### 2)-5 Evaluation for solution stability of anti-CD3 antibody fragment-heterodimer Fc

Individual samples purified in the step 2)-4 were centrifugally concentrated by Amicon Ultra-4 (Millipore). After the buffer was exchanged to PBS, the concentration of the samples were adjusted to be 20 mg/ml with PBS to prepare samples for evaluation. The HMWS (%) of each sample at the time of initiation of evaluation was obtained by size exclusion chromatography using AdvanceBio SEC 300A 2.7 µm 4.6 × 150 mm (Agilent) and calculated by the peak area percentage method. As the mobile phase, 0.2 M Ki/200 mM KCl/pH7.0 was used. Analysis was made at a flow rate of 0.2 ml/min (detection wavelength: 280 nm). The accelerated deterioration test of individual samples was carried out as follows. The samples were stored at 40°C and for 1, 7 and 14 days, and thereafter, subjected to size exclusion chromatography performed in the same conditions as above. The HMWS values (%) of individual samples were calculated by the peak area percentage method.

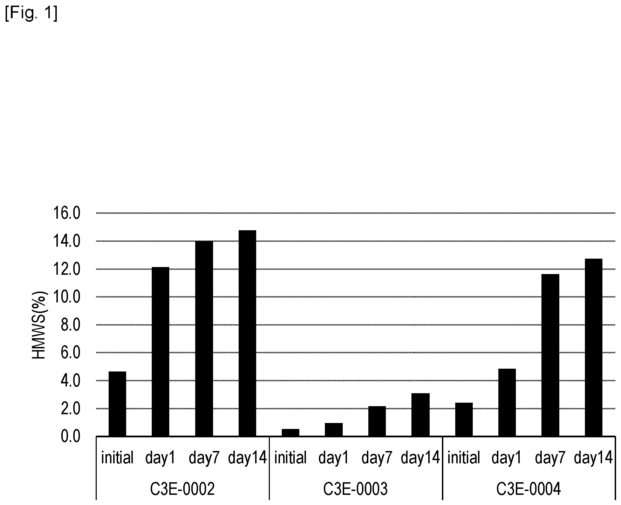

The results of the time-course changes in HMWS of individual samples stored at 40°C are shown in Figure 1. HMWS (%) of C3E-0003 after storage of 14-days was 3%. From this, it was found that an increase of HMWS (%) with time of storage was significantly low compared to 15% of C3E-0002. HMWS (%) of C3E-0004 after storage of 14-days was 13%, which was high compared to C3E-0003 but slightly low compared to C3E-0002.

The above results demonstrate that the heterodimer Fc-type molecule using C3E-7085 Fab format and the heterodimer Fc-type molecule using C3E-7096 are excellent in solution stability compared to the heterodimer Fc-type molecule using C3E-7085.

### (Example 2) Evaluation for shaking stability of anti-CD3 antibody fragment-heterodimer Fc

After individual samples purified in the step 2)-4 were centrifugally concentrated by Amicon Ultra-4 (Millipore), the concentration of the samples was adjusted to be 2 mg/ml to prepare evaluation samples. The peak area and HMWS (%) of each sample at the time of initiation of evaluation were obtained by size exclusion chromatography using AdvanceBio SEC 300A 2.7 µm 4.6 × 150 mm (Agilent) and calculated by the peak area percentage method. As the mobile phase, 0.2 M Ki/200 mM KCl/pH7.0 was used. Analysis was made at a flow rate of 0.2 ml/min (detection wavelength: 280 nm). The shaking deterioration test was carried out by shaking the samples by a universal high speed mixer (manufactured by Waken) at room temperature and 2500 rpm for one hour and two hours and subjecting the samples to size exclusion chromatography performed in the same conditions as above. The peak areas and HMWS (%) of the samples were calculated.

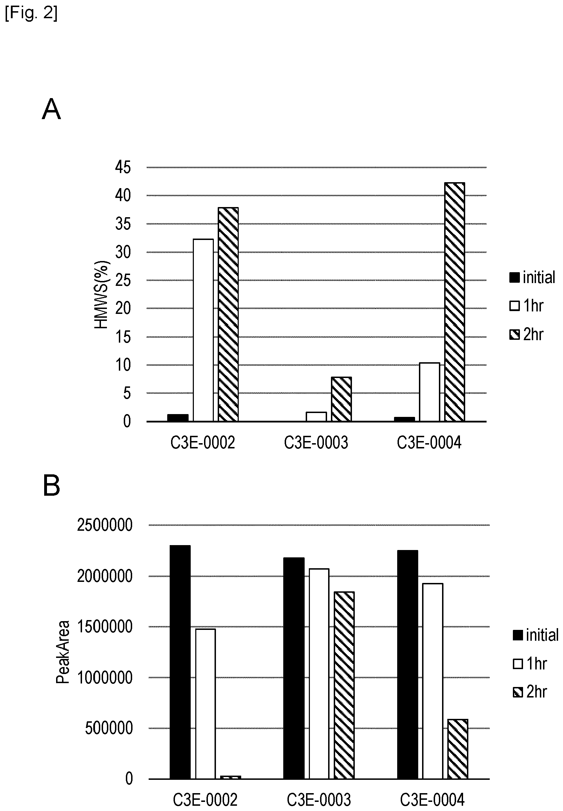

The HMWS (%) and peak area of the samples after shaking are shown in Figure 2A and Figure 2B, respectively. HMWS (%) of C3E-0002 sample shaken for one hour was remarkably high. In the sample shaken for two hours, 99% of the peak area of the initial time disappeared. A decrease in the peak-area and an increase of HMWS (%) with the elapse of shaking time in C3E-0003 were significantly low compared to those of C3E-0002. In the case of C3E-0004, HMWS (%) of the sample shaken for one hour was low and the disappearance of peak area of sample shaken for two hours was still 74%, compared to C3E-0002. To confirm whether particles having a size that cannot be detected by size exclusion chromatography are formed or not after shaking, the numbers of particles of samples before shaking and samples shaken for two hours were counted by FlowCAM8100 (made in DKSH Japan). As a result, the number of particles increased after shaking. From this, it was estimated that a monomer is formed into particles via HMWS by shaking deterioration. From the results, it can be said that a sample exhibiting a low increase rate of HMWS (%) and low decrease rate of peak area has high stability to shaking deterioration.

From the above results, it was demonstrated that the heterodimer Fc-type molecule using C3E-7085 Fab format and the heterodimer Fc-type molecule using C3E-7096 are excellent in shaking stability, compared to the heterodimer Fc-type molecule using C3E-7085.

### (Example 3) Preparation oftaFv-heterodimer Fc-type and scFv-Fab-heterodimer Fc-type bispecific molecules

### 4)-1 Preparation of vector for expressing taFv-heterodimer Fc-type bispecific molecule

Various taFv-heterodimer Fc-type bispecific molecules expression vectors were designed by using pcDNA3.1, pcDNA3.3 or pcDNA3.4 (Thermo Fisher Scientific) as a skeleton. As the anti-CD3 antibody fragment, C3E-7085 (WO2018/117237) or the heterodimer Fc sequences (hereinafter referred to as HC_h and HC_k) using C3E-7096, the sequences reported in the literature (Nat Biotechnol. 1998, Jul; 16 (7) 677-81) were used.

As an anti-Trop2-CD3 bispecific molecule expression vector, an expression vector for mammalian cells was prepared by integrating a DNA fragment encoding the amino acid sequence comprised in a polypeptide, which is prepared by adding a Fc region, in which a mutation for forming a heteromultimer was introduced, to the carboxyl terminal of a taFv, which is prepared by connecting HT1-11 scFv disclosed in International Publication No. WO2018/117237 and C3E-7085 via a GGGGS linker. The expression vector was designated as "p_TX3-0001-HC_k". Also, an expression vector for mammalian cells was prepared by integrating a DNA fragment encoding the amino acid sequence comprised in a polypeptide, which is prepared by adding a Fc region, in which a mutation for forming a heteromultimer was introduced, to the carboxyl terminal of a taFv, which is prepared by connecting HT1-11 scFv and C3E-7096 via a GGGGS linker. The expression vector was designated as "p_TX3-0003-HC_k".

As an anti-CD98-CD3 bispecific molecule expression vector, an expression vector for mammalian cells was prepared by integrating a DNA fragment encoding the amino acid sequence comprised in a polypeptide, which is prepared by adding a Fc region, in which a mutation for forming a heteromultimer was introduced, to the carboxyl terminal of a taFv, which is prepared by connecting anti-CD98 scFv (hereinafter referred to as "hM23-H1L1 scFv"), which is prepared by connecting a sequence prepared by adding glycine to the N terminal of hM23-H1 heavy chain variable region disclosed in JP Patent Publication (Kokai) No.2017-114763 A and hM23-L1 light chain variable region via a polypeptide linker having three repeats of (GGGGS), and C3E-7085 via a GGGGS linker. The expression vector was designated as "p_CX3-0001-HC_k". Also, an expression vector for mammalian cells was prepared by integrating a DNA fragment encoding the amino acid sequence comprised in a polypeptide, which is prepared by adding a Fc region, in which a mutation for forming a heteromultimer was introduced, to the carboxyl terminal of a taFv, which is prepared by connecting hM23-H1L1 scFv and C3E-7096 via a GGGGS linker. The expression vector was designated as "p_CX3-0003-HC_k".

As an anti-GPRC5D-CD3 bispecific molecule expression vector, an expression vector for mammalian cells was prepared by integrating a DNA fragment encoding the amino acid sequence comprised in a polypeptide, which is prepared by adding a Fc region, in which a mutation for forming a heteromultimer was introduced, to the carboxyl terminal of a taFv, which is prepared by connecting C3022 scFv disclosed in International Publication No. WO2018/147245 and C3E-7085 via a GGGGS linker. The expression vector was designated as "p_RX3-0001-HC_k". Also, an expression vector for mammalian cells was prepared by integrating a DNA fragment encoding the amino acid sequence comprised in a polypeptide, which is prepared by adding a Fc region, in which a mutation for forming a heteromultimer was introduced, to the carboxyl terminal of a taFv, which is prepared by connecting C3022 scFv and C3E-7096 via a GGGGS linker. The expression vector was designated as "p_RX3-0003-HC_k".

The nucleotide sequence of p_TX3-0001-HC_k was reanalyzed. As a result, it was confirmed that the full-length nucleotide sequence of TX3-0001-HC_k is the nucleotide sequence represented by SEQ ID NO: 14 (Figure 18) in the sequence listing.

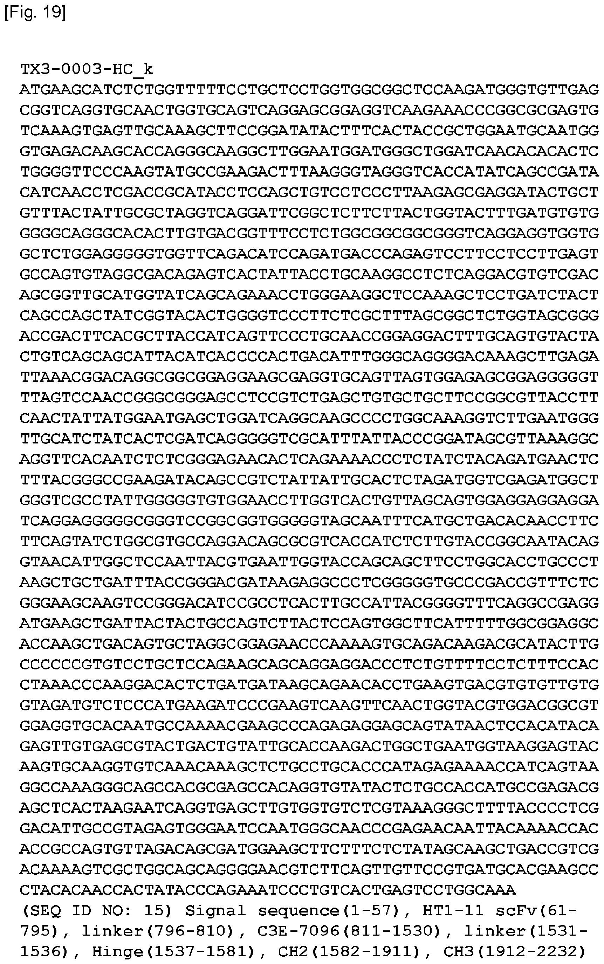

The nucleotide sequence of p_TX3-0003-HC_k was reanalyzed. As a result, it was confirmed that the full-length nucleotide sequence of TX3-0003-HC_k is the nucleotide sequence represented by SEQ ID NO: 15 (Figure 19) in the sequence listing.

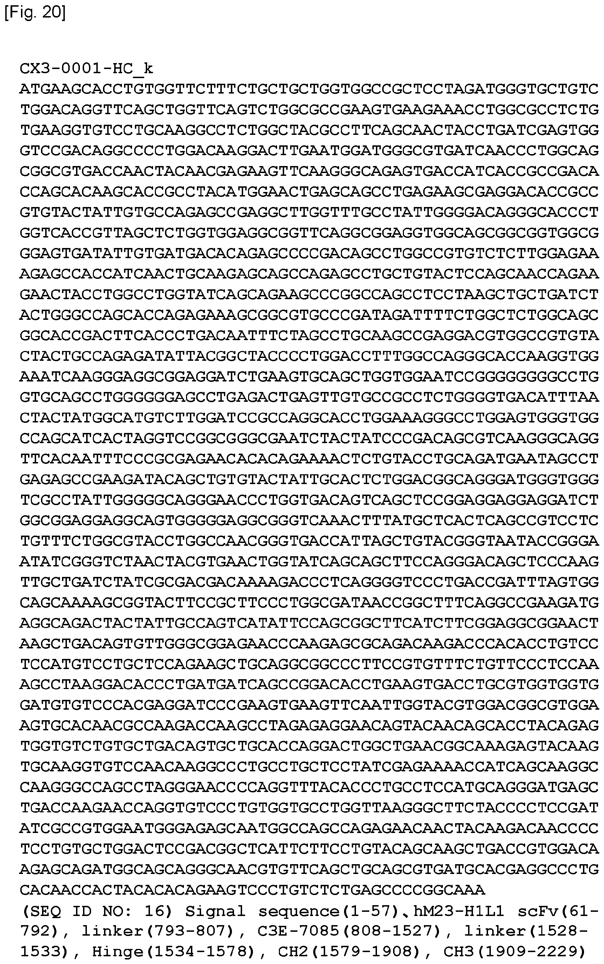

The nucleotide sequence of p_CX3-0001-HC_k was reanalyzed. As a result, it was confirmed that the full-length nucleotide sequence of CX3-0001-HC_k is the nucleotide sequence represented by SEQ ID NO: 16 (Figure 20) in the sequence listing.

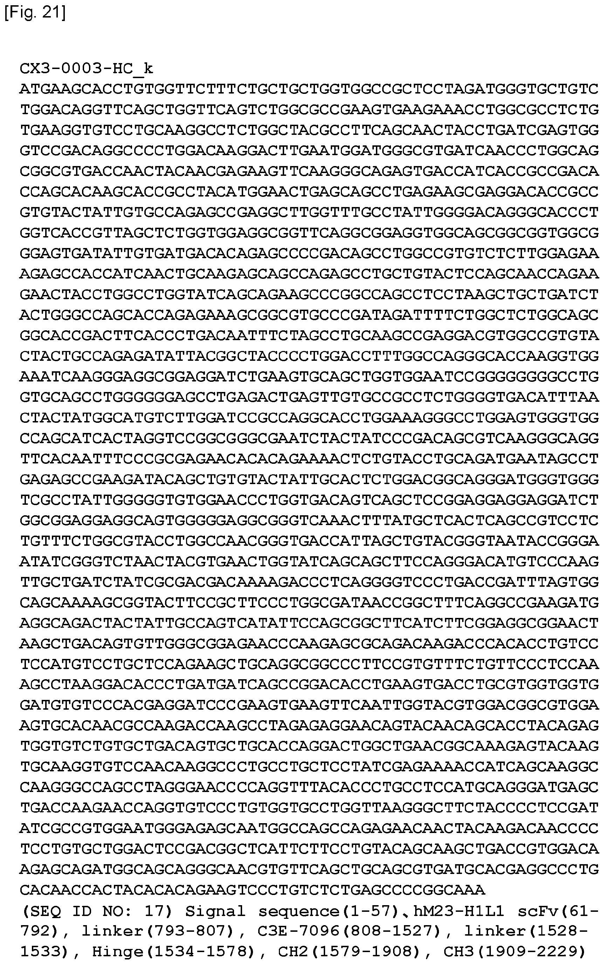

The nucleotide sequence of p_CX3-0003-HC_k was reanalyzed. As a result, it was confirmed that the full-length nucleotide sequence of CX3-0003-HC_k is the nucleotide sequence represented by SEQ ID NO: 17 (Figure 21) in the sequence listing.

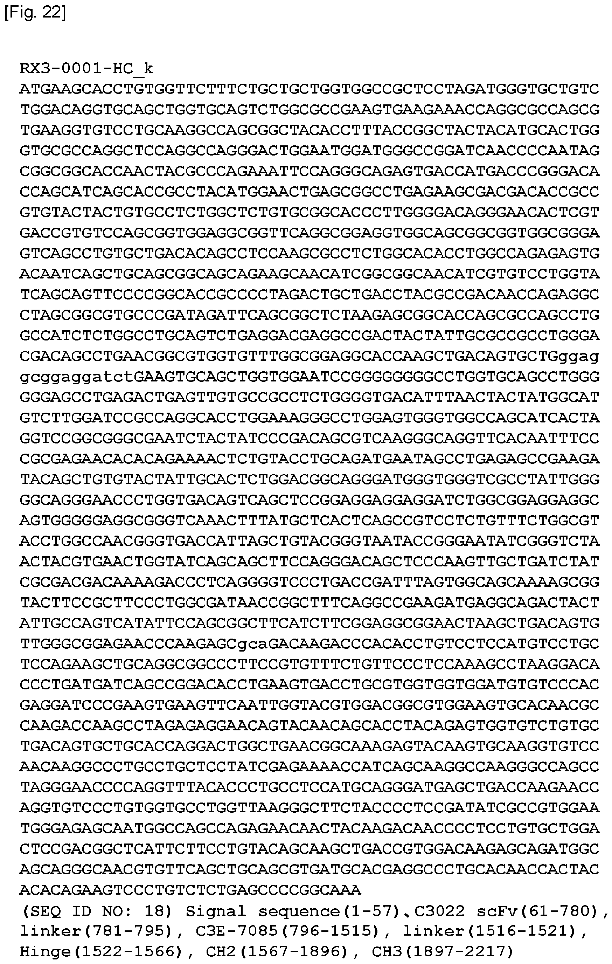

The nucleotide sequence of p_RX3-0001-HC_k was reanalyzed. As a result, it was confirmed that the full-length nucleotide sequence of RX3-0001-HC_k is the nucleotide sequence represented by SEQ ID NO: 18 (Figure 22) in the sequence listing.

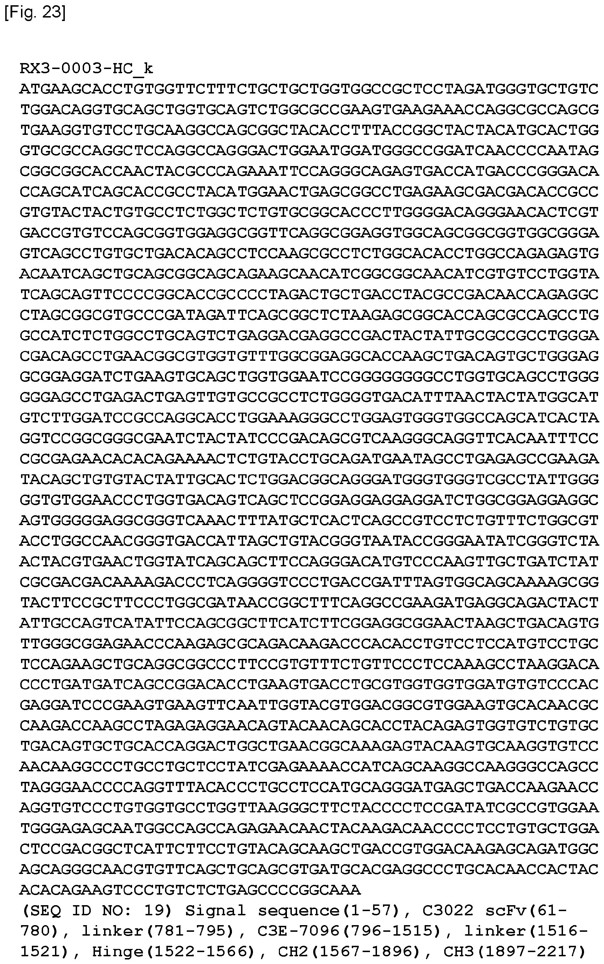

The nucleotide sequence of p_RX3-0003-HC_k was reanalyzed. As a result, it was confirmed that the full-length nucleotide sequence of RX3-0003-HC_k is the nucleotide sequence represented by SEQ ID NO: 19 (Figure 23) in the sequence listing.

The full-length amino acid sequences ofTX3-0001-HC_k, TX3-0003-HC_k, CX3-0001-HC_k, CX3-0003-HC_k, RX3-0001-HC_k and RX3-0003-HC_k encoded by the above nucleotide sequences were also confirmed based on the nucleotide sequences.

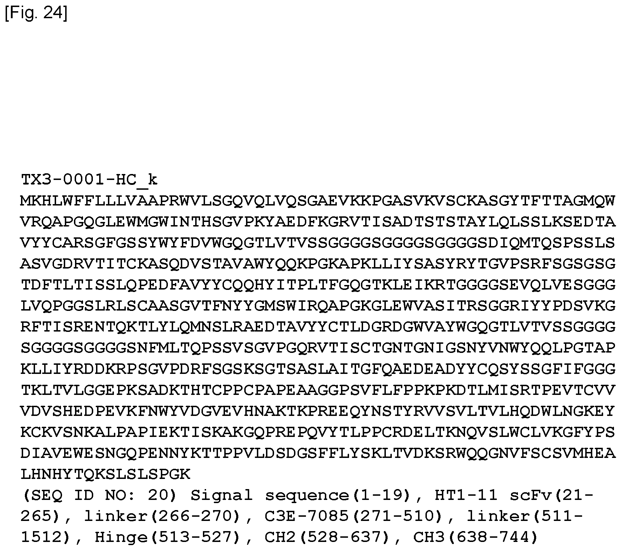

The full-length amino acid sequence of TX3-0001-HC_k is the amino acid sequence represented by SEQ ID NO: 20 (Figure 24) in the sequence listing. In the sequence, the sequence of the 1st to 19th amino acids represents a signal sequence, the sequence of the 21st to 510th amino acids represents a taFv obtained by connecting HT1-11 scFv and C3E-7085 by a GGGGS linker; the sequence of the 511st to 512nd amino acids represents a linker, and the sequence of the 513rd to 744th amino acids represents HC-k.

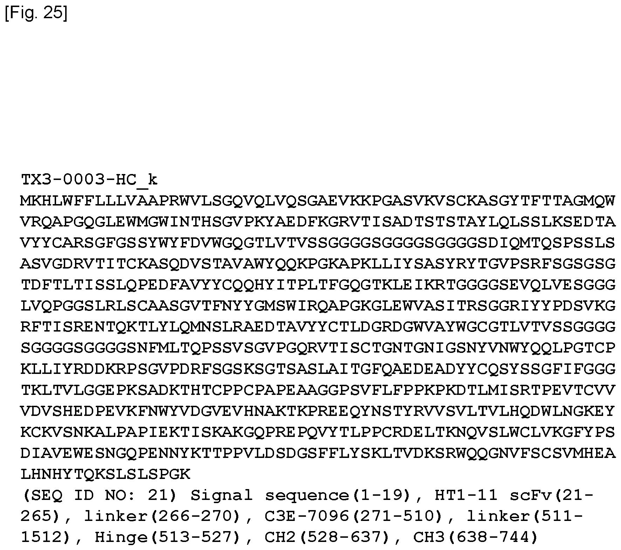

The full-length amino acid sequence of TX3-0003-HC_k is the amino acid sequence represented by SEQ ID NO: 21 (Figure 25) in the sequence listing. In the sequence, the sequence of the 1st to 19th amino acids represents a signal sequence, the sequence of the 21st to 510th amino acids represents a taFv obtained by connecting HT1-11scFv and C3E-7096 by a GGGGS linker; the sequence of the 511st to 512nd amino acids represents a linker, and the sequence of the 513rd to 744th amino acids represents HC-k.

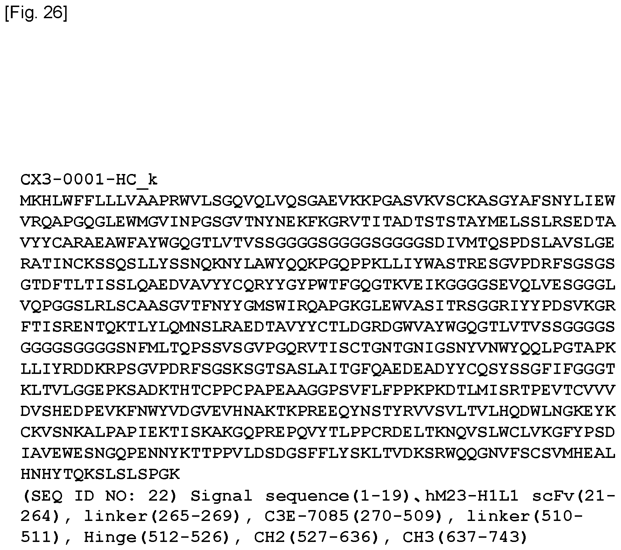

The full-length amino acid sequence of CX3-0001-HC_k is the amino acid sequence represented by SEQ ID NO: 22 (Figure 26) in the sequence listing. In the sequence, the sequence of the 1st to 19th amino acids represents a signal sequence, the sequence of the 21st to 509th amino acids represents a taFv obtained by connecting hM23-H1L1 scFv and C3E-7085 by a GGGGS linker; the sequence of the 510th to 511st amino acids represents a linker, and the sequence of the 512nd to 743rd amino acids represents HC-k.

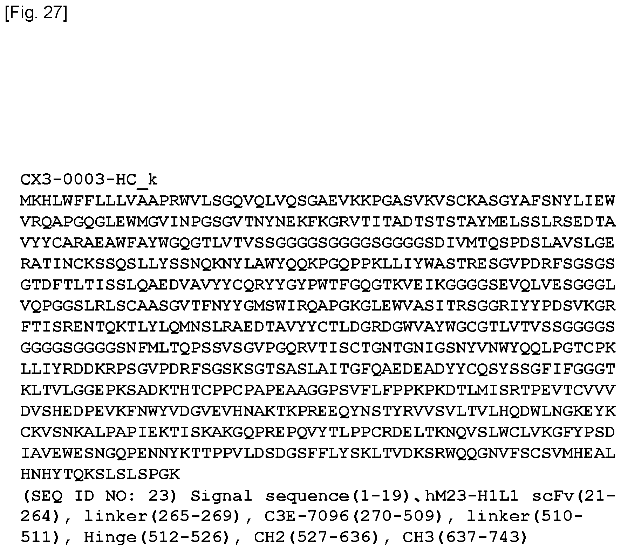

The full-length amino acid sequence of CX3-0003-HC_k is the amino acid sequence represented by SEQ ID NO: 23 (Figure 27) in the sequence listing. In the sequence, the sequence of the 1st to 19th amino acids represents a signal sequence, the sequence of the 21st to 509th amino acids represents a taFv obtained by connecting hM23-H1L1 scFv and C3E-7096 by a GGGGS linker; the sequence of the 510th to 511st amino acids represents a linker, and the sequence of the 512nd to 743rd amino acids represents HC-k.

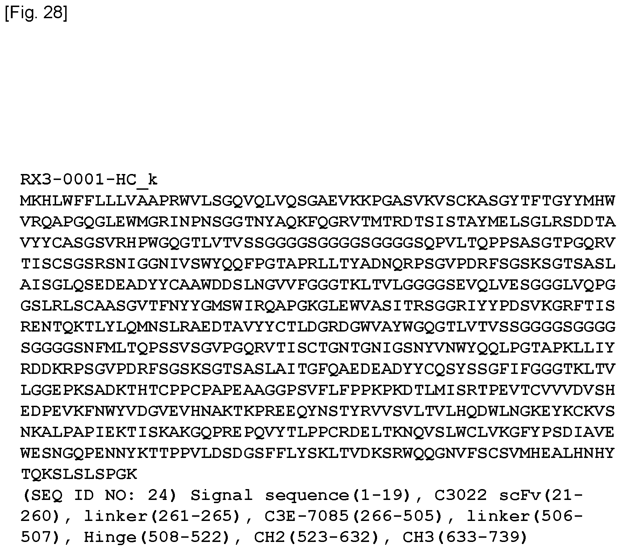

The full-length amino acid sequence of RX3-0001-HC_k is the amino acid sequence represented by SEQ ID NO: 24 (Figure 28) in the sequence listing. In the sequence, the sequence of the 1st to 19th amino acids represents a signal sequence, the sequence of the 21st to 505th amino acids represents a taFv obtained by connecting C3022 scFv and C3E-7085 by a GGGGS linker; the sequence of the 506th to 507th amino acids represents a linker, and the sequence of the 508th to 739th amino acids represents HC-k.

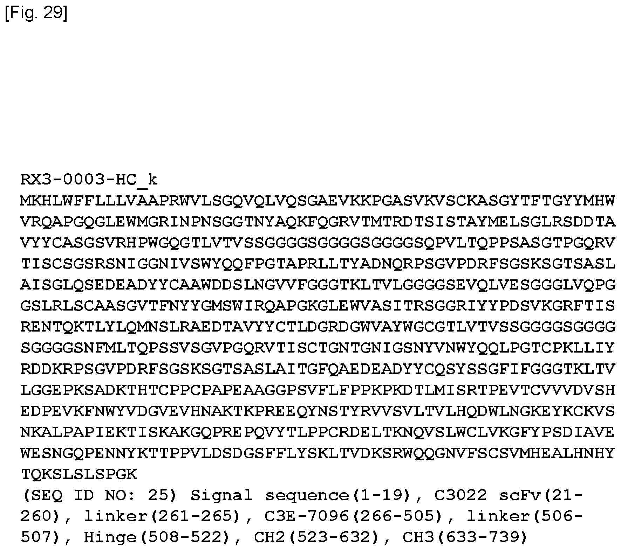

The full-length amino acid sequence of RX3-0003-HC_k is the amino acid sequence represented by SEQ ID NO: 25 (Figure 29) in the sequence listing. In the sequence, the sequence of the 1st to 19th amino acids represents a signal sequence, the sequence of the 21st to 505th amino acids represents a taFv obtained by connecting C3022 scFv and C3E-7096 by a GGGGS linker; the sequence of the 506th to 507th amino acids represents a linker, and the sequence of the 508th to 739th amino acids represents HC-k.

### 4)-2 Expression of taFv-heterodimer Fc-type bispecific molecule

An anti-Trop2-CD3taFv-heterodimer Fc (TX3-0001) was expressed by culturing using p_TX3-0001-HC_k and p_HC_h to prepare the culture supernatant of TX3-0001, in the same manner as in step 2)-3. The sequences of amino acids obtained by expressing individual vectors constituting TX3-0001 are represented by SEQ ID NO: 9 (Figure 13) and 20 (Figure 24) in the sequence listing.

An anti-Trop2-CD3taFv-heterodimer Fc (TX3-0003) was expressed by culturing using p_TX3-0003-HC_k and p_HC_h to prepare the culture supernatant of TX3-0003, in the same manner as above. The sequences of amino acids obtained by expressing individual vectors constituting TX3-0003 are represented by SEQ ID NO: 9 (Figure 13) and 21 (Figure 25) in the sequence listing.

An anti-CD98-CD3taFv-heterodimer Fc (CX3-0001) was expressed by culturing using p_CX3-0001-HC_k and p_HC_h to prepare the culture supernatant of CX3-0001, in the same manner as above. The sequences of amino acids obtained by expressing individual vectors constituting CX3-0001 are represented by SEQ ID NO: 9 (Figure 13) and 22 (Figure 26) in the sequence listing.

An anti-CD98-CD3taFv-heterodimer Fc (CX3-0003) was expressed by culturing using p_CX3-0003-HC_k and p_HC_h to prepare the culture supernatant of CX3-0003, in the same manner as above. The sequences of amino acids obtained by expressing individual vectors constituting CX3-0003 are represented by SEQ ID NO: 9 (Figure 13) and 23 (Figure 27) in the sequence listing.

An anti-GPRC5D-CD3taFv-heterodimer Fc (RX3-0001) was expressed by culturing using p_RX3-0001-HC_k and p_HC_h to prepare the culture supernatant of RX3-0001, in the same manner as above. The sequences of amino acids obtained by expressing individual vectors constituting RX3-0001 are represented by SEQ ID NO: 9 (Figure 13) and 24 (Figure 28) in the sequence listing.

An anti-CD98-CD3taFv-heterodimer Fc (RX3-0003) was expressed by culturing using p_RX3-0003-HC_k and p_HC_h to prepare the culture supernatant of RX3-0003, in the same manner as above. The sequences of amino acids obtained by expressing individual vectors constituting RX3-0003 are represented by SEQ ID NO: 9 (Figure 13) and 25 (Figure 29) in the sequence listing.

### 4)-3 Purification of taFv-heterodimer Fc-type bispecific molecule

Individual culture supernatants obtained in step 4)-2 were purified in the same manner as in step 2)-4. The purified protein samples were each subjected to analytical SEC to determine the purity and concentration and used for various evaluations.

### 4)-4 Preparation of scFv-Fab-heterodimer Fc-type bispecific molecule expression vector

Various scFv-Fab-heterodimer Fc-type bispecific molecule expression vectors were designed by using pcDNA3.1, pcDNA3.3 or pcDNA3.4 (Thermo Fisher Scientific) as a skeleton. An anti-CD3 antibody fragment was used by changing C3E-7085 (WO2018/117237) into a Fab format. As the heterodimer Fc sequence (hereinafter referred to as HC_h and HC_k), the sequence reported in the literature (Nat Biotechnol. 1998, Jul; 16 (7)677-81.) was used.

As an anti-Trop2-CD3 bispecific molecule expression vector, an expression vector for mammalian cells was prepared by integrating a DNA fragment encoding an amino acid sequence comprised in a polypeptide, which is prepared by adding a HT1-11 scFv, a C3E-7085 heavy-chain variable region, a human IgG derived CH1 region and a Fc region having a mutation for forming a heteromultimer introduced therein, sequentially in this order, and designated as "p_TX3-0002-HC_k".

As an anti-CD98-CD3 bispecific molecule expression vector, an expression vector for mammalian cells was prepared by integrating a DNA fragment encoding an amino acid sequence comprised in a polypeptide which is prepared by adding a hM23-H1L1 scFv, a C3E-7085 heavy chain variable region, a human IgG derived CH1 region, and a Fc region having a mutation for forming a heteromultimer introduced therein, sequentially in this order, and designated as "p_CX3-0002-HC_k".

As an anti-GPRC5D-CD3 bispecific molecule expression vector, an expression vector for mammalian cells was prepared by integrating a DNA fragment encoding an amino acid sequence comprised in a polypeptide which is prepared by adding a C3022 scFv, a C3E-7085 heavy chain variable region, a human IgG derived CH1 region, and a Fc region having a mutation for forming a heteromultimer introduced therein, sequentially in this order, and designated as "p_RX3-0002-HC_k".

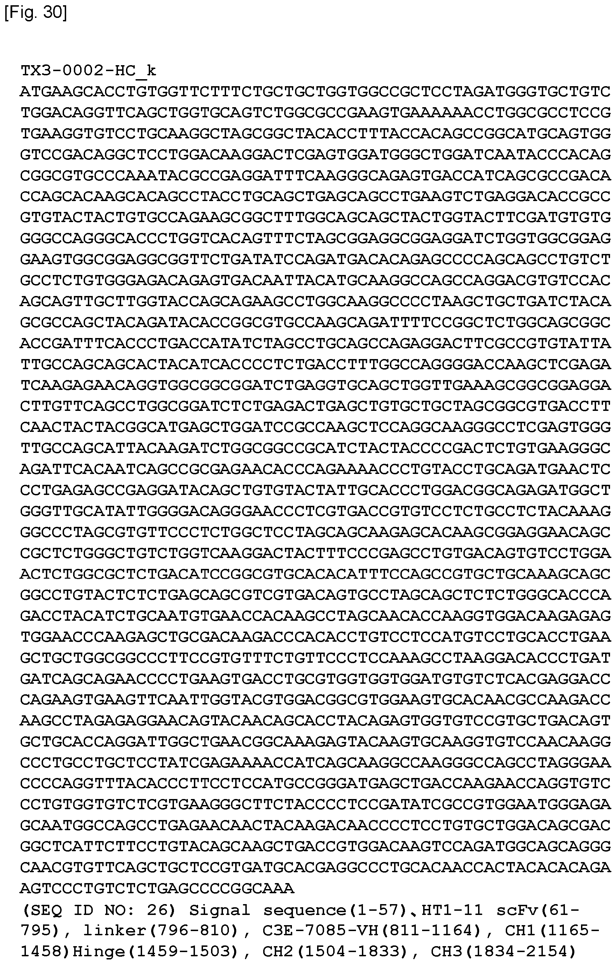

The nucleotide sequence of p_TX3-0002-HC_k was reanalyzed. As a result, it was confirmed that the full-length nucleotide sequence of TX3-0002-HC_k is the nucleotide sequence represented by SEQ ID NO: 26 (Figure 30) in the sequence listing.

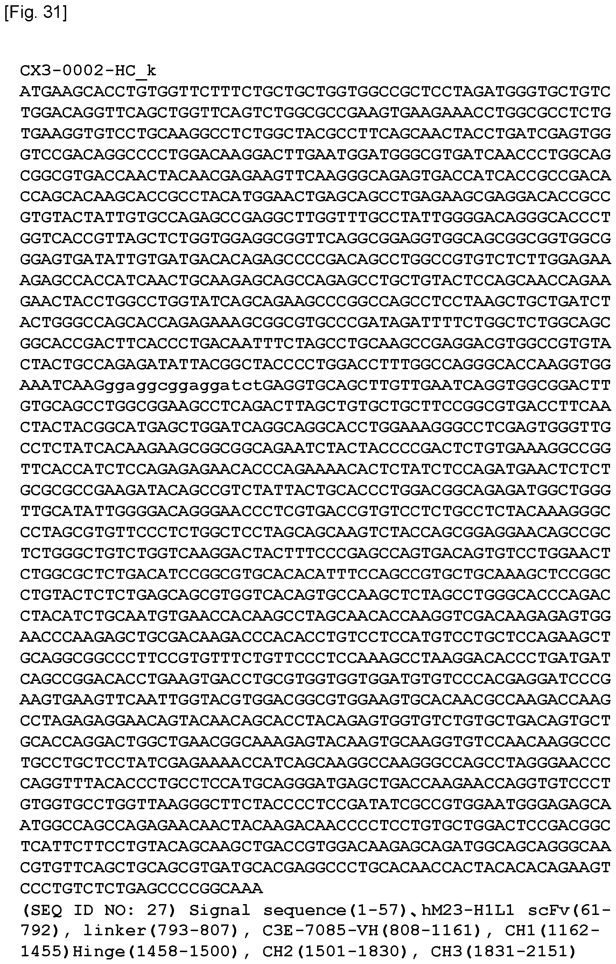

The nucleotide sequence of p_CX3-0002-HC_k was reanalyzed. As a result, it was confirmed that the full-length nucleotide sequence of CX3-0002-HC_k is the nucleotide sequence represented by SEQ ID NO: 27 (Figure 31) in the sequence listing.

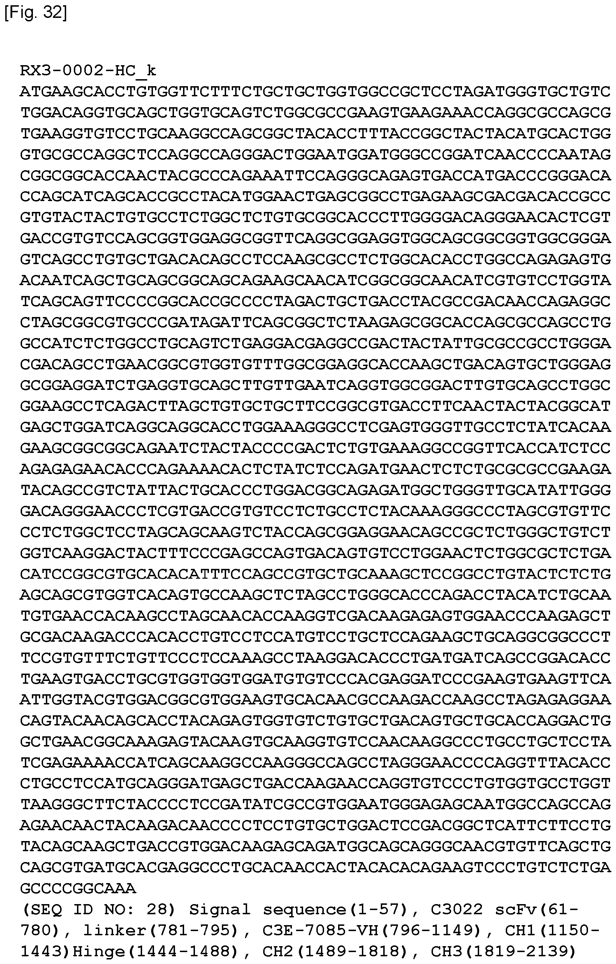

The nucleotide sequence of p_RX3-0002-HC_k was reanalyzed. As a result, it was confirmed that the full-length nucleotide sequence of RX3-0002-HC_k is the nucleotide sequence represented by SEQ ID NO: 28 (Figure 32) in the sequence listing.

Based on the above nucleotide sequences, the full-length amino acid sequences of TX3-0002-HC_k, CX3-0002-HC_k and RX3-0002-HC_k encoded by the nucleotide sequences were confirmed.

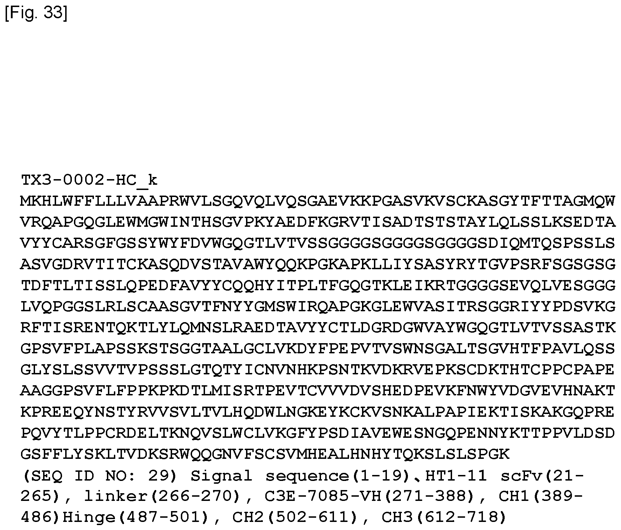

The full-length amino acid sequence of TX3-0002-HC_k is the amino acid sequence represented by SEQ ID NO: 29 (Figure 33) in the sequence listing. In the sequence, the sequence of the 1st to 19th amino acids represents a signal sequence, the sequence of the 21st to 265th amino acids represents HT1-11scFv; the sequence of the 266th to 270th amino acids represents a linker, and the sequence of the 271st to 388th amino acids represents C3E-7085-heavy chain variable region. The sequence of the 389th to 718th amino acids represents a constant region.

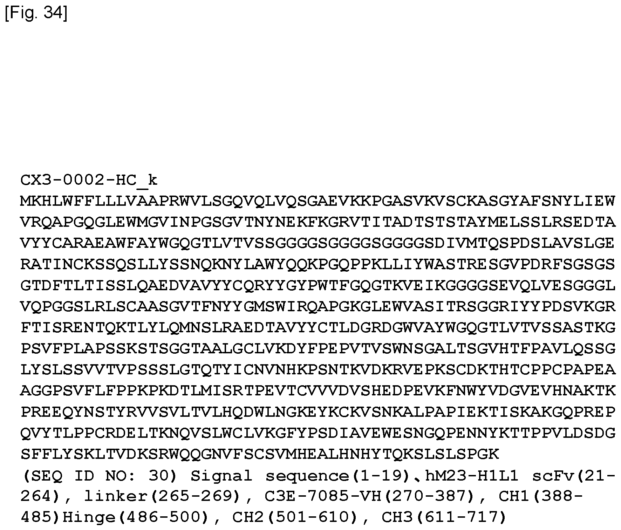

The full-length amino acid sequence of CX3-0002-HC_k is the amino acid sequence represented by SEQ ID NO: 30 (Figure 34) in the sequence listing. In the sequence, the sequence of the 1st to 19th amino acids represents a signal sequence, the sequence of the 21st to 264th amino acids represents hM23-H1L1scFv, the sequence of the 265th to 269th amino acids represents a linker, and the sequence of the 270th to 387th amino acids represents C3E-7085-heavy chain variable region. The sequence of the 388th to 717th amino acids represents a constant region.

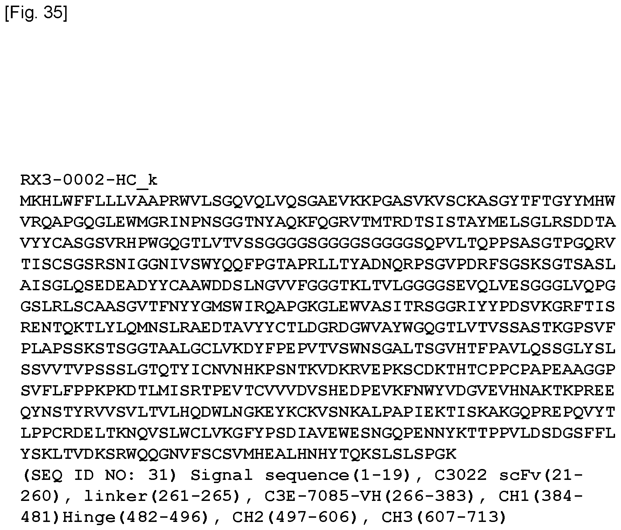

The full-length amino acid sequence of RX3-0002-HC_k is the amino acid sequence represented by SEQ ID NO: 31 (Figure 35) of the sequence listing. In the sequence, the sequence of the 1st to 19th amino acids represents a signal sequence, the sequence of the 21st to 260th amino acids represents C3022scFv, the sequence of the 261st to 265th amino acids represents a linker, and the sequence of the 266th to 383rd amino acids represents C3E-7085-heavy chain variable region. The sequence of the 384th to 713rd amino acids represents a constant region.

### 4)-5 Expression of scFv-Fab-heterodimer Fc-type bispecific molecule

An anti-Trop2scFv-CD3 Fab-heterodimer Fc (TX3-0002) was expressed by culturing using p_TX3-0002-HC_k, p_HC_h and p_C3E-7085-LC to prepare the culture supernatant of TX3-0002, in the same manner as in step 2)-3. The sequences of amino acids obtained by expressing individual vectors constituting TX3-0002 are represented by SEQ ID NO: 9 (Figure 13), SEQ ID NO:12 (Figure 16) and SEQ ID NO:29 (Figure 33) in the sequence listing.

An anti-CD98scFv-CD3 Fab-heterodimer Fc (TX3-0002) was expressed by culturing using p_CX3-0002-HC_k, p_HC_h and p_C3E-7085-LC to prepare the culture supernatant of TX3-0002, in the same manner as above. The sequences of amino acids obtained by expressing individual vectors constituting CX3-0002 are represented by SEQ ID NO: 9 (Figure 13), SEQ ID NO: 12 (Figure 16) and SEQ ID NO: 30 (Figure 34) in the sequence listing.

An anti-GPRC5DscFv-CD 3Fab-heterodimer Fc (RX3-0002) was expressed by culturing using p_RX3-0002-HC_k, p_HC_h and p_C3E-7085-LC to prepare the culture supernatant of RX3-0002, in the same manner as above. The sequences of amino acids obtained by expressing individual vectors constituting RX3-0002 are represented by SEQ ID NO: 9 (Figure 13), SEQ ID NO: 12 (Figure 16) and SEQ ID NO: 31 (Figure 35) in the sequence listing.

### 4)-6 Purification of scFv-Fab-heterodimer Fc-type bispecific molecule

Individual culture supernatants obtained in step 4)-5 were purified in the same manner as in step 2)-4. The purified protein samples were subjected to analytical SEC to determine the purity and concentration and used for various evaluations.

### (Example 4) Evaluation for solution stability of taFv-heterodimer Fc-type and scFv-Fab-heterodimer Fc-type bispecific molecule

Individual samples prepared in Example 3 were centrifugally concentrated by Amicon Ultra-4 (Millipore). After the buffer was exchanged to PBS, the concentrations of the samples were adjusted to be 20 mg/ml with PBS to prepare samples for evaluation. The HMWS (%) of the samples at the time of initiation of evaluation were obtained by size exclusion chromatography using AdvanceBio SEC 300A 2.7 µm 4.6 × 150 mm (Agilent) and calculated by the peak area percentage method. As the mobile phase, 0.2 M Ki/200 mM KCl/pH7.0 was used. Analysis was made at a flow rate of 0.2 ml/min (detection wavelength: 280 nm). The accelerated deterioration test of individual samples was carried out as follows. The samples were stored at 40°C and for 1, 7 and 14 days, and thereafter, subjected to size exclusion chromatography performed in the same conditions as above. The HMWS values (%) of individual samples were calculated by the peak area percentage method.

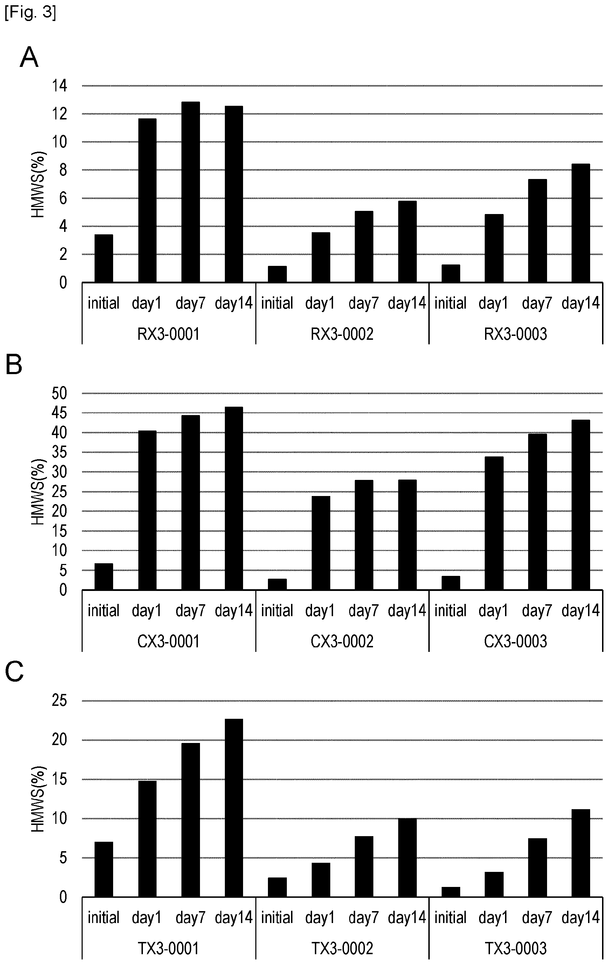

The results of the time-course change of individual samples stored at 40°C are shown in Figures 3A to C.

The amount of HMWS (%) of RX3-0001 with storage time increased up to 13%. In contrast, the amounts of HMWS (%) of RX3-0002 and RX3-0003 with storage time decreased to 6% and 8%, respectively (Figure 3A).

The amount of HMWS (%) of TX3-0001 with storage time increased up to 23%. In contrast, the amounts of HMWS (%) of TX3-0002 and TX3-0003 decreased to 10%, 11%, respectively (Figure 3B).

The amount of HMWS (%) of CX3-0001 with storage time increased up to 46% because the effect of solution stability of hM23-H1L1scFv was reflected. In contrast, the amount of HMWS (%) of CX3-0002 with storage time decreased to 28% and the amount of HMWS (%) of CX3-0003 with storage time was 43% showing a slight decreasing trend compared to CX3-0001 (Figure 3C).

The above results demonstrate that the scFv-Fab-heterodimer Fc-type molecule using C3E-7085 Fab format and the taFv-heterodimer Fc-type molecule using C3E-7096 are excellent in solution stability, compared to taFv-heterodimer Fc-type molecule using C3E-7085.

### (Example 5) Evaluation for shaking stability of taFv-heterodimer Fc-type and scFv-Fab-heterodimer Fc-type bispecific molecule

Of the samples purified in Example 3, RX3-0001 to 0003, CX3-0001 to 0003 and TX3-0001 to 0003 were selected and centrifugally concentrated by Amicon Ultra-4 (Millipore). The concentrations of the samples were adjusted to be 2 mg/ml to prepare evaluation samples. The peak area and HMWS (%) of the samples at the time of initiation of evaluation were obtained by size exclusion chromatography using AdvanceBio SEC 300A 2.7 µm 4.6 × 150 mm (Agilent) and calculated by the peak area percentage method. As the mobile phase, 0.2 M Ki/200 mM KCl/pH7.0 was used. Analysis was made at a flow rate of 0.2 ml/min (detection wavelength: 280 nm). Shaking deterioration test was carried out by shaking the samples by a universal high speed mixer (manufactured by Waken) at room temperature and 2500 rpm for one hour and two hours and subjecting the samples to size exclusion chromatography performed in the same conditions as above. The peak areas and HMWS (%) of the samples were calculated.

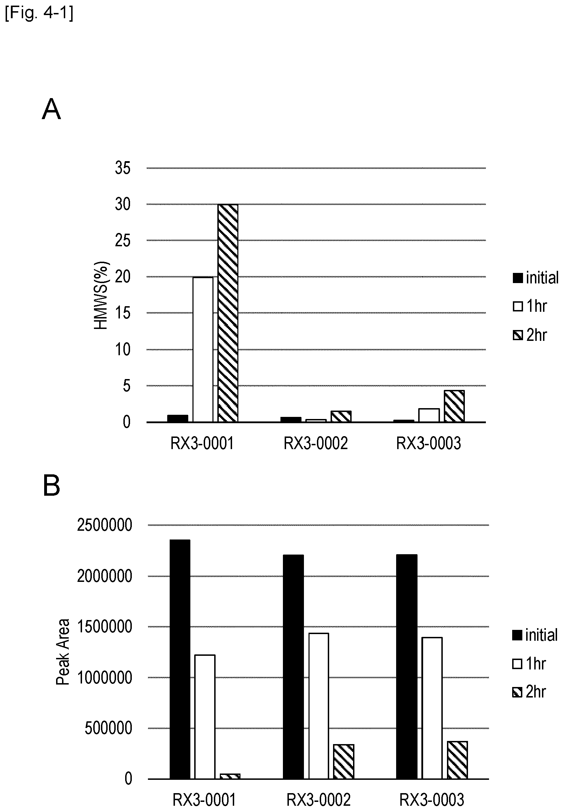

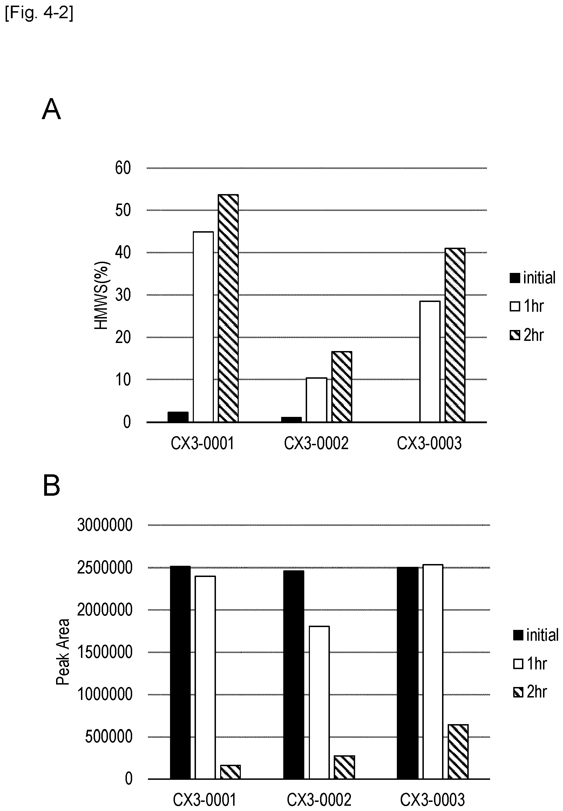

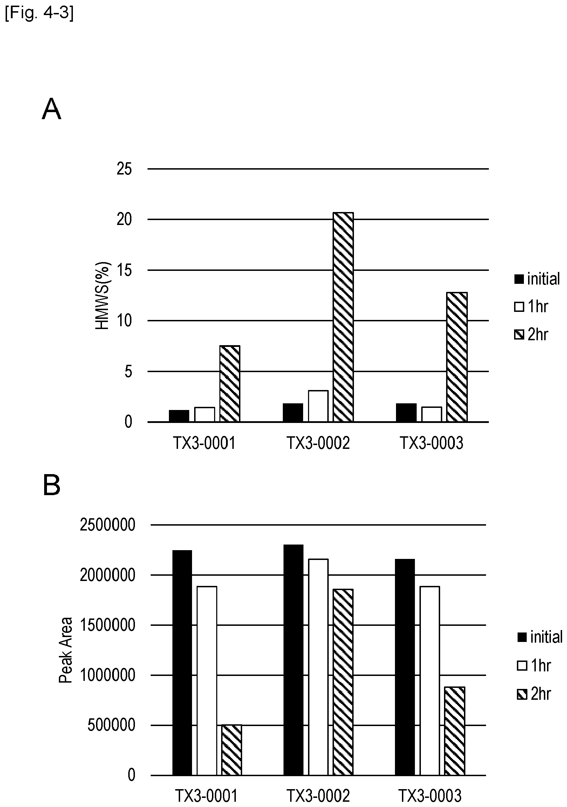

The peak area and HMWS (%) of the samples after shaking are shown in Figures 4-1A and B, Figures 4-2A and B and Figure 4-3A and B. The peak area residual rate of the sample shaken for two hours for RX3-0001 was 2%. In contrast, the peak residual rate for RX3-0002 and RX3-0003 were 15%, 17%, respectively, better than RX3-0001. Furthermore, the HMWS (%) of the sample shaken for two hours for RX3-0001 was 30%, while the HMWS (%) of the the sample shaken for two hours for RX3-0002 and RX3-0003 were both less than 5%, resulting in less HMWS (%) for RX3-0002 and RX3-0003 compared to RX3-0001.

The peak area residual rate of the sample shaken for two hours for CX3-0001 was 6%. In contrast, the peak residual rate for CX3-0002 and CX3-0003 were 11%, 26%, respectively, better than CX3-0001. Furthermore, the HMWS (%) of the sample shaken for two hours for CX3-0001, CX3-0002 and CX3-0003 were 54%, 17%, 41%, respectivley, resulting in less HMWS (%) for CX3-0002 and CX3-0003 compared to CX3-0001.

The peak area residual rate of the sample shaken for two hours for TX3-0001 was 22%. Furtheremore, the peak residual rate for TX3-0002 and TX3-0003 were 81%, 41%, respectively, resulting in good peak residual rate compared to TX3-0001. In contrast, the HMWS (%) of the sample shaken for two hours for TX3-0001, TX3-0002 and TX3-0003 were 7%, 21%, 13%, respectivley. The HMWS (%) of TX3-0001 was the lowest and showed a different trend from the other antibody groups (RX3 and CX3).

Therefore, in order to confirm the presence of large size particle formation in TX3-0001 to 0003, which cannot be detected by size exclusion chromatography, the number of particles was evaluated by FlowCAM8100 (DKSH Japan) using samples treated before and 2 hours after shaking. The changes over time in particle counts for each sample are shown in Table 2.

**[Table 2]**

| | TX3-0001 | TX3-0002 | TX3-0003 |
|---|---|---|---|
| initial | 63 | 66 | 332 |
| 1 hr | 171340 | 7107 | 78992 |
| 2 hr | 1272905 | 55243 | 562921 |

| | | | |
|---|---|---|---|
| (Particles/mL) | | | |

TX3-0001 had a particle count of 1272905 particles/mL in the sample shaken for two hours, while TX3-0002 and TX3-0003 had a particle count of 55243 particles/mL and 562921 particles/mL, respectively, in the sample shaken for two hours, indicating that the number of particles formed by aggregation was less than that of TX3-0001. Aggregates detected as HMWS are about 10-100 nm in diameter, whereas large multimeric aggregates of 1 µm or more are detected in FlowCAM. Since the number of particles in TX3-0001 is significantly larger than those in TX3-0002 and 0003, it is speculated that the low HMWS (%) of TX3-0001 is due to aggregates equivalent to HMWS aggregating to form even larger multimers. These results indicate that TX3-0002 and TX3-0003 have better shaking stability than TX3-0001.

These results show that the scFv-Fab-heterodimer Fc-type molecule using C3E-7085 Fab format and the taFv-heterodimer Fc-type molecule using C3E-7096 had superior shaking stability compared to the taFv-heterodimer Fc-type molecule using C3E-7085.

Samples are prepared by replacing anti-CD3scFv from C3E-7096 in the above examples to C3E-7097 (having the amino acid sequence represented by SEQ ID NO:39 and shown in Figure 46) , and then, tests and evaluations or the like can be carried out.

### (Example 6) Evaluation for solution stability of anti-CD3 antibody fragment-heterodimer Fc-type bispecific molecule

In order to improve physical properties for obtaining solution stability of C3E-7093 (scFv having the amino acid sequence represented by SEQ ID NO: 80 and shown in Figure 88 of International Publication No. WO2018/117237) in accordance with Example 1)-1 to 1)-4, the format of C3E-7093-Fab is modified. The amino acid sequences of C3E-7093-Fab (the amino acid sequence of C3E-7093-Fab represented by SEQ ID NO: 46 and shown in Figure 48, the amino acid sequence of C3E-7093-LC represented by SEQ ID NO: 47 and shown in Figure 49) and the amino acid sequences of C3E-7098 (amino acid sequence represented by SEQ ID NO: 48 and shown in Figure 50), which is a scFv having a disulfide bond introduced therein, are designed. Subsequently, expression vectors for preparing an anti-CD3 antibody fragment-heterodimer Fc-type molecule comprising the Fab and scFv are prepared, and both molecules are expressed and purified.

The individual samples obtained are centrifugally concentrated by Amicon Ultra-4 (Millipore). After the buffer is exchanged to PBS, the concentrations of the samples are adjusted to be 20 mg/ml with PBS to prepare samples for evaluation. The HMWS (%) of each sample at the time of initiation of evaluation is obtained by size exclusion chromatography using AdvanceBio SEC 300A 2.7 µm 4.6 × 150 mm (Agilent) and calculated by the peak area percentage method. As the mobile phase, 0.2 M Ki/200 mM KCl/pH7.0 is used. Analysis is made at a flow rate of 0.2 ml/min (detection wavelength: 280 nm). The accelerated deteriolation test of individual samples are carried out as follows. The samples are stored at 40°C and for 1, 7 and 14 days, and thereafter, subjected to size exclusion chromatography performed in the same conditions as above. The HMWS values (%) of individual samples are calculated by the peak area percentage method.

The time-course changes of the individual samples stored at 40°C are compared. As a result, it is demonstrated that the heterodimer Fc-type molecule using C3E-7093 Fab format and the heterodimer Fc-type molecule using C3E-7098, which is a scFv having a disulfide bond introduced therein, are excellent in solution stability, compared to the heterodimer Fc-type molecule using C3E-7093.

### (Example 7) Evaluation for shaking stability of anti-CD3 antibody fragment-heterodimer Fc-type bispecific molecule

After the individual samples purified in Example 6 are centrifugally concentrated by Amicon Ultra-4 (Millipore), the concentrations of the samples are adjusted to be 2 mg/ml to prepare evaluation samples. The peak area and HMWS (%) of each sample at the time of initiation of evaluation are obtained by size exclusion chromatography using AdvanceBio SEC 300A 2.7 µm 4.6 × 150 mm (Agilent) and calculated by the peak area percentage method. As the mobile phase, 0.2 M Ki/200 mM KCl/pH7.0 is used. Analysis is made at a flow rate of 0.2 ml/min (detection wavelength: 280 nm). The shaking deterioration test is carried out by shaking the samples by a universal high speed mixer (manufactured by Waken) at room temperature and 2500 rpm for one hour and two hours and subjecting the samples to size exclusion chromatography performed in the same conditions as above. The peaks areas and HMWS (%) of the individual samples are calculated.

The peak areas and HMWS (%) of the individual samples after shaking are compared. As a result, it is demonstrated that the heterodimer Fc-type molecule using C3E-7093 Fab format and the heterodimer Fc-type molecule using C3E-7098 having a disulfide bond introduced therein, are excellent in shaking stability, compared to the heterodimer Fc-type molecule using C3E-7093.

### (Example 8) Evaluation for solution stability of taFv-heterodimer Fc-type and scFv-Fab-heterodimer Fc-type bispecific molecule

In accordance with Example 3, an anti-Trop2-CD3taFv-heterodimer Fc comprising C3E-7098 as an anti-CD3scFv, an anti-CD98-CD3taFv-heterodimer Fc and an anti-GPRC5D-CD3taFv-heterodimer Fc, and, an anti-Trop2scFv-CD3Fab-heterodimer Fc comprising C3E-7093 Fab as an anti-CD3Fab, an anti-CD98scFv-CD3Fab-heterodimer Fc and an anti-GPRC5DscFv-CD3Fab-heterodimer Fc, are prepared.

The samples prepared are centrifugally concentrated by Amicon Ultra-4 (Millipore). After the buffer is exchanged to PBS, the concentrations of the samples are adjusted to be 20 mg/ml with PBS to prepare samples for evaluation. The HMWS (%) of each sample at the time of initiation of evaluation is obtained by size exclusion chromatography using AdvanceBio SEC 300A 2.7 µm 4.6 × 150 mm (Agilent) and calculated by the peak area percentage method. As the mobile phase, 0.2 M Ki/200 mM KCl/pH7.0 is used. Analysis is made at a flow rate of 0.2 ml/min (detection wavelength: 280 nm). The accelerated deterioration test of individual samples is carried out as follows. The samples are stored at 40°C and for 1, 7 and 14 days, and thereafter, subjected to size exclusion chromatography performed in the same conditions as above. The HMWS values (%) of individual samples are calculated by the peak area percentage method.

The time-course changes of the individual samples stored at 40°C are compared. As a result, it is demonstrated that the scFv-Fab heterodimer Fc-type using C3E-7093 Fab format and the taFv-heterodimer Fc-type molecule using C3E-7098 are excellent in solution stability, compared to the taFv-heterodimer Fc type molecule using C3E-7093.

### (Example 9) Evaluation for shaking stability of taFv-heterodimer Fc-type and scFv-Fab-heterodimer Fc-type bispecific molecule

After samples prepared in Example 8 are centrifugally concentrated by Amicon Ultra-4 (Millipore), the concentrations of the samples are adjusted to be 2 mg/ml to prepare evaluation samples. The peak area and HMWS (%) of each sample at the time of initiation of evaluation are obtained by size exclusion chromatography using AdvanceBio SEC 300A 2.7 µm 4.6 × 150 mm (Agilent) and calculated by the peak area percentage method. As the mobile phase, 0.2 M Ki/200 mM KCl/pH7.0 is used. Analysis is made at a flow rate of 0.2 ml/min (detection wavelength: 280 nm). The shaking deterioration test is carried out by shaking the samples by a universal high speed mixer (manufactured by Waken) at room temperature and 2500 rpm for one hour and two hours and subjecting the samples to size exclusion chromatography performed in the same conditions as above. The peak areas and HMWS (%) of the samples are calculated.

The peak areas and HMWS (%) of the individual samples after shaking are compared. As a result, it is demonstrated that the scFv-Fab-heterodimer Fc-type using C3E-7093 Fab format and the taFv-heterodimer Fc-type molecule using C3E-7098, are excellent in shaking stability, compared to the taFv-heterodimer Fc-type molecule using C3E-7093.

Samples are prepared by replacing anti-CD3scFv from C3E-7098 in Examples 6 to 9 to C3E-7099 (having the amino acid sequence represented by SEQ ID NO: 49 and shown in Figure 51), and then, tests and evaluations or the like can be carried out.

### Industrial Applicability

The bispecific antibody of the present invention can be used as a therapeutic agent or preventive agent for, e.g., cancer.

### Sequence Listing Free Text

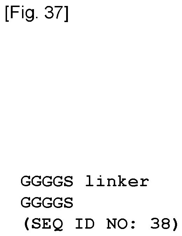

SEQ ID NO: 1: Amino acid sequence of C3E-7085-Fab (Figure 5)
SEQ ID NO: 2: Amino acid sequence of C3E-7085-LC (Figure 6)
SEQ ID NO: 3: Amino acid sequence of C3E-7096 (Figure 7)
SEQ ID NO: 4: Nucleotide sequence of HC_h full length (Figure 8)
SEQ ID NO: 5: Full-length nucleotide sequence of C3E-7085-HC-k (Figure 9)
SEQ ID NO: 6: Full-length nucleotide sequence of C3E-7085-Fab-HC-k (Figure 10)
SEQ ID NO: 7: Full-length nucleotide sequence of C3E-7085-LC (Figure 11)
SEQ ID NO: 8: Full-length nucleotide sequence of C3E-7096-HC-k (Figure 12)
SEQ ID NO: 9: Full-length amino acid sequence of HC_h (Figure 13)
SEQ ID NO: 10: Full-length amino acid sequence of C3E-7085-HC-k (Figure 14)
SEQ ID NO: 11: Full-length amino acid sequence of C3E-7085-Fab-HC-k (Figure 15)
SEQ ID NO: 12: Full-length amino acid sequence of C3E-7085-LC (Figure 16)
SEQ ID NO: 13: Full-length amino acid sequence of C3E-7096-HC-k (Figure 17)
SEQ ID NO: 14: Full-length nucleotide sequence of TX3-0001-HC_k (Figure 18)
SEQ ID NO: 15: Full-length nucleotide sequence of TX3-0003-HC_k (Figure 19)
SEQ ID NO: 16: Full-length nucleotide sequence of CX3-0001-HC_k (Figure 20)
SEQ ID NO: 17: Full-length nucleotide sequence of CX3-0003-HC_k (Figure 21)
SEQ ID NO: 18: Full-length nucleotide sequence of RX3-0001-HC_k (Figure 22)
SEQ ID NO: 19: Full-length nucleotide sequence of CX3-0001-HC_k (Figure 23)
SEQ ID NO: 20: Full-length amino acid sequence of TX3-0001-HC_k (Figure 24)
SEQ ID NO: 21: Full-length amino acid sequence of TX3-0003-HC_k (Figure 25)
SEQ ID NO: 22: Full-length amino acid sequence of CX3-0001-HC_k (Figure 26)
SEQ ID NO: 23: Full-length amino acid sequence of CX3-0003-HC_k (Figure 27)
SEQ ID NO: 24: Full-length amino acid sequence of RX3-0001-HC_k (Figure 28)
SEQ ID NO: 25: Full-length amino acid sequence of RX3-0003-HC_k (Figure 29)
SEQ ID NO: 26: Full-length nucleotide sequence of TX3-0002-HC_k (Figure 30)
SEQ ID NO: 27: Full-length nucleotide sequence of CX3-0002-HC_k (Figure 31)
SEQ ID NO: 28: Full-length nucleotide sequence of RX3-0002-HC_k (Figure 32)
SEQ ID NO: 29: Full-length amino acid sequence of TX3-0002-HC_k (Figure 33)
SEQ ID NO: 30: Full-length amino acid sequence of CX3-0002-HC_k (Figure 34)
SEQ ID NO: 31: Full-length amino acid sequence of RX3-0002-HC_k (Figure 35)
SEQ ID NO: 32: Amino acid sequence of C3E-7085 heavy chain CDRH1 (Figure 36)
SEQ ID NO: 33: Amino acid sequence of C3E-7085 heavy chain CDRH2 (Figure 36)
SEQ ID NO: 34: Amino acid sequence of C3E-7085 heavy chain CDRH3 (Figure 36)
SEQ ID NO: 35: Amino acid sequence of C3E-7085 light chain CDRL1 (Figure 36)
SEQ ID NO: 36: Amino acid sequence of C3E-7085 light chain CDRL2 (Figure 36)
SEQ ID NO: 37: Amino acid sequence of C3E-7085 light chain CDRL3 (Figure 36)
SEQ ID NO: 38: Amino acid sequence of a peptide linker (Figure 37)
SEQ ID NO: 39: Amino acid sequence of C3E-7097 (Figure 46)
SEQ ID NO: 40: Amino acid sequence of C3E-7093 heavy chain CDRH1 (Figure 47)
SEQ ID NO: 41: Amino acid sequence of C3E-7093 heavy chain CDRH2 (Figure 47)
SEQ ID NO: 42: Amino acid sequence of C3E-7093 heavy chain CDRH3 (Figure 47)
SEQ ID NO: 43: Amino acid sequence of C3E-7093 light chain CDRL1 (Figure 47)
SEQ ID NO: 44: Amino acid sequence of C3E-7093 light chain CDRL2 (Figure 47)
SEQ ID NO: 45: Amino acid sequence of C3E-7093 light chain CDRL3 (Figure 47)
SEQ ID NO: 46: Amino acid sequence of C3E-7093-Fab (Figure 48)
SEQ ID NO: 47: Amino acid sequence of C3E-7093-LC (Figure 49)
SEQ ID NO: 48: Amino acid sequence of C3E-7098 (Figure 50)
SEQ ID NO: 49: Amino acid sequence of C3E-7099 (Figure 51)

All publications, patents and patent applications cited herein are incorporated herein by reference in their entirety.

## Claims

1. A Fab that specifically binds to CD3, the Fab comprising the following CDRH1 to 3 and CDRL1 to 3:
CDRH1 consisting of the amino acid sequence represented by SEQ ID NO: 32;
CDRH2 consisting of the amino acid sequence represented by SEQ ID NO: 33;
CDRH3 consisting of the amino acid sequence represented by SEQ ID NO: 34;
CDRL1 consisting of the amino acid sequence represented by SEQ ID NO: 35;
CDRL2 consisting of the amino acid sequence represented by SEQ ID NO: 36; and
CDRL3 consisting of the amino acid sequence represented by SEQ ID NO: 37.

2. The Fab according to claim 1, comprising a heavy chain variable region consisting of amino acids at positions 1 to 118 in the amino acid sequence represented by SEQ ID NO: 1 and a light chain variable region consisting of amino acids at positions 1 to 107 in the amino acid sequence represented by SEQ ID NO: 2.

3. The Fab according to claim 1 or 2, comprising a polypeptide consisting of the amino acid sequence represented by SEQ ID NO: 1 and a light chain consisting of the amino acid sequence represented by SEQ ID NO: 2.

4. A multispecific molecule comprising the Fab according to any one of Claims 1 to 3, wherein the multispecific molecule specifically binds to a target molecule that is not a complex of a cancer-testis antigen peptide and a major histocompatibility complex (MHC).

5. The multispecific molecule according to claim 4, wherein the MHC is a human leukocyte antigen (HLA).

6. The multispecific molecule according to claim 4 or 5, comprising an antibody that specifically binds to the target molecule or an antigen binding fragment thereof.

7. The multispecific molecule according to any one of claims 4 to 6, wherein the multispecific molecule is a multispecific antibody.

8. The multispecific molecule according to any one of claims 4 to 7, wherein the multispecific molecule comprises scFv-Fab-heterodimer Fc.

9. The multispecific molecule according to any one of claims 4 to 8, wherein the multispecific molecule is a trispecific antibody.

10. The multispecific molecule according to any one of claims 4 to 8, wherein the multispecific molecule is a bispecific antibody.

11. The multispecific molecule according to any one of claims 4 to 10, wherein the target molecule is GPRC5D, CD98 or Trop 2.

12. The multispecific molecule according to any one of claims 4 to 11, which comprises one polypeptide group selected from the following (i) to (iii);
(i) a polypeptide comprising an amino acid sequence consisting of the 20-718th amino acid residues of SEQ ID NO: 29 or an amino acid sequence in which one or two amino acids are deleted at the carboxyl terminus of said amino acid sequence, a polypeptide comprising an amino acid sequence consisting of the 20-246th amino acid residues of SEQ ID NO: 9 or an amino acid sequence in which one or two amino acids are deleted at the carboxyl terminus of said amino acid sequence, and a polypeptide comprising an amino acid sequence consisting of the 21-233rd amino acid residues of SEQ ID NO: 12;
(ii) a polypeptide comprising an amino acid sequence consisting of the 20-717th amino acid residues of SEQ ID NO: 30 or an amino acid sequence in which one or two amino acids are deleted at the carboxyl terminus of said amino acid sequence, a polypeptide comprising an amino acid sequence consisting of the 20-246th amino acid residues of SEQ ID NO: 9 or an amino acid sequence in which one or two amino acids are deleted at the carboxyl terminus of said amino acid sequence, and a polypeptide comprising an amino acid sequence consisting of the 21-233rd amino acid residues of SEQ ID NO: 12; and
(iii) a polypeptide comprising an amino acid sequence consisting of the 20-713rd amino acid residues of SEQ ID NO: 31 or an amino acid sequence in which one or two amino acids are deleted at the carboxyl terminus of said amino acid sequence, a polypeptide comprising an amino acid sequence consisting of the 20-246th amino acid residues of SEQ ID NO: 9 or an amino acid sequence in which one or two amino acids are deleted at the carboxyl terminus of said amino acid sequence; and a polypeptide comprising an amino acid sequence consisting of the 21-233rd amino acid residues of SEQ ID NO: 12.

13. A polynucleotide comprising a nucleotide sequence encoding an amino acid sequence comprised in the multispecific molecule according to any one of claims 4 to 12.

14. A vector comprising the polynucleotide according to claim 13.

15. A cell comprising the polynucleotide according to claim 13 or the vector according to claim 14, or producing the multispecific molecule according to any one of claims 4 to 12.

16. A method for producing the multispecific molecule according to any one of claims 4 to 12, comprising a step of culturing the cell according to claim 15.

17. A multispecific molecule obtained by the method according to claim 16.

18. A composition comprising the Fab according to any one of claims 1 to 3, the multispecific molecule according to any one of claims 4 to 12 or claim 17, the polynucleotide according to claim 13, the vector according to claim 14 or the cell according to claim 15.

19. A scFv that specifically binds to CD3 and has a heavy chain variable region and a light chain variable region connected via a disulfide bond, the scFv comprising the following CDRH1 to 3 and CDRL1 to 3:
CDRH1 consisting of the amino acid sequence represented by SEQ ID NO: 32;
CDRH2 consisting of the amino acid sequence represented by SEQ ID NO: 33;
CDRH3 consisting of the amino acid sequence represented by SEQ ID NO: 34;
CDRL1 consisting of the amino acid sequence represented by SEQ ID NO: 35;
CDRL2 consisting of the amino acid sequence represented by SEQ ID NO: 36; and
CDRL3 consisting of the amino acid sequence represented by SEQ ID NO: 37.

20. The scFv according to claim 19, comprising a heavy chain variable region consisting of amino acids at positions 1 to 118 in the amino acid sequence represented by SEQ ID NO: 3 and a light chain variable region consisting of amino acids at positions 134 to 240 in the amino acid sequence represented by SEQ ID NO: 3.

21. The scFv according to claim 19 or 20, consisting of the amino acid sequence represented by SEQ ID NO: 3.

22. The scFv according to claim 19, comprising a heavy chain variable region consisting of amino acids at positions 1 to 118 in the amino acid sequence represented by SEQ ID NO: 39 and a light chain variable region consisting of amino acids at positions 134 to 240 in the amino acid sequence represented by SEQ ID NO: 39.

23. The scFv according to claim 19 or 22, consisting of the amino acid sequence represented by SEQ ID NO: 39

24. A multispecific molecule comprising the scFv according to any one of claims 19 to 23, wherein the multispecific molecule specifically binds to a target molecule that is not a complex of a cancer-testis antigen peptide and a major histocompatibility complex (MHC).

25. The multispecific molecule according to claim 24, wherein the MHC is a human leukocyte antigen (HLA).

26. The multispecific molecule according to claim 24 or 25, comprising an antibody that specifically binds to the target molecule or an antigen binding fragment thereof.

27. The multispecific molecule according to any one of claims 24 to 26, wherein the multispecific molecule is a multispecific antibody,

28. The multispecific molecule according to any one of claims 24 to 27, wherein the multispecific molecule comprises taFv-Fab-heterodimer Fc.

29. The multispecific molecule according to any one of claims 24 to 28, wherein the multispecific molecule is a trispecific antibody.

30. The multispecific molecule according to any one of claims 24 to 28, wherein the multispecific molecule is a bispecific antibody.

31. The multispecific molecule according to any one of claims 24 to 30, wherein the target molecule is GPRC5D, CD98 or Trop 2.

32. The multispecific molecule according to any one of claims 24 to 31, which comprises one polypeptide pair selected from the following (i) to (iii);
(i) a polypeptide comprising an amino acid sequence consisting of the 20-744th amino acid residues of SEQ ID NO: 21 or an amino acid sequence in which one or two amino acids are deleted at the carboxyl terminus of said amino acid sequence, and a polypeptide comprising an amino acid sequence consisting of the 20-246th amino acid residues of SEQ ID NO: 9 or an amino acid sequence in which one or two amino acids are deleted at the carboxyl terminus of said amino acid sequence;
(ii) a polypeptide comprising an amino acid sequence consisting of the 20-743rd amino acid residues of SEQ ID NO: 23 or an amino acid sequence in which one or two amino acids are deleted at the carboxyl terminus of said amino acid sequence, and a polypeptide comprising an amino acid sequence consisting of the 20-246th amino acid residues of SEQ ID NO: 9, or an amino acid sequence in which one or two amino acids are deleted at the carboxyl terminus of said amino acid sequence; and
(iii) a polypeptide comprising an amino acid sequence consisting of the 20-739th amino acid residues of SEQ ID NO: 25 or an amino acid sequence in which one or two amino acids are deleted at the carboxyl terminus of said amino acid sequence, and a polypeptide comprising an amino acid sequence consisting of the 20-246th amino acid residues of SEQ ID NO: 9 or an amino acid sequence in which one or two amino acids are deleted at the carboxyl terminus of said amino acid sequence.

33. A polynucleotide comprising a nucleotide sequence encoding an amino acid sequence comprised in the multispecific molecule according to any one of claims 24 to 32.

34. A vector comprising the polynucleotide according to claim 33.

35. A cell comprising the polynucleotide according to claim 33 or the vector according to claim 34, or producing the multispecific molecule according to any one of claims 24 to 32.

36. A method for producing the multispecific molecule according to any one of claims 24 to 32, comprising a step of culturing the cell according to claim 35.

37. A multispecific molecule obtained by the method according to claim 36.

38. A composition comprising the scFv according to any one of claims 19 to 23, the multispecific molecule according to any one of claims 24 to 32 or claim 37, the polynucleotide according to claim 33, the vector according to claim 34 or the cell according to claim 35.

39. A Fab that specifically binds to CD3 and comprises the following CDRH1 to 3 and CDRL1 to 3:
CDRH1 consisting of the amino acid sequence represented by SEQ ID NO: 40;
CDRH2 consisting of the amino acid sequence represented by SEQ ID NO: 41;
CDRH3 consisting of the amino acid sequence represented by SEQ ID NO: 42;
CDRL1 consisting of the amino acid sequence represented by SEQ ID NO: 43;
CDRL2 consisting of the amino acid sequence represented by SEQ ID NO: 44; and
CDRL3 consisting of the amino acid sequence represented by SEQ ID NO: 45.

40. The Fab according to claim 39, comprising a heavy chain variable region consisting of amino acids at positions 1 to 118 in the amino acid sequence represented by SEQ ID NO: 46 and a light chain variable region consisting of amino acids at positions 1 to 109 in the amino acid sequence represented by SEQ ID NO: 47.

41. The Fab according to claim 39 or 40, comprising a polypeptide consisting of the amino acid sequence represented by SEQ ID NO: 46 and a light chain consisting of the amino acid sequence represented by SEQ ID NO: 47.

42. A multispecific molecule comprising the Fab according to any one of claims 39 to 41, wherein the multispecific molecule specifically binds to a target molecule that is not a complex of a cancer-testis antigen peptide and a major histocompatibility complex (MHC).

43. The multispecific molecule according to claim 42, wherein the MHC is a human leukocyte antigen (HLA).

44. The multispecific molecule according to claim 42 or 43, comprising an antibody that specifically binds to the target molecule or an antigen binding fragment thereof.

45. The multispecific molecule according to any one of claims 42 to 44, wherein the multispecific molecule is a multispecific antibody.

46. The multispecific molecule according to any one of claims 42 to 45, wherein the multispecific molecule is a bispecific antibody.

47. A polynucleotide comprising a nucleotide sequence encoding an amino acid sequence comprised in the multispecific molecule according to any one of claims 42 to 46.

48. A vector comprising the polynucleotide according to claim 47.

49. A cell comprising the polynucleotide according to claim 47 or the vector according to claim 48, or producing the multispecific molecule according to any one of claims 42 to 46.

50. A method for producing the multispecific molecule according to any one of claims 34 to 38, comprising a step of culturing the cell according to claim 49.

51. A multispecific molecule obtained by the method according to claim 50.

52. A composition comprising the Fab according to any one of claims 39 to 41, the multispecific molecule according to any one of claims 42 to 46, the polynucleotide according to claim 47, the vector according to claim 48 or the cell according to claim 49.

53. A scFv that specifically binds to CD3 and has a heavy chain variable region and a light chain variable region connected via a disulfide bond, the scFv comprising the following CDRH1 to 3 and CDRL1 to 3:
CDRH1 consisting of the amino acid sequence represented by SEQ ID NO: 40;
CDRH2 consisting of the amino acid sequence represented by SEQ ID NO: 41;
CDRH3 consisting of the amino acid sequence represented by SEQ ID NO: 42;
CDRL1 consisting of the amino acid sequence represented by SEQ ID NO: 43;
CDRL2 consisting of the amino acid sequence represented by SEQ ID NO: 44; and
CDRL3 consisting of the amino acid sequence represented by SEQ ID NO: 45.

54. The scFv according to claim 53, comprising a heavy chain variable region consisting of amino acids at positions 1 to 118 in the amino acid sequence represented by SEQ ID NO: 48 and a light chain variable region consisting of amino acids at positions 134 to 242 in the amino acid sequence represented by SEQ ID NO: 48.

55. The scFv according to claim 53 or 54, consisting of the amino acid sequence represented by SEQ ID NO: 48.

56. The scFv according to claim 53, comprising a heavy chain variable region consisting of amino acids at positions 1 to 118 in the amino acid sequence represented by SEQ ID NO: 49 and a light chain variable region consisting of amino acids at positions 134 to 242 in the amino acid sequence represented by SEQ ID NO: 49.

57. The scFv according to claim 53 or 56, consisting of the amino acid sequence represented by SEQ ID NO: 49.

58. A multispecific molecule comprising the scFv according to any one of claims 53 to 57, wherein the multispecific molecule specifically binds to a target molecule that is not a complex of a cancer-testis antigen peptide and a major histocompatibility complex (MHC).

59. The multispecific molecule according to claim 58, wherein the MHC is a human leukocyte antigen (HLA).

60. The multispecific molecule according to claim 58 or 59, comprising an antibody that specifically binds to the target molecule or an antigen binding fragment thereof.

61. The multispecific molecule according to any one of claims 58 to 60, wherein the multispecific molecule is a multispecific antibody.

62. The multispecific molecule according to any one of claims 58 to 61, wherein the multispecific molecule is a bispecific antibody.

63. A polynucleotide comprising a nucleotide sequence encoding an amino acid sequence comprised in the multispecific molecule according to any one of claims 58 to 62.

64. A vector comprising the polynucleotide according to claim 63.

65. A cell comprising the polynucleotide according to claim 63 or the vector according to claim 64, or producing the multispecific molecule according to any one of claims 58 to 62.

66. A method for producing the multispecific molecule according to any one of claims 58 to 62, comprising a step of culturing the cell according to claim 65.

67. A multispecific molecule obtained by the method according to claim 66.

68. A composition comprising the scFv according to any one of claims 53 to 57, the multispecific molecule according to any one of claims 58 to 62, the polynucleotide according to claim 63, the vector according to claim 64 or the cell according to claim 65.

69. A pharmaceutical composition comprising the multispecific molecule according to any one of claims 4 to 12, 24 to 32, 42 to 46 and 58 to 62.
